# EUROPEAN PATENT APPLICATION

(11) **EP 4 711 464 A1**
(43) Date of publication of application: **18.03.2026**
(21) Application number: 24818516.7
(22) Date of filing: 24.05.2024
(51) Int. Cl.: C12N 15/50, C12N 15/62, C12N 15/85, C07K 14/165, C07K 19/00, A61K 39/215, A61P 31/14

(54) **PHARMACEUTICAL COMPOSITION FOR PREVENTING AND TREATING INFECTIOUS PERITONITIS IN ANIMALS**

(30) Priority: 09.06.2023 CN 202310683619
(71) Applicant: Beijing Vjtbio Co., Ltd., Beijing 100085 (CN); Zhejiang VJTBio Co., Ltd., Taizhou, Zhejiang 317300 (CN)
(72) Inventor: LUO, Haoshu, Beijing 100085 (CN); LI, Jianxu, Taizhou, Zhejiang 317300 (CN); TAN, Zemin, Beijing 100085 (CN); DUAN, Junye, Beijing 100085 (CN); SHI, Lei, Beijing 100085 (CN); YANG, Youqiao, Taizhou, Zhejiang 317300 (CN); CHEN, Sikai, Taizhou, Zhejiang 317300 (CN)
(74) Representative: reuteler & cie SA
(86) International application number: PCT/CN2024/095252
(87) International publication number: WO 2024/250983

(57) **Abstract**

Provided are a pharmaceutical composition for treating and preventing infectious peritonitis in animals. The pharmaceutical composition is mainly composed of M protein, N protein, and S protein which contain a FCoV virus strain, and is mainly used for preventing and treating feline infectious peritonitis. Experiments proved that the pharmaceutical composition can effectively play a role in protecting an animal body and has important significance in preventing FIP.

## Description

### CROSS-REFERENCE TO RELATED APPLICATIONS

The present application claims priority to Chinese Patent Application No. 202310683619.6, filed on June 9, 2023, the content of which is incorporated herein by reference in its entirety.

### TECHNICAL FIELD

The present invention relates to a pharmaceutical composition for preventing and treating infectious peritonitis in animals.

### BACKGROUND

Infectious peritonitis often refers to tuberculous peritonitis and peritonitis secondary to cirrhotic ascites caused by hepatitis, generally transmitted through oral and nasal routes. Among them, Feline Infectious Peritonitis (FIP) is a systemic, lethal infectious disease caused by Feline Coronavirus (FCoV) infection. The main characteristics of the disease are peritonitis, significant accumulation of ascitic fluid, and high mortality. Early symptoms are insidious, primarily presented as reduced appetite, lethargy, weight loss, and persistent fever. Later symptoms become more apparent and can be categorized into two forms: the effusive (wet) form and the non-effusive (dry) form. The effusive form is characterized by the accumulation of fluid in body cavities, particularly the abdominal cavity. The non-effusive form is characterized by granulomatous lesions in various organs and associated clinical symptoms (such as ataxia, mild paralysis, etc.). FIP predominantly affects young cats aged 3 to 9 months, especially in cat population fed in groups. With the onset of the disease, the mortality rate is nearly 100%, making it one of the most common causes of death in cats in recent years.

Feline Coronavirus (FCoV) has two biotypes: Feline Enteric Coronavirus (FECV) and Feline Infectious Peritonitis Virus (FIPV), wherein FIPV is derived from the mutation of FECV and is the viral strain responsible for causing FIP. FCoV is an enveloped positive-sense RNA virus belonging to the order Nidovirales, family Coronaviridae, genus Coronavirus. Its genome is approximately 29 kb in size, containing two non-structural genes respectively encoding replicase 1a and 1b; four structural genes encoding the Spike (S) protein, Envelope (E) protein, Membrane (M) protein, and Nucleocapsid (N) protein; and five accessory genes: 3a, 3b, 3c, 7a, and 7b. Wherein, the full-length S protein consists of of two parts, S1 and S2. S1, composed of an N-Terminal Domain (NTD) and a Receptor-Binding Domain (RBD), is primarily responsible for binding to ACE2. S2 is primarily responsible for fusion with the feline cell membrane to facilitate the release of genetic material. The M and E proteins are mainly involved in viral assembly and fabricating. The N protein is abundant in coronaviruses and is highly immunogenic, participating in genome replication and cellular signaling pathway regulation.

There is currently no reliably effective treatment for FIP, and certain medications can only prolong lifespan and improve the quality of life. Therefore, preventing the occurrence of FIP is particularly crucial. Currently, a commercialized FCoV intranasal vaccine, namely Pfizer's attenuated live vaccine Primucell FIP, is available for kittens older than 16 weeks. However, it primarily targets the type II FCoV mutant strain DF2-FIPV, while type I FCoV is more prevalent worldwide. As a result, the protective efficacy of this vaccine remains controversial. Meanwhile, there is no commercially available vaccine effective against type I FIPV currently.

mRNA vaccines are a type of nucleic acid formulation functioned by constructing mRNA sequences encoding specific antigens according to various diseases, packaging and delivery into host cells by novel Lipid Nanoparticle (LNP) carriers, and exploiting the host cell's ribosomes to translate the mRNA sequences to produce target antigen proteins to trigger a specific immune response in the body, thereby conferring immune protection. Compared to traditional vaccines, mRNA vaccines offer multiple advantages, such as enhanced safety, efficacy, rapid production, lower production cost, and the ability to address viral mutations.

In summary, the research and development of mRNA vaccines for the prevention of FIP is of significant importance.

### SUMMARY

### DEFINITIONS

Unless otherwise defined, all technical and scientific terms used herein have the same meanings as commonly understood by those skilled in the art to which the present invention belongs.

### DEFINITION OF GENERAL TERMS

In the claims and/or specification, when used in conjunction with the term "comprising", the words " a" or " an" may refer to "one", but may also refer to "one or a plurality of', "at least one", and "one or more".

As used in the claims and specification, the words "comprising", "having" "including" or "containing" refer to inclusive or open-ended, and do not exclude additional, unrecited elements or method steps.

Throughout the entire application, the term "approximately" indicates that a value includes the standard deviation of the error of the device or method used to determine the value. The term "etc." indicates substitutable features that can be reasonably expected within the scope of the enumeration.

Although the disclosed content supports the definition of the term "or" as denoting alternatives only and "and/or", unless explicitly stated as denoting alternatives only or unless the alternatives are mutually exclusive, the term "or" in the claims refers to "and/or".

The terms "polypeptide", "peptide", and "protein" are used interchangeably herein and refer to amino acid polymer of any length. The polymer can be linear or branched. It can contain modified amino acids, and it can be partitioned by non-amino acids. The terms also include amino acid polymers that have been modified (e.g., formation of disulfide bonds, glycosylation, lipidation, acetylation, phosphorylation, or any other operation, such as conjugation with a labeling component).

As used herein, the terms "optionally," "optional," or "option" typically mean that the subsequently described event or circumstance may but may not necessarily occur, and that the description includes instances where said event or circumstance occurs and instances where said event or circumstance does not occur.

The terms "identity" and "homology" as used in the present invention refer to that those skilled in the art may adjust the sequences according to actual work needs when using amino acid sequences or nucleotide sequences, such that the used sequences, compared to sequences obtained from the prior art, possess a similarity of (including but not limited to) 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, and still have the same function as the original amino acid sequence or nucleotide sequence.

As used with respect to peptides or polypeptides, the term "variant" refers to a peptide or polypeptide that differs in amino acid sequence due to the insertion, deletion, or conservative substitution of amino acids but retains at least one biological activity. Variants can also refer to proteins with amino acid sequences that are essentially identical to the reference protein, where the reference protein has an amino acid sequence that retains at least one biological activity. Conservative substitutions of amino acids, i.e., the replacement of an amino acid with a different amino acid having similar properties (e.g., hydrophilicity, degree and distribution of charged regions), are generally considered in the art to involve minor changes. As understood in the art, these minor changes can be partially identified by considering the hydrophilicity index of the amino acids (Kyte et al., 1982, J. Mol. Biol. 157:105-132). The hydrophilicity index of an amino acid is based on considerations of its hydrophobicity and charge. It is known in the art that amino acids with similar hydrophilicity indices can be substituted and still retain protein function. In one aspect, amino acids having a hydrophilicity index within ±2 are substituted. The hydrophilicity of amino acids can also be used to reveal substitutions that result in the retention of biological function in a protein. Consideration of the hydrophilicity of amino acids in the context of a peptide allows for the calculation of the peptide's maximum local average hydrophilicity, which is a useful measurement reported to be closely correlated with antigenicity and immunogenicity. U.S. Patent No. 4,554,101 is incorporated herein by reference in its entirety. Substituting amino acids having similar hydrophilicity values can result in peptides that retain biological activity, such as immunogenicity, as is understood in the art. Substitutions can be made using amino acids whose hydrophilicity values differ from each other within a range of ±2. Both the hydrophobicity index and the hydrophilicity value of an amino acid are influenced by the specific side chain of the amino acid. Consistent with this observation, amino acid substitutions compatible with biological function are understood to depend on the relative similarity of the amino acids, particularly side chains of the amino acids, such as hydrophobicity, hydrophilicity, charge, size, and other attributes.

As used herein regarding nucleic acids, the term "variant" refers to (i) a portion or fragment of a reference nucleotide sequence; (ii) a complementary sequence of the reference nucleotide sequence or a portion thereof; (iii) a nucleic acid that is substantially identical to the reference nucleic acid or its complementary sequence; (iv) a nucleic acid that hybridizes under stringent conditions to the reference nucleic acid, its complementary sequence, or a sequence substantially identical thereto. A variant can be a nucleic acid sequence that is substantially identical over the entire length of the complete gene sequence or a fragment thereof. The nucleic acid or protein sequence may be 66%, 70%, 75%, 80%, 81%, 82%, 83%, 84%, 85%, 86%, 87%, 88%, 89%, 90%, 91%, 92%, 93%, 94%, 95%, 96%, 97%, 98%, 99%, or 100% identical over the entire length of the gene, protein sequence, or a fragment thereof.

As used herein regarding the description of amino acid sequences or nucleic acid sequences relative to a reference sequence, the terms "identity," "homology," or "similarity" are the percentage of identical amino acids or nucleotides between two amino acid sequences or two nucleic acid sequences, determined using conventional methods, see, for example, Ausubel et al., (1995) Current Protocols in Molecular Biology, Chapter 19 (Greene Publishing and Wiley-Interscience, New York); and the ALIGN program (Dayhoff (1978), Atlas of Protein Sequence and Structure 5: Suppl. 3 (National Biomedical Research Foundation, Washington, D.C.)). There are numerous algorithms for aligning sequences and determining sequence identity, including: the homology alignment algorithm of Needleman et al. (1970) J. Mol. Biol. 48: 443; the local homology algorithm of Smith et al. (1981) Adv. Appl. Math. 2: 482; the similarity search method of Pearson et al. (1988) Proc. Natl. Acad. Sci. 85: 2444; the Smith-Waterman algorithm, Meth. Mol. Biol. 70: 173-187 (1997); and the BLASTP, BLASTN, and BLASTX algorithms (see Altschul et al. (1990) J. Mol. Biol. 215: 403-410). Computer programs utilizing these algorithms are also available and include, but are not limited to: ALIGN or Megalign (DNASTAR) software; or WU-BLAST-2 (Altschul et al., Meth. Enzym., 266: 460-480 (1996)); or GAP, BESTFIT, BLAST (Altschul et al., supra), FASTA, and TFASTA, available in the Genetics Computing Group (GCG) package, Version 8, Madison, Wisconsin, USA; and CLUSTAL in the PC/Gene program provided by Intelligenetics, Mountain View, California.

As used herein, the term "fragment" refers to a target protein or polypeptide, as well as a target protein or polypeptide with N-terminal and/or C-terminal truncations, and/or internal deletions.

"Encoding" refers to the inherent property of a specific nucleotide sequence in a polynucleotide (e.g., a gene, cDNA, or mRNA) that can serve as a template for the biosynthesis of other polymers and macromolecules in biological processes. These polymers and macromolecules have defined nucleotide sequences (i.e., rRNA, tRNA, and mRNA) or defined amino acid sequences and the resulting biological properties. Thus, a gene encodes a protein if transcription and translation of the mRNA corresponding to that gene produces the protein in cells or other biological system. Both the coding strand (whose nucleotide sequence is identical to the mRNA sequence and is typically provided in sequence listings) and the non-coding strand (served as the template for transcription of the gene or cDNA) can be referred to as encoding the protein or other product of the gene or cDNA.

### mRNA Vaccine Terminology Definitions

The term "immunogen" as used herein is intended to refer to a substance capable of inducing an adaptive immune response in a subject, wherein said adaptive immune response is capable of inducing an immune response against a pathogen sharing immunological features with the immunogen. An "immunogen" refers to any substance introduced into the body to generate an immune response. The substance can be a physical molecule, such as a protein, or can be encoded by a vector, such as DNA, mRNA, or a virus.

The term "immune response," as used herein, refers to a process involving the activation and/or induction of effector functions in, as non-limiting examples, T cells, B cells, Natural Killer (NK) cells, and/or Antigen Presenting Cells (APCs). Thus, as will be appreciated by those skilled in the art, an immune response includes, but is not limited to, any detectable antigen-specific activation and/or induction of helper T cell or cytotoxic T cell activity or response, production of antibodies, antigen-presenting cell activity or infiltration, macrophage activity or infiltration, neutrophil activity or infiltration, and the like.

The term "vaccine" refers to a composition that, when administered to a subject, induces an immune response. In some embodiments, the induced immune response provides protective immunity.

The term "antigen" or "Ag" is defined as a molecule that elicits an adaptive immune response. This immune response may involve the production of antibodies, or the activation of specific immunogenic cells, or both. Those skilled in the art will appreciate that any macromolecule, including nearly all proteins or peptides, can serve as an antigen. In addition, an antigen may be derived from recombinant or genomic DNA or RNA. Those skilled in the art will appreciate that any DNA or RNA comprising a nucleotide sequence or partial nucleotide sequence that encodes a protein that elicits an adaptive immune response therefore encodes an "antigen" as the term is used herein. Furthermore, it will be appreciated by those skilled in the art that an antigen need not be encoded solely by the full-length nucleotide sequence of a gene. It is apparent that the present invention includes, but is not limited to, the use of partial nucleotide sequences of more than one gene, and that these nucleotide sequences are arranged in various combinations to elicit the desired immune response. Moreover, those skilled in the art will appreciate that an antigen need not be encoded by a "gene" at all. It is apparent that an antigen can be generated synthetically or can be derived from a biological sample. Such a biological sample may include, but is not limited to, a tissue sample, a tumor sample, a cell, or a biological fluid.

As used herein, the term "vector" typically refers to a nucleic acid molecule capable of insertion and self-replication in a suitable host, transferring the inserted nucleic acid molecule into and/or between host cells. The vectors may include vectors primarily for inserting DNA or RNA into cells, vectors primarily for replicating DNA or RNA, and vectors primarily for the transcription and/or translation expression of DNA or RNA. The vectors also include those vectors having several of the aforementioned functions. The vector can be a polynucleotide that, when introduced into a suitable host cell, can be transcribed and translated into a polypeptide. Typically, by culturing suitable host cells containing the vector, the vector can produce a desired expression product.

The term "expression vector" refers to a vector comprising a recombinant polynucleotide, said recombinant polynucleotide containing an expression control sequence operably linked to a nucleotide sequence to be expressed. An expression vector contains sufficient cis-acting elements for expression; other elements for expression can be supplied by the host cell or in an in vitro expression system. Expression vectors include all those known by those known in the art, such as cosmids, plasmids (e.g., naked plasmids or those encapsulated in liposomes), RNA, and viruses (e.g., lentiviruses, retroviruses, adenoviruses, and adeno-associated viruses) that incorporate into the recombinant polynucleotide.

The mRNA can be monocistronic, bicistronic, or multicistronic mRNA. The bicistronic or multicistronic mRNA refers to an mRNA containing two or more coding regions.

In a specific embodiment of the present invention, the coding regions of the bicistronic or multicistronic mRNA can be separated by at least one Internal Ribosome Entry Site (IRES) sequence. Usable IRES sequences include, but are not limited to, those from picornaviruses (e.g., FMDV), pestiviruses (e.g., CFFV), poliovirus (e.g., PV), encephalomyocarditis virus (e.g., ECMV), foot-and-mouth disease virus (e.g., FMDV), hepatitis C virus (e.g., HCV), classical swine fever virus (e.g., CSFV), murine corneal leukoplakia virus (e.g., MLV), simian immunodeficiency virus (e.g., SIV), or cricket paralysis virus (e.g., CrPV), among others. The coding regions can also be separated by "2A linkers". The "2A linker" is a short peptide (18-25 amino acids) derived from viruses, also known as a "self-cleaving" peptide, which enables the production of multiple proteins from a single transcript, including but not limited to P2A, T2A, E2A, F2A, and the like.

Preferably, the mRNA is composed of several structural elements. That is, in addition to the coding region mentioned above, the mRNA can also include a 5' cap structure, a promoter, a 5' untranslated region (5' UTR), a signal peptide, a 3' untranslated region (3' UTR), and/or a polyadenylate tail (polyA) mRNA sequence. However, it is not limited to the aforementioned elements, and any elements necessary for the construction of mRNA fall within the scope of protection of the present invention.

The M protein is the membrane protein of FCoV, which can interact with other structural proteins to promote virion assembly. The N protein is the nucleocapsid (N) protein of FCoV, which can bind to the FCoV genome, package the viral genomic RNA into ribonucleoprotein complexes, and increase the efficiency of virus-like particle (VLP) assembly. The S protein is the spike (S) protein, which mediates virus attachment and entry into host cells, and can determine the host range. The S1 region of the S protein is responsible for binding to receptors on host cells, and the S2 region is responsible for the fusion of the virus with the host cell membrane. The E protein is the small envelope (E) protein, which participates in virus assembly and budding.

### Definitions of Treatment-Related Terminology

As used herein, the term "treatment" refers to achieving a desired pharmacological and/or physiological effect. The effect can be prophylactic, in terms of completely or partially preventing a disease or its symptoms, and/or can be therapeutic, in terms of partially or completely curing the disease and/or adverse effects attributable to the disease. As used herein, "treatment" encompasses treatment of a disease in a mammal, particularly a cat, and includes: (a) preventing the disease or condition from occurring in a subject who may be predisposed to the disease but has not yet been diagnosed; (b) inhibiting the disease, e.g., arresting its development; or (c) relieving the disease, e.g., causing regression of the symptoms associated with the disease. As used herein, "treatment" encompasses any administration of a medication or compound to a subject to treat, cure, alleviate, ameliorate, relieve, or inhibit the subject's disease, including, but not limited to, administering a medication containing the compound described herein to a subject in need thereof.

The terms "object", "individual", "patient", or "subject" include mammals (optionally added depending on the presence of a therapeutic use). Mammals include, but are not limited to, domesticated animals (e.g., cattle, sheep, cats, dogs, and horses), primates (e.g., humans and non-human primates such as monkeys), rabbits, and rodents (e.g., mice and rats).

"Prevention" referred to in the present invention refers to all actions that avoid symptoms or delay the progression of specific symptoms by administering the product of the present invention, whether before or after the disease begins to develop.

"Effective dose" referred to in the present invention refers to the amount or dosage of the product of the present invention that provides the desired therapeutic or preventive effect after administration of a single or multiple doses to a patient or organ.

As used herein, the term "pharmaceutical composition" typically refers to a unit dosage form and can be prepared by any one of the methods well known in the pharmaceutical field. All methods include the step of combining the active ingredient with a carrier which constitutes one or more accessory ingredients. Generally, a composition is prepared by uniformly and sufficiently combining the active compound with a liquid carrier, a finely divided solid carrier, or both.

The mRNA or composition of the present invention may further comprise a pharmaceutically acceptable excipient. The pharmaceutically acceptable excipient can be a carrier, diluent, adjuvant or nucleotide sequence encoding an adjuvant, solubilizer, binder, lubricant, suspending agent, transfection-promoting agent, or the like.

The transfection-promoting agents include, but are not limited to, surfactants such as immunostimulating complexes, Freund's incomplete adjuvants, Freund's complete adjuvants, aluminum hydroxide adjuvants, aluminum phosphate adjuvants, emulsion adjuvants, liposomal adjuvants, microbial adjuvants, LPS analogs (e.g., monophosphoryl lipid A), muropeptide, quinone analogs, squalene, hyaluronic acid, lipids, calcium ions, viral proteins, cations, polycations (e.g., poly-L-glutamate (LGS)), nanoparticles, or other known transfection-promoting agents.

The nucleotide sequence encoding an adjuvant is a nucleotide sequence encoding at least one adjuvant selected from the group consisting of: GM-CSF, IL-17, IFNγ, IL-15, IL-21, anti-PD1/2, Lactoferrin, Protamine, IL-1, IL-2, IL-3, IL-4, IL-5, IL-6, IL-7, IL-8, IL-9, IL-10, IL-12, INF-α, INF-γ, Lymphotoxin-α, hGH, MCP-1, MIP-1a, MIP-1p, IL-8, RANTES, L-selectin, P-selectin, E-selectin, CD34, GlyCAM-1, MadCAM-1, LFA-1, VLA-1, Mac-1, pl50.95, PECAM, ICAM-1, ICAM-2, ICAM-3, CD2, LFA-3, M-CSF, CD40, CD40L, vascular growth factor, fibroblast growth factor, nerve growth factor, vascular endothelial growth factor, Apo-1, p55, WSL-1, DR3, TRAMP, Apo-3, AIR, LARD, NGRF, DR4, DR5, KILLER, TRAIL-R2, TRICK2, DR6, Caspase ICE, Fos, c-jun, Sp-1, Ap-1, Ap-2, p38, p65Rel, MyD88, IRAK, TRAF6, IkB, inactive NIK, SAP K, SAP-1, JNK, NFkB, Bax, TRAIL, TRAILrec, TRAILrecDRC5, TRAIL-R3, TRAIL-R4, RANK, RANK LIGAND, Ox40, Ox40LIGAND, NKG2D, MICA, MICB, NKG2A, NKG2B, NKG2C, NKG2E, NKG2F, TAP1, TAP2, and functional fragments thereof.

As used herein, the term "pharmaceutically acceptable excipients" can include any solvents, solid excipients, diluents, or other liquid excipients, etc., suitable for the particular target dosage form. Their use is within the contemplation of the present invention, except to the extent that any conventional excipient is incompatible with the compounds of the present invention, e.g., by producing any undesirable biological effect or interacting in a deleterious manner with any other component(s) of the pharmaceutically acceptable composition.

### Delivery System

The terms "transformation," "transfection," "transduction," and "introduction" have their commonly understood meanings in the art, i.e., the process of introducing exogenous nucleic acids (DNA, RNA, etc.) into a host. Methods for said transformation, transfection, and transduction include any method for introducing nucleic acids into cells, including but not limited to electroporation, calcium phosphate (CaP_{O4}) precipitation, calcium chloride (CaCl₂) precipitation, microinjection, polyethylene glycol (PEG) method, DEAE-dextran method, cationic liposome method, and lithium acetate-DMSO method.

The composition of the present invention can be a lipid, lipid complex, or lipid nanoparticle (LNP). The lipid can be a lipid prepared in the following form: 1,2-dioleyloxy-N, N-dimethylaminopropane (DODMA) lipid, 1,2-dilinoleyloxy-3-dimethylaminopropane (DLin-DMA), 2,2-dilinoleyl-4-(2-dimethylaminoethyl)-[1,3]-dioxolane (DLin-KC2-DMA) lipid. The lipid complex or lipid nanoparticle can be formed from lipids selected from the group consisting of: DLin-DMA, DLin-K-DMA, 98N12-5, C12-200, DLin-MC3-DMA, DLin-KC2-DMA, DODMA, PLGA, PEG, PEG-DMG, pegylated lipids, and aminoalcohol lipids.

"LNP" referred to in the present invention refers to lipid nanoparticle.

The effective amount of the mRNA, mRNA vaccine, protein or polypeptide vaccine, vector vaccine, conjugate, or pharmaceutical composition of the present invention can vary depending on the mode of administration, the severity of the disease to be treated, and the like. The selection of a preferred effective dose can be determined by those skilled in the art based on various factors (e.g., through clinical trials). Said factors include, but are not limited to: the pharmacokinetic parameters of the active ingredient such as bioavailability, metabolism, half-life, etc.; the severity of the disease to be treated of the patient, the patient's weight, the patient's immune status, the route of administration, and the like. For example, depending on the exigencies of the therapeutic situation, several separate doses may be administered daily, or the dose may be reduced proportionally.

### Dosage Forms

The mRNA, mRNA vaccine, protein or polypeptide vaccine, vector vaccine, or pharmaceutical composition of the present invention can be incorporated into pharmaceuticals suitable for parenteral administration (e.g., intravenous, subcutaneous, intraperitoneal, intramuscular). These pharmaceuticals can be prepared in various forms, such as liquid, semi-solid, and solid dosage forms, including but not limited to liquid solutions (e.g., injection and infusion solutions) or lyophilized powders.

### Routes of Administration

The term "administration" refers to introducing a predetermined amount of a substance into a patient by a suitable route. The antibody or antigen binding fragment, recombinant protein, multi-specific antibody, conjugate, or pharmaceutical composition of the present invention can be administered by any conventional route, provided it can reach the target tissue. Various modes of administration are contemplated, including peritoneal, intravenous, intramuscular, subcutaneous, intranasal, or inhalation, etc., but the present invention is not limited to these exemplified routes of administration. Preferably, the composition of the present invention is administered by intramuscular, intravenous, or subcutaneous injection, particularly by intramuscular injection.

The inhalation administration mode is selected from at least one of powder inhalation and nebulization inhalation.

According to the embodiments of the present application, the number of administrations is 1 to 20 times, preferably 1 to 10 times, more preferably 1, 2, 3, 4, 5, or 6 times.

### First Aspect of the Present Invention provides DNA Element

A DNA element, obtained by linking nucleotides comprising at least one element selected from M protein, N protein, and S protein of an FCoV virus strain;
Further, the elements can be linked with or without linker peptides, where when the number of linker peptides is greater than one, the plurality of linker peptides can be the same or different, but are preferably the same;
Further, the number of said elements is preferably 1-3 or 2-3, and can be 1, 2, or 3;
Further, the structure of said recombinant DNA molecule can be a single element of M protein, N protein, or S protein; or can be the sequential linked nucleotide sequences of dual elements selected from M+N, N+M, M+S, S+M, N+S, S+N; or can be the sequential linkage of nucleotide sequences of triple elements selected from N+M+S, N+S+M, S+N+M, M+N+S, M+S+N, S+M+N; wherein said "sequential" refers to the order from the 5' terminal to the 3' terminal.

Merely as examples, preferably, sequential linkage combinations of M+N, N+M, N+M+S, N+S+M, S+N+M, M+N+S, M+S+N, S+M+N can be selected; more preferably, sequential linkage combinations of N+M, N+M+S, N+S+M can be selected.

Further, the elements within said recombinant DNA molecule can be linked via linker peptides. Said linker peptide can be an internal ribosome entry site (IRES) or a self-cleaving 2A peptide (2A), wherein the 2A linker peptide includes P2A linker peptide, T2A linker peptide, E2A linker peptide, and F2A linker peptide, where P2A linker peptide or T2A linker peptide is preferably selected.

Wherein, said S protein can be any one of full-length S, S1 protein, or S2 protein, but full-length S and S2 protein are preferably selected;
Said DNA element is preferably at least one selected from the group consisting of: M+N(T2A), N+M(T2A), M+N(P2A), N+M(P2A), N+M+S(T2A), N+S+M(T2A), N+M+S2(T2A), N+S2+M(T2A), N+M+S(P2A), N+S+M(P2A), N+M+S2(P2A), N+S2+M(P2A).

More preferably, said nucleotide and protein sequences can be as shown in Table 1, as well as sequences having 66% or more, 75% or more, 85% or more, or 95% or more identity and capable of achieving the same function.

For example, the DNA sequences of said elements are set forth in SEQ ID NOs: 7-23, and the amino acid sequences are set forth in SEQ ID NOs: 60-76; the DNA sequences of said linker peptides are set forth in SEQ ID NOs: 4-5, and the amino acid sequences are set forth in SEQ ID NOs: 58-59; as well as sequences having 66% or more, 75% or more, 85% or more, or 95% or more identity and capable of achieving the same function.

### Second Aspect of the Present Invention provides Recombinant Nucleic Acid Molecule

A recombinant nucleic acid molecule, with its structure formed by linking a plurality of elements consisting of 5' untranslated region, a Kozak sequence region, at least one antigen-coding region, a 3' untranslated region, and polyA, where the order is not particularly limited, but sequential linkage from 5' to 3' is preferred;

Said antigen-coding region is the recombinant DNA molecule combined using the plurality of elements described above; said nucleic acid molecule can be a DNA or RNA molecule, and also includes various specific subtypes.

Said polyA is formed by linking a varying number of A, including the linkage of 1-200 A. Specifically, it can be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200 A being linked together, preferably 50-150 A being linked together, and more preferably 100 A being linked together.

Further, a promoter can be further included at the 5' terminal of the 5' untranslated region; the promoter of the template DNA can be a promoter capable of initiating translation, including but not limited to T7, CMV, or SP6 promoters;
Further, a signal peptide coding region can be further included at the 3' terminal of the 5' untranslated region, where said signal peptide can be selected from TPA signal peptide, IgE signal peptide, or its native signal peptide, etc. It is noteworthy that the DNA molecule may or may not contain a signal peptide. In fact, the absence of a signal peptide does not affect the translation and expression of the molecule;
The amino acid sequence of the linker is, for example, at least one selected from 2A linker peptides (P2A, T2A, E2A, and F2A) and IRES. Preferably, the DNA sequences of the linker peptides are set forth in SEQ ID NOs: 4-5, and the amino acid sequences are set forth in SEQ ID NOs: 58-59;
Said nucleotide and protein sequences are as shown in Table 1 and Table 2, as well as sequences having 66% or more, 75% or more, 85% or more, or 95% or more identity and capable of achieving the same function.

For example, for a recombinant DNA molecule: the 5' untranslated region sequence is set forth in SEQ ID NO: 1; the Kozak sequence is set forth in SEQ ID NO:2; the 3' untranslated region is set forth in SEQ ID NO:3; the polyA sequence is set forth in SEQ ID NO:6; the recombinant DNA molecule has a DNA sequence set forth in SEQ ID NOs: 24-40; as well as sequences having 66% or more, 75% or more, 85% or more, or 95% or more identity and capable of achieving the same function.

An mRNA molecule obtained by transcription of the aforementioned DNA molecule, preferably, can be the mRNA set forth in SEQ ID NOs: 41-57.

### Third Aspect of the Present Invention Relates to Structure Conatining Aforementioned DNA Element, Recombinant Nucleic Acid Molecule

A recombinant vector comprising the aforementioned recombinant DNA element or recombinant DNA molecules, wherein said vector can use vectors known in the art, including but not limited to, pUC57, pTZ19R, PMD18T, PQE30, pUC18, and pUC19, etc.;
An mRNA, obtained by transcription of the aforementioned recombinant DNA molecule;
A protein, obtained by translation of the aforementioned recombinant DNA molecule; as well as various DNA molecules capable of expressing said protein, the meaning of which is that the scope of protection of the present invention encompasses all DNA molecules that can be deduced through the codon expression patterns, not merely the DNA sequences shown in the embodiments.

A recombinant cell, to be introduced into the aforementioned recombinant vector; said cells include, but are not limited to, human-derived or animal-derived cell lines, such as CRFK feline kidney cells, PG-4 (S+L-) feline brain cells, F81 feline kidney cells, FCA-S1 feline skin cells, CATK1 feline kidney cells, MDCK (NBL-2) canine kidney cells, SCBN canine duodenal cells, DH82 canine renal histiocytosis cells, HEK293T, HEK293, BHK, CHO, 3T3, NS0, Hela, HT-1080, PERC6, CAP, HKB-11, Huh-7, etc.;
Said introduction methods include, but are not limited to, liposome transfection, microvesicle transfection, exosome transfection, electroporation, nanocarrier transfection, chemical transfection using transfection reagents; Nanocarriers include lipids, polymers, or lipid-polymer hybrids.

A delivery system for delivering the aforementioned recombinant DNA molecule, recombinant nucleic acid vector, or mRNA.

Said mRNA may contain appropriate modifications, including but not limited to, pseudouridine (Ψ), N1-methylpseudouridine (m1Ψ), 5-methoxyuridine (5moU), N6-methyladenosine (m6A), 5-methylcytidine (5m5C), etc.

### Fourth Aspect of the Present Invention Provides Pharmaceutical Composition

Said composition comprises the aforementioned DNA element, recombinant DNA molecules, mRNA, proteins, recombinant cells, recombinant vectors, and delivery systems.

Further, said composition can be a pharmaceutical composition. For example, it can be a vaccine composition, a therapeutic pharmaceutical composition. More preferably, it can be a DNA vaccine, mRNA vaccine, protein vaccine, therapeutic protein, therapeutic nucleic acid composition, and the like.

### Main Ingredients:

The DNA elements, recombinant nucleic acid molecules (DNA, RNA), mRNA, proteins, etc., provided in the present invention can be individual components, or can be mixed in different proportions; the components used for mixing can be the same or different;
Merely as examples, an mRNA vaccine for the N protein can be mixed with an mRNA vaccine for the M protein; or an mRNA vaccine for the N protein, an mRNA vaccine for the M protein, and an mRNA vaccine for the S2 protein can be mixed; or mRNA vaccines obtained from the expression of recombinant vectors combining the aforementioned plurality of elements can be mixed in certain proportions.

When the components are divided into two types, the mixing ratio can be various ratios such as 1:1, 2:1, 3:1, 1:2, 1:3, 1:4, 4:1, etc., but a 1:1 equal ratio mixture is preferred. When the components are divided into three types, the mixing ratio can be 1:1:1, 1:1:2, 2:1:1, 1:2:1, etc., but a 1:1:1 equal ratio mixture is preferred.

### Auxiliary Ingredients:

Based on the active ingredients, optionally, said pharmaceutical composition further comprises one or more pharmaceutically acceptable excipients, carriers, adjuvants, or vehicles. Examples of excipients, carriers, adjuvants, and vehicles include, but are not limited to, antiadherents, binders, coatings, compression aids, disintegrants, dyes, glidants, emulsifiers, fillers (dilutents), film formers or coatings, flavoring agents, fragrances, flow enhancers, lubricants, adsorbents, suspending or dispersing agents, or sweeteners. Exemplary excipients, carriers, adjuvants, and vehicles include, but are not limited to: butylated hydroxytoluene (BHT), calcium carbonate, calcium phosphate (monohydrogen), calcium stearate, croscarmellose sodium, crospovidone, citric acid, crospovidone, cysteine, ethyl cellulose, gelatin, hydroxypropyl cellulose, hydroxypropyl methylcellulose, lactose, magnesium stearate, maltitol, mannitol, methionine, methyl cellulose, methyl paraben, microcrystalline cellulose, polyethylene glycol, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E, vitamin C, and xylitol.

The pharmaceutical composition can be prepared in the form of an injection formulation or an oral formulation. The injection formulations are classified according to their physical state, including liquid injections, powders for injection, and tablets for injection; or are classified according to injection site, including intradermal injection, subcutaneous injection, intramuscular injection, intravenous injection, and spinal cavity injection. The preferred solvent for the injection formulation includes injection water or physiological saline.

Formulations for oral use include tablets comprising the active ingredient mixed with non-toxic pharmaceutically acceptable excipients. These excipients can be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binders (e.g., sucrose, glucose, sorbitol, acacia, alginic acid, sodium alginate, gelatin, starch, pregelatinized starch, microcrystalline cellulose, magnesium aluminum silicate, carboxymethyl cellulose sodium, methyl cellulose, hydroxypropyl methylcellulose, ethyl cellulose, polyvinylpyrrolidone, or polyethylene glycol); and lubricants, glidants, and anti-adherents (e.g., magnesium stearate, zinc stearate, stearic acid, silica, hydrogenated vegetable oil, or talc). Formulations for oral use can also be in the form of chewable tablets or hard gelatin capsules, wherein the active ingredient is mixed with inert solid diluents (e.g., potato starch, lactose, microcrystalline cellulose, calcium carbonate, calcium phosphate, or kaolin), or in the form of soft gelatin capsules, wherein the active ingredient is mixed with a water or oil medium (e.g., peanut oil, liquid paraffin, or olive oil). Powders, granules, and pellets can be prepared in conventional manners using the ingredients mentioned above for tablets or capsules, using equipment such as mixers, fluidized bed apparatus, or spray drying equipment.

Other pharmaceutically acceptable excipients for oral formulations include, but are not limited to, coloring agents, flavoring agents, plasticizers, humectants, and buffering agents. Formulations for oral use can also be in the form of chewable tablets or hard gelatin capsules, wherein the active ingredient is mixed with inert solid diluents (e.g., potato starch, lactose, microcrystalline cellulose, calcium carbonate, calcium phosphate, or kaolin), or in the form of soft gelatin capsules, wherein the active ingredient is mixed with a water or oil medium (e.g., peanut oil, liquid paraffin, or olive oil). Powders, granules, and pellets can be prepared in conventional manners using the ingredients mentioned above for tablets or capsules, using equipment such as mixers, fluidized bed apparatus, or spray drying equipment.

### Administration Methods

In some embodiments, administration includes intramuscular, intravenous (e.g., in the form of a sterile solution and in a solvent system suitable for intravenous use), intradermal, intra-arterial, intraperitoneal, intralesional, intracranial, intra-articular, intraprostatic, intrapleural, intratracheal, intranasal, intravitreal, intravaginal, intrarectal, topical, intratumoral, intraperitoneal, subcutaneous, subconjunctival, intracapsular, transmucosal, intrapericardial, intraumbilical, intraocular, transoral (e.g., tablets, capsules, caplets, gelcaps, or syrups), topical (e.g., in the form of creams, gels, lotions, or ointments), local, and the like. The composition described herein is administered by inhalation, by injection, or by infusion (e.g., continuous infusion in the form of a cream or lipid composition, local perfusion by direct soaking of target cells, cannulation, lavage).

### Liposome delivery

The composition may further comprise a cationic or polycationic compound, wherein said cationic or polycationic compound is free or complexed with the mRNA. To enhance the stability of the composition, the form where the cationic or polycationic compound is complexed with the mRNA is selected.

Preferably, the composition further comprises lipids. Preferably, said lipids include, but are not limited to, lipids that promote self-assembly to form virus-sized particles (~100 nm), lipids that promote the release of mRNA from endosomes into the cytoplasm, lipids that support the phospholipid bilayer structure, or lipids that function as stabilizers.

More preferably, to increase the half-life of the LNP, said lipids may further comprise PEGylated lipids.

In a specific embodiment of the present invention, said lipids comprise cationic lipids, PEGylated lipids, cholesterol, and/or a phospholipids.

The liposomal structure can be obtained by mixing the phospholipid MC3, distearoylphosphatidylcholine (DSPC), cholesterol, and lipid-polyethylene glycol (e.g., DMG-PEG2000) in a certain ratio with ethanol, for example, mixed with ethanol at a molar ratio of 50:10:38.5:1.5.

### Fifth Aspect of the Present Invention Provides Method for Preparing a Vaccine

A method for preparing an mRNA vaccine, comprising the steps of:
(1) DNA template synthesis
(2) mRNA transcription
   performing in vitro transcription of mRNA
(3) mRNA capping,
   said step comprising: mRNA dissolution and dilution, a denaturation step, and preparation of a capping reaction system;
   the dissolution and dilution step involves appropriately diluting the recovered mRNA mentioned above with RNase-free water;
   the denaturation step may employ the method of heating followed by cooling, e.g., heating at 65°C for 5 minutes, then immediately placing on ice for 5 minutes;
   the capping reaction involves adding the denatured RNA to a capping enzyme for incubation.
(4) mRNA purification step
   said purification method includes but is not limited to: oligo- (dT)-cellulose column chromatography, oligo- (dT)-cellulose liquid-phase centrifugation, and oligo-(dT)-magnetic bead method, etc.; Electrophoresis, including but not limited to denaturing gel electrophoresis such as denaturing agarose gel electrophoresis, capillary electrophoresis, and the like.

### Sixth Aspect of the Present Invention Provides Preventive and Therapeutic Uses

Use of the aforementioned DNA elements, recombinant DNA molecules, mRNA, recombinant vectors, recombinant cells, or pharmaceutical compositions for the treatment and/or prevention of animal coronavirus infection diseases, and in the preparation of medications for the treatment and/or prevention of diseases caused by animal coronavirus infections.

Further, said animal coronavirus infection disease is a disease caused by enteric coronavirus infection or infectious peritonitis virus infection;
a method for treating and/or preventing an animal coronavirus infection disease, said method comprising administering the aforementioned DNA elements, recombinant nucleic acid molecules, mRNA, recombinant cells, or pharmaceutical compositions to a subject in need of treatment or prevention;
said administration is single or multiple administrations, where the interval between two administrations can be 18-26 days, such as 18, 19, 20, 21, 22, 23, 24, 25, 26 days, preferably 20, 21, 22, 23, 24 days, and more preferably 22 days.

The medication administered each time can be the same or different;
The administration dose is 1-50 µg/kg, preferably 1-30 µg/kg, and more preferably 1-20 µg/kg;
Further, the infected subject is preferably a mammal, including but not limited to humans, cats, dogs, mice, horses, cattle, sheep, pigs, monkeys, etc.; the inventors have used cell lines from the aforementioned mammals to establish validation models and conducted in vivo challenge experiments in animals, demonstrating universality in mammals and the effectiveness of prevention and treatment.

Further, it can be preferably used for preventing and/or treating Feline Infectious Peritonitis (FIP) caused by Feline Coronavirus (FCoV);
According to embodiments of the present application, the number of administrations is 1 to 20 times, preferably 1 to 10 times, and more preferably 1, 2, 3, 4, 5, or 6 times.

### Seventh Aspect of the Present Invention Provides Variant Validation

To further verify the technical effects, the inventors conducted validation on a plurality of variants of the M protein, N protein, full-length S, S1 protein, S2 protein, and the recombinant DNA molecules and protein structures formed by combining different elements described in the present invention based on the sequences disclosed herein. Based on at least 66%, 67%, 70%, 75%, 80%, 81%, 82%, 83%, 84%, 85%, 86%, 87%, 88%, 89%, 90%, 91%, 92%, 93%, 94%, 95%, 96%, 97%, 98%, 99%, 99.5%, 99.9% identity, for example, validation of introns of different types and sources, validation of spacers of different lengths, etc. were performed. While ensuring homology above 66% through truncation, mutation, segmentation and other methods, and using the validation methods described in the following disclosure and specific embodiments, recombinant nucleic acid molecules were first synthesized, prepared into mRNA vaccines in vitro, and validated using the same methods as in the examples at various cellular and animal levels. The results demonstrated that the same technical effects could be achieved, thus supporting a scope of protection for identity above 66%. More preferably, the scope of protection can be for identity above 75%, even more preferably above 85%, and most preferably above 95%.

The validation method for said variants can involve comparing the new sequence with the query sequence to determine the percentage of sequence identity. Standard methods commonly used for comparing the similarity and amino acid positions of two nucleic acids or polypeptides can be used for this comparison. Computer programs, such as BLAST or FASTA, for example, can align two polypeptides to achieve the best alignment of their respective amino acids (which can be along the full length of one or both sequences, or along a predetermined portion of one or both sequences). Such programs provide "default" gap opening penalties and "default" gap extension penalties, and a scoring matrix such as PAM250 (a standard scoring matrix; see Dayhoff et al., Atlas of Protein Sequence and Structure, Vol. 5, Supp.3 (1978)) or BLOSUM scoring matrices can be used in conjunction with the computer program. The percent identity is then calculated.

### BRIEF DESCRIPTION OF THE DRAWINGS

FIG. 1 is an electrophoretogram of mRNA integrity assessed by 1% formaldehyde-denaturing agarose gel electrophoresis;
FIG. 2 is a graph showing the analysis of mRNA expression levels in cells;
FIG. 3 is a graph showing the analysis of mRNA expression levels in cells;
FIG. 4 shows the detection of binding antibody and neutralizing antibody levels by ELISA;
FIG. 5 shows the results of antibody titer measurement experiments;
FIG. 6A shows FCoV detection results; FIG. 6B shows body temperature monitoring; FIG. 6C shows body weight measurement results; FIG. 6D shows N and S2 protein binding antibody detection; FIG. 6E shows neutralizing antibody detection; FIG. 6F shows grouping information;
FIG. 7A shows observations of reactions post-immunization; FIGS. 7B and 7C show body temperature and body weight post-immunization, respectively;
FIGS. 8A and 8B show binding antibody detection; FIG. 8C shows neutralizing antibody detection; FIG. 8D shows lymphocyte proliferation assay;
FIG. 9A shows body temperature monitoring post-challenge; FIG. 9B shows body weight monitoring post-challenge; and
FIG. 10A shows survival rate statistics; FIG. 10B shows the viral titer detection results of Feline Infectious Peritonitis Virus in major organs of animals from each group.

### DETAILED DESCRIPTION

### Example 1: Synthesis of mRNA Sequences

### 1) Acquisition of Target Genes:

Gene Element Sources: The following sequence were retrieved from the GenBank database: DNA sequences for the M protein (MW030108.1:26093-26881), N protein (MW030108.1:26894-28024), and S protein (MW030109.1:20437-24795) of the FCoV strain rQS-79; the 5'-UTR sequences (derived from the feline DNAH2, feline hemoglobin, or feline albumin genes), and the 3'-UTR sequences (derived from the feline hemoglobin or feline albumin genes), whrein the S protein includes the full-length S protein, S1 protein, and S2 protein. Specific sequences are listed in Table 1.

Recombinant Gene Combinations: The element sequences (as shown in Table 1) were combined. The structure of each combination from 5' to 3' terminal is: 5'-UTR (SEQ ID NO:1) + Kozak (SEQ ID NO:2) + Target DNA Sequence (SEQ ID NO:7-23) + 3'-UTR (SEQ ID NO:3) + 100polyA (SEQ ID NO:6). DNA templates were synthesized by a gene synthesis company, and the recombinant plasmids were obtained by ligating the DNA templates into the pUC57 vector (GenScript, SD1176). Wherein, different target DNA sequences within each combination were linked by linkers. Two different 2A peptide linkers were selected, with amino acid sequences respectively being T2A peptide: GSGEGRGSLLTCGDVEENPGP (SEQ ID NO.58), and P2A peptide: GSGATNFSLLKQAGDVEENPGP (SEQ ID NO.59).

**Table 1**

| No. | Element Name | DNA Sequence No. | Amino Acid Sequence No. |
|---|---|---|---|
| 1 | 5'-UTR | SEQ ID NO:1 | |
| 2 | Kozak Sequence | SEQ ID NO:2 | |
| 3 | 3'-UTR Sequence | SEQ ID NO:3 | |
| 4 | T2A Peptide Sequence | SEQ ID NO:4 | SEQ ID NO:58 |
| 5 | P2A Peptide Sequence | SEQ ID NO:5 | SEQ ID NO:59 |
| 6 | 100 polyA Sequence | SEQ ID NO:6 | |
| 7 | M | SEQ ID NO:7 | SEQ ID NO:60 |
| 8 | N | SEQ ID NO:8 | SEQ ID NO:61 |
| 9 | S full-length | SEQ ID NO:9 | SEQ ID NO:62 |
| 10 | S1 | SEQ ID NO:10 | SEQ ID NO:63 |
| 11 | S2 | SEQ ID NO:11 | SEQ ID NO:64 |
| 12 | M+N (T2A) | SEQ ID NO:12 | SEQ ID NO:65 |
| 13 | N+M (T2A) | SEQ ID NO:13 | SEQ ID NO:66 |
| 14 | M+N(P2A) | SEQ ID NO:14 | SEQ ID NO:67 |
| 15 | N+M(P2A) | SEQ ID NO:15 | SEQ ID NO:68 |
| 16 | N+M+S(T2A) | SEQ ID NO:16 | SEQ ID NO:69 |
| 17 | N+S+M(T2A) | SEQ ID NO:17 | SEQ ID NO:70 |
| 18 | N+M+S2(T2A) | SEQ ID NO:18 | SEQ ID NO:71 |
| 19 | N+S2+M(T2A) | SEQ ID NO:19 | SEQ ID NO:72 |
| 20 | N+M+S(P2A) | SEQ ID NO:20 | SEQ ID NO:73 |
| 21 | N+S+M(P2A) | SEQ ID NO:21 | SEQ ID NO:74 |
| 22 | N+M+S2(P2A) | SEQ ID NO:22 | SEQ ID NO:75 |
| 23 | N+S2+M(P2A) | SEQ ID NO:23 | SEQ ID NO:76 |

The recombinant plasmids shown as 5'-UTR+Kozak+target DNA sequence+3'-UTR+100 polyA structure (Table 2, SEQ ID NO: 24-40) were transduced into competent cells DH5α, and a large number of bacterial cells were obtained through cloning screening and amplification. Large quantities of recombinant plasmids were obtained using an Endo-Free Plasmid Maxi Kit (Tiangen, DP117). The plasmids were linearized by digestion with restriction enzymes, and the templates for mRNA synthesis were recovered using a recovery kit.

**Table 2**

| No. | Recombinant Nucleic Acid Molecule Name | DNA Sequence No. | mRNA Sequence No. |
|---|---|---|---|
| 1 | 5'-UTR+Kozak +M+3'-UTR+100 polyA | SEQ ID NO:24 | SEQ ID NO:41 |
| 2 | 5'-UTR+Kozak +N+3'-UTR+100 polyA | SEQ ID NO:25 | SEQ ID NO:42 |
| 3 | 5'-UTR+Kozak +S+3'-UTR+100 polyA | SEQ ID NO:26 | SEQ ID NO:43 |
| 4 | 5'-UTR+Kozak +S1+3'-UTR+100 polyA | SEQ ID NO:27 | SEQ ID NO:44 |
| 5 | 5'-UTR+Kozak +S2+3'-UTR+100 polyA | SEQ ID NO:28 | SEQ ID NO:45 |
| 6 | 5'-UTR+Kozak +M+N+3'-UTR+100 polyA(T2A) | SEQ ID NO:29 | SEQ ID NO:46 |
| 7 | 5'-UTR+Kozak +N+M+3'-UTR+100 polyA(T2A) | SEQ ID NO:30 | SEQ ID NO:47 |
| 8 | 5'-UTR+Kozak +M+N+3'-UTR+100 polyA(P2A) | SEQ ID NO:31 | SEQ ID NO:48 |
| 9 | 5'-UTR+Kozak+N+M+3'-UTR+100 polyA(P2A) | SEQ ID NO:32 | SEQ ID NO:49 |
| 10 | 5'-UTR+Kozak+N+M+S+3'-UTR+100 polyA(T2A) | SEQ ID NO:33 | SEQ ID NO:50 |
| 11 | 5'-UTR+Kozak+N+S+M+3'-UTR+100 polyA(T2A) | SEQ ID NO:34 | SEQ ID NO:51 |
| 12 | 5'-UTR+Kozak+N+M+S2+3'-UTR+100 polyA(T2A) | SEQ ID NO:35 | SEQ ID NO:52 |
| 13 | 5'-UTR+Kozak+N+S2+M+3'-UTR+100 polyA(T2A) | SEQ ID NO:36 | SEQ ID NO:53 |
| 14 | 5'-UTR+Kozak+N+M+S+3'-UTR+100 polyA(P2A) | SEQ ID NO:37 | SEQ ID NO:54 |
| 15 | 5'-UTR+Kozak+N+S+M+3'-UTR+100 polyA(P2A) | SEQ ID NO:38 | SEQ ID NO:55 |
| 16 | 5'-UTR+Kozak+N+M+S2+3'-UTR+100 polyA(P2A) | SEQ ID NO:39 | SEQ ID NO:56 |
| 17 | 5'-UTR+Kozak+N+S2+M+3'-UTR+100 polyA(P2A) | SEQ ID NO:40 | SEQ ID NO:57 |

### 2) mRNA Transcription

mRNA was synthesized in vitro using an mRNA in vitro Transcription Kit (NEB, E2050). The reaction system is shown in Table 3 below:

**Table 3**

| Component | Volume |
|---|---|
| Nuclease-Free Water | XµL |
| NTP Buffer Mix | 10µL |
| DNA Template | XµL(1µg) |
| T7 RNA Polymerase Mix | 2µL |
| Total Reaction Volume | 20µL |

Reaction was carried out at 37°C for 2 hours. The mRNA was recovered using an mRNA Recovery Kit.

### 3) mRNA Capping:

The mRNA capping reaction was performed using an mRNA Capping Kit (NEB, M2080, M0366), capable of adding a Cap1 structure. The reaction system was as follows:
a) 10 µg of the aforementioned recovered mRNA was diluted to 13.5 µL with RNase-free water;
b) Heating was performed at 65°C for 5 minutes, followed by immediately placing on ice for 5 minutes; and
c) The reaction system was prepared according to Table 4 below:

**Table 4**

| Component | Volume |
|---|---|
| Denatured RNA | 13.5µL |
| 10×Capping Buffer | 2µL |
| 10 mM GTP | 1µL |
| 20 mM SAM | 1µL |
| Capping Enzyme | 1µL |
| 2'-O-Methyltransferase | 1µL |
| RNase Enzyme Inhibitor | 0.5µL |
| Total Reaction Volume | 20µL |

d) Incubation was carried out at 37°C for 1 hour;
e) The capped mRNA was purified.

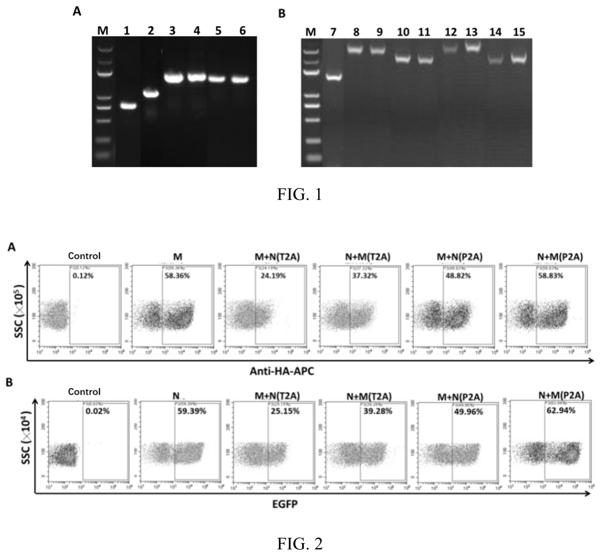

The integrity and size of the mRNA were assessed by 1% formaldehyde-denaturing agarose gel electrophoresis. The electrophoretogram is shown in FIG. 1. Lanes 1-6 in FIG. 1A correspond to the mRNA of target mRNA sequences M, N, M+N (T2A), N+M (T2A), M+N (P2A), N+M (P2A) (i.e., SEQ ID NO:41, 42, 46-49) as shown in Table 2, whose corresponding target DNA sequences are SEQ ID NO:24, 25, 29-32. Lanes 7-15 in FIG. 1B correspond to the mRNA of target mRNA sequences S2, N+M+S (T2A), N+S+M (T2A), N+M+S2 (T2A), N+S2+M (T2A), N+M+S (P2A), N+S+M (P2A), N+M+S2 (P2A), N+S2+M (P2A) (i.e., SEQ ID NO:45, 50-57) as shown in Table 2, whose corresponding target DNA sequences are SEQ ID NO:28, 33-40. The test results show that the in vitro synthesized mRNA has the correct molecular weight and is essentially undegraded.

### Example 2: Expressibility at Cellular Level

To test the expression levels of some mRNA combinations in Table 2, including M, N, M+N (T2A), N+M (T2A), M+N (P2A), N+M (P2A), in cells, experiments were conducted using Feline Kidney Cells (CRFK). According to the sequence combinations in Table 2, an HA tag was fused to the C-terminus of the M protein, and an EGFP tag was fused to the C-terminus of the N protein. CRFK cells were seeded in a 24-well plate and cultured for 18 hours. The medium was replaced with serum-free Opti-MEM medium 1 hour before transfection. The mRNA of M, N, M+N (T2A), N+M (T2A), M+N (P2A), N+M (P2A) combinations were transfected using lipofectamine 2000. After 6 hours of culture, the medium was replaced with fresh medium, and culture continued for 42 hours. The cell supernatant was removed, cells were digested and resuspended in PBS containing 1% FBS. APC-conjugated anti-HA antibody was added, and the cells were incubated at 4°C for 1 hour. The cells were then washed twice with cold PBS containing 1% FBS, and resuspended in 200 µL of PBS containing 1% FBS. Subsequently, FACS analysis was performed using a flow cytometer (Beckman Coulter, CytoFLEX).

The results are shown in FIG. 2, where FIG. 2A shows the expression level of the M protein, and FIG. 2B shows the expression level of the N protein. The expression levels of the P2A peptide-linked groups were significantly higher than those of the T2A peptide-linked groups. And, among the P2A peptide-linked combinations, the expression levels of the two proteins in the N+M tandem combination were significantly higher than those in the M+N tandem combination, and were comparable to the expression levels of the individual M and N proteins.

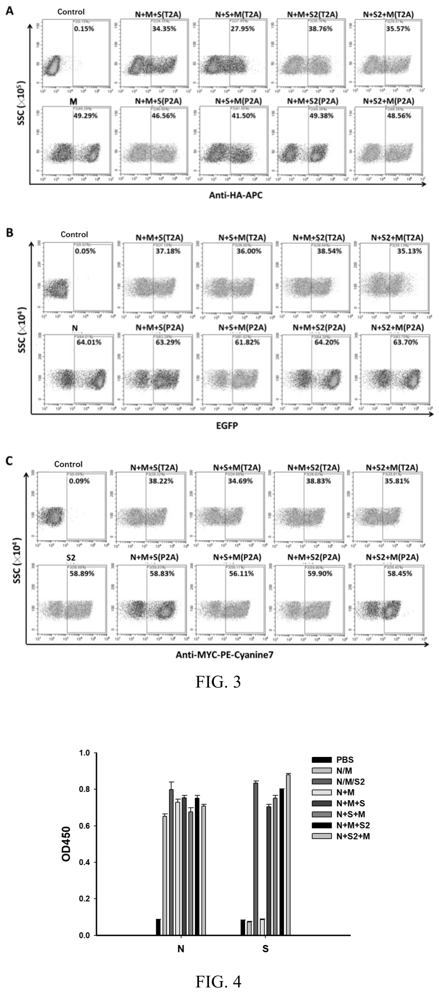

From the above results, it can be concluded that placing the N protein upstream of the 2A peptide is more conducive to the expression of both proteins upstream and downstream of the linker peptide. Therefore, we designed the combinations listed in Table 2 with target RNA sequences being S2, N+M+S (T2A), N+S+M (T2A), N+M+S2 (T2A), N+S2+M (T2A), N+M+S (P2A), N+S+M (P2A), N+M+S2 (P2A), N+S2+M (P2A) (i.e., SEQ ID NO:45, 50-57), where the C-terminus of S2 protein was fused with a MYC tag. The expression levels were detected using the flow cytometry experimental method described above, with the S2 protein detected using a PE-Cyanine7- conjugated anti-MYC antibody. The detection results are shown in FIG. 3.

Similar to the results in FIG. 2, the expression levels of the P2A peptide-linked groups were significantly higher than those of the T2A peptide-linked groups. And, among the P2A peptide-linked combinations, there were no significant differences in the expression levels of the M, N, and S proteins among the different tandem combinations, all being comparable to the expression levels of the individual proteins.

### Example 3: Preparation Process of mRNA Vaccine

Based on the expression levels of the mRNA combinations at the cellular level, we selected the P2A peptide-linked N+M, N+M+S, N+S+M, N+M+S2, and N+S2+M groups for the preparation of lipid nanoparticles (LNPs). Simultaneously, individual M, N, and S2 mRNA LNPs were prepared. The preparation process was as follows:
1) mRNA Loading: FCoV-M-mRNA, FCoV-N-mRNA, FCoV-S2-mRNA, FCoV-N+M-mRNA, FCoV-N+M+S-mRNA, FCoV-N+S+M-mRNA, FCoV-N+M+S2-mRNA, and FCoV-N+S2+M-mRNA were mixed with a citrate buffer in a 1:1 ratio, respectively. Under specific temperature and time conditions, they were allowed to fully undergo complexation to form suitable complexes.
2) Liposome Preparation: The phospholipid MC3, distearoylphosphatidylcholine (DSPC), cholesterol, and lipid-polyethylene glycol (DMG-PEG2000) (Shanghai A.V.T. Pharmaceutical Co., Ltd.) were mixed with ethanol at a molar ratio of 50:10:38.5:1.5. The mixture was homogenized using methods like ultrasonication to dissolve the components uniformly.
3) Mixing Liposomes and mRNA: The buffer from step 1 and the liposome solution from step 2 were mixed. Shear forces, such as from ultrasonication or glass beads, were applied to ensure thorough mixing of the two components.
4) Purification: The mixed reagent was purified to remove excess liposomes, degraded RNA, unloaded mRNA, or impurities.
5) Particle Size and Concentration Measurement: The particle size and concentration of the liposomal particles were determined using dynamic light scattering.

It should be noted that contamination by RNA hydrolases and other degrading enzymes should be avoided during the preparation process to ensure the integrity and stability of mRNA.

The test results showed that the diameter of the packaged lipid nanoparticles was between 70-100 nM, with good homogeneity.

### Example 4: Evaluation of Antibody Titers Induced by FIP mRNA Vaccine in Mice

Male BALB/c mice aged 6-8 weeks were randomly divided into 8 groups (5 mice per group): FCoV-M-mRNA + FCoV-N-mRNA, FCoV-M-mRNA + FCoV-N-mRNA + FCoV-S2-mRNA, FCoV-N+M-mRNA, FCoV-N+M+S-mRNA, FCoV-N+S+M-mRNA, FCoV-N+M+S2-mRNA, FCoV-N+S2+M-mRNA vaccine groups, and a PBS blank control group. Immunization was performed via intramuscular injection. The primary immunization was on day 1, followed by a booster immunization on day 28, with a dose of 10 µg for each group. Blood was collected from the tail vein 14 days after the secondary immunization to collect serum, and binding antibody and neutralizing antibody levels were detected by ELISA.

The results are shown in FIG. 4: Compared with the control group, all vaccine groups achieved high levels of binding antibodies. And, the binding antibody levels of the tandem combination vaccines were comparable to those of the mixed vaccines. (In the figure: N/M means a 1:1 mixture of FCoV-N-mRNA vaccine and FCoV-M-mRNA vaccine; N/M/S2 means a 1:1:1 mixture of FCoV-N-mRNA vaccine, FCoV-M-mRNA vaccine, and FCoV-S2-mRNA vaccine; the plus sign means that two or three proteins are co-expressed in tandem and packaged into a single vaccine.)

The neutralizing antibody titers are shown in Table 5 below. Compared with the control group, the neutralizing antibody titers of all vaccine groups were significantly increased. Among the tandem combination vaccines, the top three vaccines with the highest neutralizing antibody titers were the FCoV-N+M+S2-mRNA vaccine group, the FCoV-N+M-mRNA vaccine group, and the FCoV-N+M+S-mRNA vaccine group. Wherein, the neutralizing antibody titer of the FCoV-N+M+S2-mRNA vaccine group was comparable to that of the FCoV-M-mRNA vaccine + FCoV-N-mRNA vaccine + FCoV-S2-mRNA vaccine mixture group, and the neutralizing antibody titer of the FCoV-N+M-mRNA vaccine group was comparable to that of the FCoV-M-mRNA vaccine + FCoV-N-mRNA vaccine mixture group.

**Table 5**

| Group | Neutralizing Titer |
|---|---|
| FCoV-M-mRNA vaccine + FCoV-N-mRNA vaccine | 1:514 |
| FCoV-M-mRNAvaccine+ FCoV-N-mRNA vaccine+ FCoV-S2-mRNA vaccine | 1:812 |
| FCoV-N+M-mRNA vaccine | 1:507 |
| FCoV- N+M+S-mRNA vaccine | 1:435 |
| FCoV- N+S+M-mRNA vaccine | 1:162 |
| FCoV- N+M+S2-mRNA vaccine | 1:824 |
| FCoV- N+S2+M-mRNA vaccine | 1:216 |
| PBS control | 1:3 |

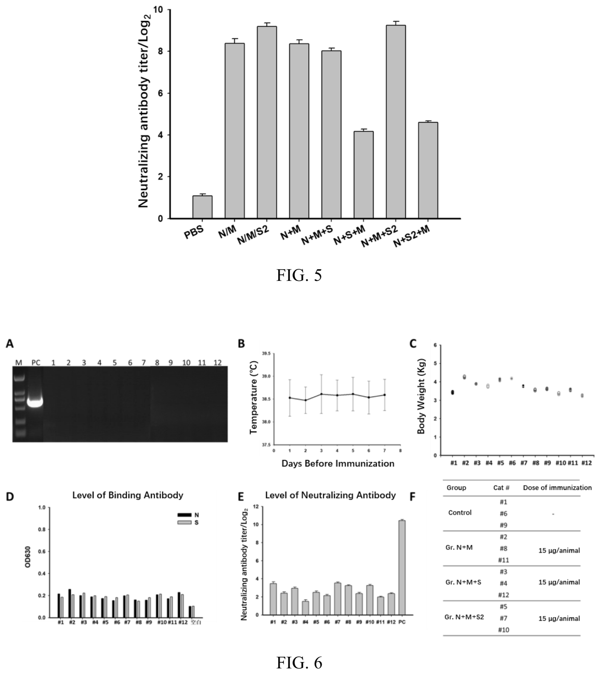

The results of the antibody titer measurement experiment are shown in FIG. 5. The antibody titers of the FCoV-N+M-mRNA, FCoV-N+M+S-mRNA, and FCoV-N+M+S2-mRNA vaccine groups were all above 2⁶. And, the antibody titer of the FCoV-N+M+S2-mRNA vaccine group was comparable to that of the FCoV-M-mRNA vaccine + FCoV-N-mRNA vaccine + FCoV-S2-mRNA vaccine mixture group, and the antibody titer of the FCoV-N+M-mRNA vaccine group was comparable to that of the FCoV-M-mRNA vaccine + FCoV-N-mRNA vaccine mixture group.

From the above results, it can be concluded that the FCoV-N+M-mRNA, FCoV-N+M+S-mRNA, and FCoV-N+M+S2-mRNA vaccines prepared in the present invention can provide good protection, and the protective effect is comparable to that of the mixed immunization with vaccines expressing individual proteins separately.

### Example 5: Evaluation of the Efficacy of FIP mRNA Vaccine in Target Animals

To detect the efficacy of the FIP mRNA vaccine in animals, the protective effect of the FCoV-N+M-mRNA, FCoV-N+M+S-mRNA, and FCoV-N+M+S2-mRNA vaccines against FIP virus disease was evaluated using a target animal model for FIP.

The specific experimental procedure and results are as follows:

### 1) Phase I - Pre-immunization

One-year-old breed cats that were physically robust, without injuries, and in good mental state were selected for laboratory examination. Finally, 12 test animals meeting the experimental criteria were selected.

Screening results are shown in FIG. 6. FIG. 6A shows the detection of FcoV, and all results were negative. FIG. 6B shows body temperature monitoring, all within the normal range (37.8°C ~ 39.5°C). FIG. 6C shows body weight monitoring, all within the normal range; FIG. 6D shows the detection of N and S protein binding antibodies, but no antibodies were detected; FIG. 6E shows the detection of neutralizing antibody, but no neutralizing antibodies were detected; FIG. 6F shows the grouping information.

### 2) Phase II - Immunization and Observation of Post-Immunization Reactions

Animals were observed for 7 days before immunization. The first subcutaneous immunization was performed on day 8, and the second subcutaneous immunization was performed on day 29. The dose for each vaccine group was 15 µg per animal. After each immunization, body temperature, body weight, food intake, subcutaneous swelling and pain, and other abnormal indicators were recorded daily.

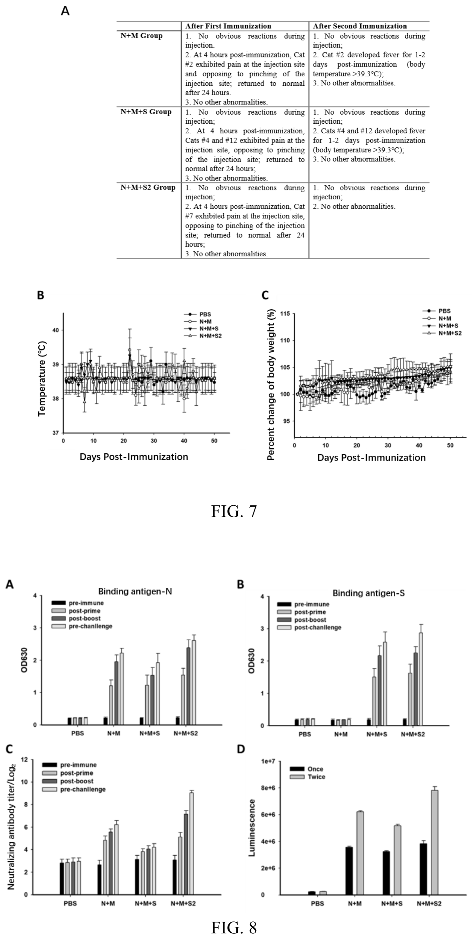

The recorded results are shown in FIG. 7. FIG. 7A shows observations of post-immunization reactions. FIG. 7B and 7C show body temperature and body weight post-immunization, respectively, wherein the FcoV-N+M+S-mRNA group showed transient fever after both the first and second immunizations, while the other groups were normal.

### 3) Phase III - Post-Immunization Assays

Serum was collected before immunization, 14 days after the first immunization, 14 days and 28 days after the second immunization for the detection of N and S protein binding antibodies and neutralizing antibodies. Simultaneously, whole blood was collected 14 days after the first immunization, 14 days and 28 days after the second immunization for PBMC isolation and lymphocyte proliferation assays.

The experimental results are shown in FIG. 8. FIGS. 8A and 8B show the detection of binding antibodies, and binding antibodies were detected in all groups, with no significant differences in antibody levels. FIG. 8C shows the detection of neutralizing antibodies, where the neutralizing antibody levels from highest to lowest were the N+M+S2 group, the N+M group, and the N+M+S group. FIG. 8D shows the lymphocyte proliferation assay, where the level of lymphocyte proliferation promotion from highest to lowest was the N+M+S2 group, the N+M group, and the N+M+S group, consistent with the neutralizing antibody detection results.

### 4) Phase IV - Challenge

After the second immunization, animals were observed for 28 days. On day 29, the challenge was initiated. The FIP virus strain rQS-79 (10⁵ TCID50, half of the tissue culture infection dose) was administered orally to the test animals, followed by a 28-day observation period. Daily body temperature, body weight, water consumption, food consumption, and other abnormalities were recorded. Supportive treatment was provided based on the condition. If experimental animals died during the test period, necropsy was performed promptly, and the viral titer of FIPV in major organs was detected.

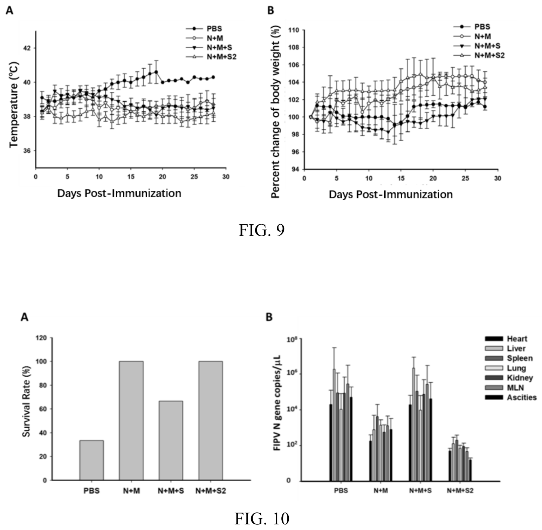

Body temperature and body weight monitoring post-challenge are shown in FIGS. 9A and 9B: The N+M+S2 group was entirely normal; in the N+M group, cat No. 11 had a persistent fever for multiple days post-challenge, while the other two were normal; in the N+M+S group, cats No. 3 and No. 12 had persistent fever for multiple days post-challenge, and cat No. 3 died 18 days post-challenge; all three cats in the PBS group developed fever post-challenge, and cats No. 1 and No. 6 died 20 days post-challenge.

On day 29 after the challenge (28 days post-challenge), the survival rate was counted. All surviving test animals were euthanized and subjected to necropsy to detect the viral titer of FIPV in major organs. The survival rate statistics are shown in FIG. 10A: The survival rates of the N+M+S2 group and the N+M group were both 100%; the N+M+S group had the next highest survival rate; the PBS group had the lowest survival rate. The viral titer detection results of FIPV in the major organs of animals from each group are shown in FIG. 10B: The tested organs included heart, liver, spleen, lung, kidney, mesenteric lymph nodes (MLN), and ascites. The viral titer in the N+M+S group was comparable to that of the control group, the viral titer in the N+M group was next, and the viral titer in the N+M+S2 group was the lowest.

Based on the comprehensive results, it can be concluded that: the selected N+M+S2 tandem-expressing mRNA vaccine provides excellent protection against FIP, and cats immunized with it showed no significant adverse reactions, indicating safety and efficacy; the N+M tandem-expressing mRNA vaccine had the next best protective effect, with some cats showing transient fever after immunization; the N+M+S tandem-expressing mRNA vaccine had the worst protective effect. This is of great significance for the clinical prevention of FIP.

### Example 6 Therapeutic Evaluation of FIP mRNA Vaccine in Target Animals Already Infected with FIP Virus

To further evaluate the therapeutic effect of the FIP mRNA vaccine on animals already infected with the FIP virus, a total of 12 one-year-old breed cats that were physically robust, without injuries, and in good mental state, were selected. First, the FIP virus strain rQS-79 (10⁵ TCID50) was administered orally to the test animals. 6 hours later, the test animals were divided into 4 groups, 3 animals per group, and vaccinated three times (day 0 / day 7 / day 21) with the P2A-linked FCoV-N+M-mRNA, FCoV-N+M+S-mRNA, FCoV-N+M+S2-mRNA vaccines, and PBS, respectively, at a dose of 20 µg per animal. Clinical observations were conducted, the number of surviving cats was recorded, and the survival rate was calculated. The results showed that: The survival rates of the N+M+S2 group and the N+M group were both 100%.

To further verify the safety of the FIP mRNA vaccine and obtain the maximum tolerated dose in the target animal (cats), preliminary results indicate good vaccine safety.

### Sequence Listing

(Note: Sequence 2 was not recognized by WIPO Sequence due to being too short, and sequences 41-57 were not recognized by WIPO Sequence due to the presence of U in RNA, so "intentional skipping" was applied in the software, which does not affect retrieval. The aforementioned sequences are listed below for reference, and the original sequences as disclosed here shall prevail).

### DNA Sequences

Kozak sequence (SEQ ID NO:2)
   GCCACC
**mRNA Sequences**
   5'-UTR+Kozak +M+3'-UTR+100 poly(A) sequence (SEQ ID NO:41) 
GGAUAGCUACCAGCUUACACUUGCUUCUGACACAACUGUGUUCGCUAUCAACCACACAAACAGACACCGCCACCA
UGAAGUACAUUUUGUUAAUACUCGCGUGCAUUAUUGCAUGCGUUUAUGGAGAACGCUACUGUGCCAUGCAAA
AUACAGGCUCGCAGUGCAUUAAUGGCACAGAUUCAUCAUGUAGCACCUGUUUUGAACGUGGUGGUCUUAUUU
GGCAUCUGGCUAACUGGAACUUCAGCUGGUCUGUAAUAUUGAUUGUUUUUAUAACAGUGUUAAAAUAUGGAA
GACCGCAAUUCAGCUGGCUCGUUUAUGGCAUUAAAAUGCUGAUCAUGUGGCUAUUAUGGCCUAUUGUUCUAG
CGCUUACGAUUUUUAAUGCAUACUCUGAGUACCAAGUUUCCAGAUAUGUAAUGUUCGGCUUUAGUGUUGCAG
GUGCAGUUGUAACGUUUGCACUAUGGAUGAUGUAUUUUGUGAGAUCUAUUCAGCUGUAUAGACGGACCAAAU
CAUGGUGGUCUUUUAAUCCUGAAACCAAUGCGAUUCUUUGUGUCAAUGCAUUGGGUAGAAGCUAUGUACUCC
CUCUUGAUGGCACUCCUACAGGUGUUACUCUUACCCUACUUUCAGGAAAUCUAUACGCUGAAGGUUUUAAAAU
GGCUGGUGGUCUUACCAUCGAGCAUUUGCCUAAAUAUGUCAUGAUUGCUACGCCUAGUAGAACCAUCGUUUAC
ACAUUAGUUGGAAAACAACUAAAGGCAACUACUGCCACUGGAUGGGCUUACUAUGUAAAAUCUAAAGCUGGUG
AUUACUCAACAGAAGCACGUACUGAUAAUUUGAGUGAACAUGAAAAAUUAUUACAUAUGGUGUAAGCUCCCUU
UCUUGCUUUCCAGGGAAAGUCCCUUGGUCCCCAGACCCUAAUACCUGGACAUGUCUAAAUCAUGAAAUUCUUU
GAACAUCUGGCUCCUGCCUAAUAAAGAAUAUUUAUUUUCAUUGCAAAAAAAAAAAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAAA
   5'-UTR+Kozak +N+3'-UTR+100 poly(A) sequence (SEQ ID NO:42) 
GGAUAGCUACCAGCUUACACUUGCUUCUGACACAACUGUGUUCGCUAUCAACCACACAAACAGACACCGCCACCA
UGGCCACACAGGGACAACGCGUCAACUGGGGAGAUGAACCUUCCAAAAGACGUGGUCGUUCUAACUCUCGUGG
UCGGAAGAAUAACACUAUACCUCUUUCAUUCUUCAAUCCCAUCCAACUCGAACCAGGAUCAAAAUUUUGGAGCG
UAUGUCCGAGAGAUUUUGUUCCCAAGGGAAUAGGUAACAAGGAUCAACAAAUUGGUUAUUGGAAUAGACAAG
AGCGUUACCGCAUUGUCAAAGGUCAGCGUAAGGAACUUCCUGAGAGGUGGUUUUUCUACUUCUUAGGCACAG
GACCUCAAGCUGAUGCUAAAUUUAAAGACAAGAUUGAUGGAGUCUUCUGGGUUGCAAAGGAUGGUGCCAUGA
AUAAACCAACAACACUUGGCACUCGUGGUACCAACAAUGAAUCCAAACCACUGAAAUUUGAUGGUAAGAUACCA
CCGCAAUUUCAGCUUGAAGUGAACCGAUCUAGGAACAACUCAAGAAGUGGUUCUCAGUCUAGAUCUGUCUCUA
GAAACAGGUCUCAAUCCAGAGGAAGACAACAAUCCAAUAAUCAGAAUAAUGUUGAGGAUACAAUUGUAGCUGU
GCUUCAGAAAUUAGGUGUUACUGAAAAGCAAAGGUCACGUUCUAAAUCUAGAGAUCGUGGUGACUCUAAACCU
AGAGACACAACACCUAAUAAUGCCAACAAACACACCUGGAAGAAGACUGCAGGUAAAGGUGAUGUGACAAAUUU
CUAUGGUGCUAGAAGUGCUUCAGCUAACUUUGGUGAUAGUGAUCUCGUUGCCAACGGUAACGCUGCCAAAUCC
UACCCUCAGAUAGCUGAAUGCGUUCCAUCAGUGUCUAGCAUGCUCUUCGGUAGUCAAUGGUCUGCUGAAGAUG
AUGGUGAUCAAGUGAAAGUCACGCUCACUCAUACCUAUUACCUGCCAAAAGAUGAUGCCAAAACCAGCCAAUUC
CUAGAACAGAUUGACGCUUACAAGCGGCCAUCUCAAGUGGCUAAAGAUCAGAGGCAAAGAAAAUCUCGUUCUA
AGUCUGCUGAGAAGAAGCCUGAGGAAUUGUCUGUAACUCUUGUAGAGGCAUAUACGGAUGUGUUUGAUGACA
CACAGGUUGAGAUGAUUGAUGAGGUUACGAACUAAGCUCCCUUUCUUGCUUUCCAGGGAAAGUCCCUUGGUCC
CCAGACCCUAAUACCUGGACAUGUCUAAAUCAUGAAAUUCUUUGAACAUCUGGCUCCUGCCUAAUAAAGAAUA
UUUAUUUUCAUUGCAAAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAA
   5'-UTR+Kozak +S+3'-UTR+100 poly(A) sequence (SEQ ID NO:43) 
GGAUAGCUACCAGCUUACACUUGCUUCUGACACAACUGUGUUCGCUAUCAACCACACAAACAGACACCGCCACCA
UGAUUGUGCUCGUAACUUGCCUCUUGUUGUUAUGUUCAUACCACACAGUUUUGAGUACAACAAAUAAUGAAU
GCAUACAAGUUAACGUAACACAAUUGGCUGGCAAUGAAAACCUUAUCAGAGAUUUUCUGUUUAGUAACUUUAA
AGAAGAAGGAAGUGUAGUUGUUGGUGGUUAUUACCCUACAGAGGUGUGGUACAACUGCUCUAGAACAGCUCG
AACUACUGCCUUUCAGUAUUUUAAUAAUAUACAUGCCUUUUAUUUUGUUAUGGAAGCCAUGGAAAAUAGCAC
 
UGGUAAUGCACGUGGUAAACCAUUAUUAUUUCAUGUGCAUGGUGAGCCUGUUAGUGUUAUUAUAUCGGCUU
AUAGGGAUGAUGUGCAACAAAGGCCCCUUUUAAAACAUGGGUUAGUGUGCAUAACUAAAAAUCGCCAUAUUAA
CUAUGAACAAUUCACCUCCAACCAGUGGAAUUCCACAUGUACGGGUGCUGACAGAAAAAUUCCUUUCUCUGUCA
UACCCACGGACAAUGGAACAAAAAUCUAUGGUCUUGAGUGGAAUGAUGACUUUGUUACAGCUUAUAUUAGUG
GUCGUUCUUAUCACUUGAACAUCAAUACUAAUUGGUUUAACAAUGUCACACUUUUGUAUUCACGCUCAAGCAC
UGCUACCUGGGAAUACAGUGCUGCAUAUGCUUACCAAGGUGUUUCUAACUUCACUUAUUACAAGUUAAAUAAC
ACCAAUGGUCUAAAAACCUAUGAAUUAUGUGAAGAUUAUGAACAUUGCACUGGCUAUGCUACCAAUGUAUUU
GCUCCGACAUCAGGUGGUUACAUACCUGAUGGAUUUAGUUUUAACAAUUGGUUCUUGCUUACAAAUAGUUCC
ACUUUUGUUAGUGGCAGGUUUGUAACAAAUCAACCAUUAUUGAUUAAUUGCUUGUGGCCAGUGCCCAGUUUU
GGUGUAGCAGCACAAGAAUUUUGUUUUGAAGGUGCACAGUUUAGCCAAUGUAAUGGUGUGUCUUUAAAUAAC
ACAGUGGAUGUUAUUAGAUUCAACCUUAAUUUCACUGCAGAUGUACAAUCUGGUAUGGGUGCUACAGUAUUU
UCACUGAAUACAACAGGUGGUGUCAUUCUUGAAAUUUCAUGUUAUAGUGACACAGUGAGUGAGUCUAGUUCU
UACAGUUAUGGUGAAAUCCCGUUCGGCAUAACUGACGGACCACGAUACUGUUAUGUACUUUACAAUGGCACAG
CUCUUAAAUAUUUAGGAACAUUACCACCCAGUGUAAAGGAAAUUGCUAUUAGUAAGUGGGGCCAUUUUUAUA
UUAAUGGUUACAAUUUCUUUAGCACAUUUCCUAUUGGUUGUAUAUCUUUUAAUUUAACCACUGGUGUUAGU
GGAGCUUUUUGGACAAUUGCUUACACAUCGUAUACUGAAGCAUUAGUACAAGUUGAAAACACAGCUAUUAAAA
AUGUGACGUAUUGUAACAGUCACAUUAAUAACAUUAAAUGUUCUCAACUUACUGCUAAUUUGAAUAAUGGAU
UUUAUCCUGUUGCUUCAAGUGAAGUAGGUUUCGUUAAUAAGAGUGUUGUGUUAUUACCUAGCUUUUUCACA
UACACCGCUGUCAAUAUAACCAUUGAUCUUGGUAUGAAGCUUAGUGGUUAUGGUCAACCCAUAGCCUCGACAC
UAAGUAACAUCACACUACCAAUGCAGGAUAACAAUACUGAUGUGUACUGUAUUCGUUCUAACCAAUUCUCAGU
UUAUGUUCAUUCCACUUGCAAAAGUUCUUUAUGGGACAAUAUUUUUAAUCAAGACUGCACGGAUGUUUUAGA
GGCUACAGCUGUUAUAAAAACUGGUACUUGUCCUUUCUCAUUUGAUAAAUUGAACAAUUACUUGACUUUUAA
CAAGUUCUGUUUGUCGUUGAGUCCUGUUGGUGCUAAUUGCAAGUUUGAUGUUGCUGCACGUACAAGAACCAA
UGAGCAGGUUGUUAGAAGUCUAUAUGUAAUAUAUGAAGAAGGAGACAACAUAGUGGGUGUACCGUCUGAUAA
UAGCGGUCUGCACGAUUUGUCUGUGCUACACCUAGACUCCUGUACAGAUUACAAUAUAUAUGGUAGAACUGG
UGUUGGUAUUAUUAGACGAACUAACAGUACGCUACUUAGUGGCUUAUAUUACACAUCACUAUCAGGUGAUUU
GUUAGGCUUUAAAAAUGUUAGUGAUGGUGUCAUUUAUUCUGUGACGCCAUGUGAUGUAAGCGCACAAGCGGC
UGUUAUUGAUGGUGCCAUAGUUGGAGCUAUGACUUCCAUUAACAGUGAACUGUUAGGUCUAACACAUUGGAC
AACGACACCUAAUUUUUAUUACUACUCUAUAUAUAAUUACACAAGUGAGAGGACUCGUGACACUGCAAUUGAC
AGUAACGAUGUUGAUUGUGAACCUGUCAUAACCUAUUCUAAUAUAGGUGUUUGUAAAAAUGGUGCUUUGGU
UUUUAUUAACGUCACACAUUCUGACGGAGACGUGCAACCAAUUAGCACUGGUAAUGUCACGAUACCUACAAAU
UUUACCAUAUCUGUGCAAGUUGAAUACAUGCAGGUUUACACUACACCAGUAUCAAUAGAUUGUGCAAGAUACG
UUUGUAAUGGUAACCCUAGAUGUAACAAAUUGUUAACACAAUAUGUGUCUGCAUGUCAAACUAUUGAACAAGC
ACUUGCAAUGGGUGCCAGACUUGAAAACAUGGAGGUUGAUUCCAUGUUGUUUGUCUCGGAAAAUGCCCUUAA
AUUGGCAUCUGUUGAGGCGUUCAAUAGUACAGAAAAUUUAGAUUCUAUUUACAAAGAAUGGCCUAGCAUAGG
UGGUUCUUGGCUAGGAGGUCUAAAAGAUAUACUACCGUCCCAUAAUAGCAAACGUAAGUAUGGUUCUGCUAU
AGAAGAUUUGCUUUUUGAUAAAGUUGUAACAUCUGGUUUAGGUACAGUUGAUGAAGAUUAUAAACGUUGUA
CUGGUGGUUACGACAUAGCAGACUUGGUGUGUGCUCAAUAUUACAAUGGCAUCAUGGUUCUACCAGGUGUAG
CUAAUGCUGACAAGAUGACUAUGUACACAGCAUCACUUGCAGGUGGUAUAACAUUAGGUGCACUUGGUGGUG
GCGCCGUGGCUAUACCUUUUGCAGUAGCAGUACAGGCUAGACUUAAUUAUGUUGCUCUACAAACUGAUGUAU
UGAAUAAAAACCAACAGAUCCUGGCUAAUGCCUUCAAUCAAGCUAUUGGUAACAUUACACAGGCUUUUGGUAA
GGUUAAUGAUGCUAUACAUCAAACAUCACAAGGUCUUGCCACUGUUGCUAAAGCGUUGGCAAAAGUGCAAGAU
GUUGUCAACACACAAGGGCAAGCUUUAAGUCACCUUACAGUACAAUUGCAAAAUAAUUUUCAAGCCAUUAGUA
GUUCUAUUAGUGAUAUUUAUAACAGGCUUGACGAACUGAGUGCUGAUGCACAAGUUGAUAGGCUGAUUACAG
GUAGACUUACAGCACUUAAUGCAUUUGUGUCUCAGACUCUAACCAGACAAGCAGAGGUUAGGGCUAGUAGACA
ACUUGCCAAAGACAAGGUUAAUGAAUGUGUUAGGUCUCAGUCUCAGAGAUUCGGAUUCUGUGGUAAUGGUAC
ACAUUUGUUUUCACUAGCAAAUGCAGCACCAAAUGGCAUGAUUUUCUUUCAUACAGUACUAUUACCAACAGCU
UAUGAAACUGUAACAGCUUGGUCAGGUAUUUGUGCUUCAGAUGGCGAUCGCACUUUCGGACUUGUCGUUAAA
GAUGUGCAGUUGACGUUGUUUCGUAAUCUAGAUGACAAGUUCUAUUUGACCCCCAGAACUAUGUAUCAGCCU
AGAGUUGCAACUAGUUCUGAUUUUGUUCAAAUUGAAGGGUGUGAUGUGUUGUUUGUCAACGCGACUGUAAU
UGAUUUGCCUAGUAUUAUACCUGACUAUAUUGACAUUAAUCAAACUGUUCAAGACAUAUUAGAAAAUUACAGA
CCAAACUGGACUGUACCUGAAUUUACACUUGAUAUUUUCAACGCAACCUAUUUAAAUCUGACUGGUGAAAUUG
AUGACUUAGAGUUUAGGUCAGAAAAGCUACAUAACACUACAGUAGAACUUGCCAUUCUCAUUGAUAACAUUAA
UAAUACAUUAGUCAAUCUUGAAUGGCUCAAUAGAAUUGAAACUUAUGUAAAAUGGCCUUGGUAUGUGUGGCU
ACUGAUAGGUUUAGUAGUAGUAUUUUGCAUACCAUUACUGCUAUUUUGCUGUUUUAGCACAGGUUGUUGUG
GAUGCAUAGGUUGUUUAGGAAGUUGUUGUCACUCUAUAUGUAGUAGAAGACAAUUUGAAAAUUAUGAACCAA
UUGAAAAAGUGCAUGUCCACUAAGCUCCCUUUCUUGCUUUCCAGGGAAAGUCCCUUGGUCCCCAGACCCUAAU
ACCUGGACAUGUCUAAAUCAUGAAAUUCUUUGAACAUCUGGCUCCUGCCUAAUAAAGAAUAUUUAUUUUCAUU
GCAAAAAAAAAAAAAAAAAAAAAAAAAAAAAAAAAAAAAAAAAAAAAAAAAAAAAAAAAAAAAAAAAAAAAAAA
AAAAAAAAAAAAAAAAAAAAAAAAAAAA
   5'-UTR+Kozak +S1+3'-UTR+100 poly(A) sequence (SEQ ID NO:44) 
GGAUAGCUACCAGCUUACACUUGCUUCUGACACAACUGUGUUCGCUAUCAACCACACAAACAGACACCGCCACCA
UGAUUGUGCUCGUAACUUGCCUCUUGUUGUUAUGUUCAUACCACACAGUUUUGAGUACAACAAAUAAUGAAU
 
GCAUACAAGUUAACGUAACACAAUUGGCUGGCAAUGAAAACCUUAUCAGAGAUUUUCUGUUUAGUAACUUUAA
AGAAGAAGGAAGUGUAGUUGUUGGUGGUUAUUACCCUACAGAGGUGUGGUACAACUGCUCUAGAACAGCUCG
AACUACUGCCUUUCAGUAUUUUAAUAAUAUACAUGCCUUUUAUUUUGUUAUGGAAGCCAUGGAAAAUAGCAC
UGGUAAUGCACGUGGUAAACCAUUAUUAUUUCAUGUGCAUGGUGAGCCUGUUAGUGUUAUUAUAUCGGCUU
AUAGGGAUGAUGUGCAACAAAGGCCCCUUUUAAAACAUGGGUUAGUGUGCAUAACUAAAAAUCGCCAUAUUAA
CUAUGAACAAUUCACCUCCAACCAGUGGAAUUCCACAUGUACGGGUGCUGACAGAAAAAUUCCUUUCUCUGUCA
UACCCACGGACAAUGGAACAAAAAUCUAUGGUCUUGAGUGGAAUGAUGACUUUGUUACAGCUUAUAUUAGUG
GUCGUUCUUAUCACUUGAACAUCAAUACUAAUUGGUUUAACAAUGUCACACUUUUGUAUUCACGCUCAAGCAC
UGCUACCUGGGAAUACAGUGCUGCAUAUGCUUACCAAGGUGUUUCUAACUUCACUUAUUACAAGUUAAAUAAC
ACCAAUGGUCUAAAAACCUAUGAAUUAUGUGAAGAUUAUGAACAUUGCACUGGCUAUGCUACCAAUGUAUUU
GCUCCGACAUCAGGUGGUUACAUACCUGAUGGAUUUAGUUUUAACAAUUGGUUCUUGCUUACAAAUAGUUCC
ACUUUUGUUAGUGGCAGGUUUGUAACAAAUCAACCAUUAUUGAUUAAUUGCUUGUGGCCAGUGCCCAGUUUU
GGUGUAGCAGCACAAGAAUUUUGUUUUGAAGGUGCACAGUUUAGCCAAUGUAAUGGUGUGUCUUUAAAUAAC
ACAGUGGAUGUUAUUAGAUUCAACCUUAAUUUCACUGCAGAUGUACAAUCUGGUAUGGGUGCUACAGUAUUU
UCACUGAAUACAACAGGUGGUGUCAUUCUUGAAAUUUCAUGUUAUAGUGACACAGUGAGUGAGUCUAGUUCU
UACAGUUAUGGUGAAAUCCCGUUCGGCAUAACUGACGGACCACGAUACUGUUAUGUACUUUACAAUGGCACAG
CUCUUAAAUAUUUAGGAACAUUACCACCCAGUGUAAAGGAAAUUGCUAUUAGUAAGUGGGGCCAUUUUUAUA
UUAAUGGUUACAAUUUCUUUAGCACAUUUCCUAUUGGUUGUAUAUCUUUUAAUUUAACCACUGGUGUUAGU
GGAGCUUUUUGGACAAUUGCUUACACAUCGUAUACUGAAGCAUUAGUACAAGUUGAAAACACAGCUAUUAAAA
AUGUGACGUAUUGUAACAGUCACAUUAAUAACAUUAAAUGUUCUCAACUUACUGCUAAUUUGAAUAAUGGAU
UUUAUCCUGUUGCUUCAAGUGAAGUAGGUUUCGUUAAUAAGAGUGUUGUGUUAUUACCUAGCUUUUUCACA
UACACCGCUGUCAAUAUAACCAUUGAUCUUGGUAUGAAGCUUAGUGGUUAUGGUCAACCCAUAGCCUCGACAC
UAAGUAACAUCACACUACCAAUGCAGGAUAACAAUACUGAUGUGUACUGUAUUCGUUCUAACCAAUUCUCAGU
UUAUGUUCAUUCCACUUGCAAAAGUUCUUUAUGGGACAAUAUUUUUAAUCAAGACUGCACGGAUGUUUUAGA
GGCUACAGCUGUUAUAAAAACUGGUACUUGUCCUUUCUCAUUUGAUAAAUUGAACAAUUACUUGACUUUUAA
CAAGUUCUGUUUGUCGUUGAGUCCUGUUGGUGCUAAUUGCAAGUUUGAUGUUGCUGCACGUACAAGAACCAA
UGAGCAGGUUGUUAGAAGUCUAUAUGUAAUAUAUGAAGAAGGAGACAACAUAGUGGGUGUACCGUCUGAUAA
UAGCGGUCUGCACGAUUUGUCUGUGCUACACCUAGACUCCUGUACAGAUUACAAUAUAUAUGGUAGAACUGG
UGUUGGUAUUAUUAGACGAACUAACAGUACGCUACUUAGUGGCUUAUAUUACACAUCACUAUCAGGUGAUUU
GUUAGGCUUUAAAAAUGUUAGUGAUGGUGUCAUUUAUUCUGUGACGCCAUGUGAUGUAAGCGCACAAGCGGC
UGUUAUUGAUGGUGCCAUAGUUGGAGCUAUGACUUCCAUUAACAGUGAACUGUUAGGUCUAACACAUUGGAC
AACGACACCUAAUUUUUAUUACUACGCUCCCUUUCUUGCUUUCCAGGGAAAGUCCCUUGGUCCCCAGACCCUAA
UACCUGGACAUGUCUAAAUCAUGAAAUUCUUUGAACAUCUGGCUCCUGCCUAAUAAAGAAUAUUUAUUUUCAU
UGCAAAAAAAAAAAAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAA
   5'-UTR+Kozak +S2+3'-UTR+100 poly(A) sequence (SEQ ID NO:45) 
GGAUAGCUACCAGCUUACACUUGCUUCUGACACAACUGUGUUCGCUAUCAACCACACAAACAGACACCGCCACCA
UGUCUAUAUAUAAUUACACAAGUGAGAGGACUCGUGACACUGCAAUUGACAGUAACGAUGUUGAUUGUGAAC
CUGUCAUAACCUAUUCUAAUAUAGGUGUUUGUAAAAAUGGUGCUUUGGUUUUUAUUAACGUCACACAUUCUG
ACGGAGACGUGCAACCAAUUAGCACUGGUAAUGUCACGAUACCUACAAAUUUUACCAUAUCUGUGCAAGUUGA
AUACAUGCAGGUUUACACUACACCAGUAUCAAUAGAUUGUGCAAGAUACGUUUGUAAUGGUAACCCUAGAUGU
AACAAAUUGUUAACACAAUAUGUGUCUGCAUGUCAAACUAUUGAACAAGCACUUGCAAUGGGUGCCAGACUUG
AAAACAUGGAGGUUGAUUCCAUGUUGUUUGUCUCGGAAAAUGCCCUUAAAUUGGCAUCUGUUGAGGCGUUCA
AUAGUACAGAAAAUUUAGAUUCUAUUUACAAAGAAUGGCCUAGCAUAGGUGGUUCUUGGCUAGGAGGUCUAA
AAGAUAUACUACCGUCCCAUAAUAGCAAACGUAAGUAUGGUUCUGCUAUAGAAGAUUUGCUUUUUGAUAAAG
UUGUAACAUCUGGUUUAGGUACAGUUGAUGAAGAUUAUAAACGUUGUACUGGUGGUUACGACAUAGCAGACU
UGGUGUGUGCUCAAUAUUACAAUGGCAUCAUGGUUCUACCAGGUGUAGCUAAUGCUGACAAGAUGACUAUGU
ACACAGCAUCACUUGCAGGUGGUAUAACAUUAGGUGCACUUGGUGGUGGCGCCGUGGCUAUACCUUUUGCAG
UAGCAGUACAGGCUAGACUUAAUUAUGUUGCUCUACAAACUGAUGUAUUGAAUAAAAACCAACAGAUCCUGGC
UAAUGCCUUCAAUCAAGCUAUUGGUAACAUUACACAGGCUUUUGGUAAGGUUAAUGAUGCUAUACAUCAAACA
UCACAAGGUCUUGCCACUGUUGCUAAAGCGUUGGCAAAAGUGCAAGAUGUUGUCAACACACAAGGGCAAGCUU
UAAGUCACCUUACAGUACAAUUGCAAAAUAAUUUUCAAGCCAUUAGUAGUUCUAUUAGUGAUAUUUAUAACA
GGCUUGACGAACUGAGUGCUGAUGCACAAGUUGAUAGGCUGAUUACAGGUAGACUUACAGCACUUAAUGCAU
UUGUGUCUCAGACUCUAACCAGACAAGCAGAGGUUAGGGCUAGUAGACAACUUGCCAAAGACAAGGUUAAUGA
AUGUGUUAGGUCUCAGUCUCAGAGAUUCGGAUUCUGUGGUAAUGGUACACAUUUGUUUUCACUAGCAAAUGC
AGCACCAAAUGGCAUGAUUUUCUUUCAUACAGUACUAUUACCAACAGCUUAUGAAACUGUAACAGCUUGGUCA
GGUAUUUGUGCUUCAGAUGGCGAUCGCACUUUCGGACUUGUCGUUAAAGAUGUGCAGUUGACGUUGUUUCG
UAAUCUAGAUGACAAGUUCUAUUUGACCCCCAGAACUAUGUAUCAGCCUAGAGUUGCAACUAGUUCUGAUUUU
GUUCAAAUUGAAGGGUGUGAUGUGUUGUUUGUCAACGCGACUGUAAUUGAUUUGCCUAGUAUUAUACCUGA
CUAUAUUGACAUUAAUCAAACUGUUCAAGACAUAUUAGAAAAUUACAGACCAAACUGGACUGUACCUGAAUUU
ACACUUGAUAUUUUCAACGCAACCUAUUUAAAUCUGACUGGUGAAAUUGAUGACUUAGAGUUUAGGUCAGAA
AAGCUACAUAACACUACAGUAGAACUUGCCAUUCUCAUUGAUAACAUUAAUAAUACAUUAGUCAAUCUUGAAU
GGCUCAAUAGAAUUGAAACUUAUGUAAAAUGGCCUUGGUAUGUGUGGCUACUGAUAGGUUUAGUAGUAGUA
 
UUUUGCAUACCAUUACUGCUAUUUUGCUGUUUUAGCACAGGUUGUUGUGGAUGCAUAGGUUGUUUAGGAAG
UUGUUGUCACUCUAUAUGUAGUAGAAGACAAUUUGAAAAUUAUGAACCAAUUGAAAAAGUGCAUGUCCACUA
AGCUCCCUUUCUUGCUUUCCAGGGAAAGUCCCUUGGUCCCCAGACCCUAAUACCUGGACAUGUCUAAAUCAUGA
AAUUCUUUGAACAUCUGGCUCCUGCCUAAUAAAGAAUAUUUAUUUUCAUUGCAAAAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAA
AAAAA
   5'-UTR+Kozak +M+N+3'-UTR+100 poly(A)(T2A) (SEQ ID NO:46) 
GGAUAGCUACCAGCUUACACUUGCUUCUGACACAACUGUGUUCGCUAUCAACCACACAAACAGACACCGCCACCA
UGAAGUACAUUUUGUUAAUACUCGCGUGCAUUAUUGCAUGCGUUUAUGGAGAACGCUACUGUGCCAUGCAAA
AUACAGGCUCGCAGUGCAUUAAUGGCACAGAUUCAUCAUGUAGCACCUGUUUUGAACGUGGUGGUCUUAUUU
GGCAUCUGGCUAACUGGAACUUCAGCUGGUCUGUAAUAUUGAUUGUUUUUAUAACAGUGUUAAAAUAUGGAA
GACCGCAAUUCAGCUGGCUCGUUUAUGGCAUUAAAAUGCUGAUCAUGUGGCUAUUAUGGCCUAUUGUUCUAG
CGCUUACGAUUUUUAAUGCAUACUCUGAGUACCAAGUUUCCAGAUAUGUAAUGUUCGGCUUUAGUGUUGCAG
GUGCAGUUGUAACGUUUGCACUAUGGAUGAUGUAUUUUGUGAGAUCUAUUCAGCUGUAUAGACGGACCAAAU
CAUGGUGGUCUUUUAAUCCUGAAACCAAUGCGAUUCUUUGUGUCAAUGCAUUGGGUAGAAGCUAUGUACUCC
CUCUUGAUGGCACUCCUACAGGUGUUACUCUUACCCUACUUUCAGGAAAUCUAUACGCUGAAGGUUUUAAAAU
GGCUGGUGGUCUUACCAUCGAGCAUUUGCCUAAAUAUGUCAUGAUUGCUACGCCUAGUAGAACCAUCGUUUAC
ACAUUAGUUGGAAAACAACUAAAGGCAACUACUGCCACUGGAUGGGCUUACUAUGUAAAAUCUAAAGCUGGUG
AUUACUCAACAGAAGCACGUACUGAUAAUUUGAGUGAACAUGAAAAAUUAUUACAUAUGGUGGGCAGCGGCGA
AGGCCGCGGCAGCCUGCUGACCUGCGGCGAUGUGGAAGAAAACCCGGGCCCGAUGGCCACACAGGGACAACGCG
UCAACUGGGGAGAUGAACCUUCCAAAAGACGUGGUCGUUCUAACUCUCGUGGUCGGAAGAAUAACACUAUACC
UCUUUCAUUCUUCAAUCCCAUCCAACUCGAACCAGGAUCAAAAUUUUGGAGCGUAUGUCCGAGAGAUUUUGUU
CCCAAGGGAAUAGGUAACAAGGAUCAACAAAUUGGUUAUUGGAAUAGACAAGAGCGUUACCGCAUUGUCAAAG
GUCAGCGUAAGGAACUUCCUGAGAGGUGGUUUUUCUACUUCUUAGGCACAGGACCUCAAGCUGAUGCUAAAU
UUAAAGACAAGAUUGAUGGAGUCUUCUGGGUUGCAAAGGAUGGUGCCAUGAAUAAACCAACAACACUUGGCAC
UCGUGGUACCAACAAUGAAUCCAAACCACUGAAAUUUGAUGGUAAGAUACCACCGCAAUUUCAGCUUGAAGUG
AACCGAUCUAGGAACAACUCAAGAAGUGGUUCUCAGUCUAGAUCUGUCUCUAGAAACAGGUCUCAAUCCAGAG
GAAGACAACAAUCCAAUAAUCAGAAUAAUGUUGAGGAUACAAUUGUAGCUGUGCUUCAGAAAUUAGGUGUUA
CUGAAAAGCAAAGGUCACGUUCUAAAUCUAGAGAUCGUGGUGACUCUAAACCUAGAGACACAACACCUAAUAAU
GCCAACAAACACACCUGGAAGAAGACUGCAGGUAAAGGUGAUGUGACAAAUUUCUAUGGUGCUAGAAGUGCUU
CAGCUAACUUUGGUGAUAGUGAUCUCGUUGCCAACGGUAACGCUGCCAAAUCCUACCCUCAGAUAGCUGAAUG
CGUUCCAUCAGUGUCUAGCAUGCUCUUCGGUAGUCAAUGGUCUGCUGAAGAUGAUGGUGAUCAAGUGAAAGU
CACGCUCACUCAUACCUAUUACCUGCCAAAAGAUGAUGCCAAAACCAGCCAAUUCCUAGAACAGAUUGACGCUU
ACAAGCGGCCAUCUCAAGUGGCUAAAGAUCAGAGGCAAAGAAAAUCUCGUUCUAAGUCUGCUGAGAAGAAGCC
UGAGGAAUUGUCUGUAACUCUUGUAGAGGCAUAUACGGAUGUGUUUGAUGACACACAGGUUGAGAUGAUUG
AUGAGGUUACGAACUAAGCUCCCUUUCUUGCUUUCCAGGGAAAGUCCCUUGGUCCCCAGACCCUAAUACCUGG
ACAUGUCUAAAUCAUGAAAUUCUUUGAACAUCUGGCUCCUGCCUAAUAAAGAAUAUUUAUUUUCAUUGCAAAA
AAAAAAAAAAAAAAAAAAAAAAAAAAAAAAAAAAAAAAAAAAAAAAAAAAAAAAAAAAAAAAAAAAAAAAAAAAAA
AAAAAAAAAAAAAAAAAAAAAA
   5'-UTR+Kozak +N+M+3'-UTR+100 poly(A) (T2A) (SEQ ID NO:47) 
GGAUAGCUACCAGCUUACACUUGCUUCUGACACAACUGUGUUCGCUAUCAACCACACAAACAGACACCGCCACCA
UGGCCACACAGGGACAACGCGUCAACUGGGGAGAUGAACCUUCCAAAAGACGUGGUCGUUCUAACUCUCGUGG
UCGGAAGAAUAACACUAUACCUCUUUCAUUCUUCAAUCCCAUCCAACUCGAACCAGGAUCAAAAUUUUGGAGCG
UAUGUCCGAGAGAUUUUGUUCCCAAGGGAAUAGGUAACAAGGAUCAACAAAUUGGUUAUUGGAAUAGACAAG
AGCGUUACCGCAUUGUCAAAGGUCAGCGUAAGGAACUUCCUGAGAGGUGGUUUUUCUACUUCUUAGGCACAG
GACCUCAAGCUGAUGCUAAAUUUAAAGACAAGAUUGAUGGAGUCUUCUGGGUUGCAAAGGAUGGUGCCAUGA
AUAAACCAACAACACUUGGCACUCGUGGUACCAACAAUGAAUCCAAACCACUGAAAUUUGAUGGUAAGAUACCA
CCGCAAUUUCAGCUUGAAGUGAACCGAUCUAGGAACAACUCAAGAAGUGGUUCUCAGUCUAGAUCUGUCUCUA
GAAACAGGUCUCAAUCCAGAGGAAGACAACAAUCCAAUAAUCAGAAUAAUGUUGAGGAUACAAUUGUAGCUGU
GCUUCAGAAAUUAGGUGUUACUGAAAAGCAAAGGUCACGUUCUAAAUCUAGAGAUCGUGGUGACUCUAAACCU
AGAGACACAACACCUAAUAAUGCCAACAAACACACCUGGAAGAAGACUGCAGGUAAAGGUGAUGUGACAAAUUU
CUAUGGUGCUAGAAGUGCUUCAGCUAACUUUGGUGAUAGUGAUCUCGUUGCCAACGGUAACGCUGCCAAAUCC
UACCCUCAGAUAGCUGAAUGCGUUCCAUCAGUGUCUAGCAUGCUCUUCGGUAGUCAAUGGUCUGCUGAAGAUG
AUGGUGAUCAAGUGAAAGUCACGCUCACUCAUACCUAUUACCUGCCAAAAGAUGAUGCCAAAACCAGCCAAUUC
CUAGAACAGAUUGACGCUUACAAGCGGCCAUCUCAAGUGGCUAAAGAUCAGAGGCAAAGAAAAUCUCGUUCUA
AGUCUGCUGAGAAGAAGCCUGAGGAAUUGUCUGUAACUCUUGUAGAGGCAUAUACGGAUGUGUUUGAUGACA
CACAGGUUGAGAUGAUUGAUGAGGUUACGAACGGCAGCGGCGAAGGCCGCGGCAGCCUGCUGACCUGCGGCGA
UGUGGAAGAAAACCCGGGCCCGAUGAAGUACAUUUUGUUAAUACUCGCGUGCAUUAUUGCAUGCGUUUAUGG
AGAACGCUACUGUGCCAUGCAAAAUACAGGCUCGCAGUGCAUUAAUGGCACAGAUUCAUCAUGUAGCACCUGU
UUUGAACGUGGUGGUCUUAUUUGGCAUCUGGCUAACUGGAACUUCAGCUGGUCUGUAAUAUUGAUUGUUUU
UAUAACAGUGUUAAAAUAUGGAAGACCGCAAUUCAGCUGGCUCGUUUAUGGCAUUAAAAUGCUGAUCAUGUG
GCUAUUAUGGCCUAUUGUUCUAGCGCUUACGAUUUUUAAUGCAUACUCUGAGUACCAAGUUUCCAGAUAUGU
AAUGUUCGGCUUUAGUGUUGCAGGUGCAGUUGUAACGUUUGCACUAUGGAUGAUGUAUUUUGUGAGAUCUA
 
UUCAGCUGUAUAGACGGACCAAAUCAUGGUGGUCUUUUAAUCCUGAAACCAAUGCGAUUCUUUGUGUCAAUG
CAUUGGGUAGAAGCUAUGUACUCCCUCUUGAUGGCACUCCUACAGGUGUUACUCUUACCCUACUUUCAGGAAA
UCUAUACGCUGAAGGUUUUAAAAUGGCUGGUGGUCUUACCAUCGAGCAUUUGCCUAAAUAUGUCAUGAUUGC
UACGCCUAGUAGAACCAUCGUUUACACAUUAGUUGGAAAACAACUAAAGGCAACUACUGCCACUGGAUGGGCU
UACUAUGUAAAAUCUAAAGCUGGUGAUUACUCAACAGAAGCACGUACUGAUAAUUUGAGUGAACAUGAAAAAU
UAUUACAUAUGGUGUAAGCUCCCUUUCUUGCUUUCCAGGGAAAGUCCCUUGGUCCCCAGACCCUAAUACCUGG
ACAUGUCUAAAUCAUGAAAUUCUUUGAACAUCUGGCUCCUGCCUAAUAAAGAAUAUUUAUUUUCAUUGCAAAA
AAAAAAAAAAAAAAAAAAAAAAAAAAAAAAAAAAAAAAAAAAAAAAAAAAAAAAAAAAAAAAAAAAAAAAAAA
AAAAAAAAAAAAAAAAAAAAAA
   5'-UTR+Kozak +M+N+3'-UTR+100 poly(A)(P2A) sequence (SEQ ID NO:48) 
GGAUAGCUACCAGCUUACACUUGCUUCUGACACAACUGUGUUCGCUAUCAACCACACAAACAGACACCGCCACCA
UGAAGUACAUUUUGUUAAUACUCGCGUGCAUUAUUGCAUGCGUUUAUGGAGAACGCUACUGUGCCAUGCAAA
AUACAGGCUCGCAGUGCAUUAAUGGCACAGAUUCAUCAUGUAGCACCUGUUUUGAACGUGGUGGUCUUAUUU
GGCAUCUGGCUAACUGGAACUUCAGCUGGUCUGUAAUAUUGAUUGUUUUUAUAACAGUGUUAAAAUAUGGAA
GACCGCAAUUCAGCUGGCUCGUUUAUGGCAUUAAAAUGCUGAUCAUGUGGCUAUUAUGGCCUAUUGUUCUAG
CGCUUACGAUUUUUAAUGCAUACUCUGAGUACCAAGUUUCCAGAUAUGUAAUGUUCGGCUUUAGUGUUGCAG
GUGCAGUUGUAACGUUUGCACUAUGGAUGAUGUAUUUUGUGAGAUCUAUUCAGCUGUAUAGACGGACCAAAU
CAUGGUGGUCUUUUAAUCCUGAAACCAAUGCGAUUCUUUGUGUCAAUGCAUUGGGUAGAAGCUAUGUACUCC
CUCUUGAUGGCACUCCUACAGGUGUUACUCUUACCCUACUUUCAGGAAAUCUAUACGCUGAAGGUUUUAAAAU
GGCUGGUGGUCUUACCAUCGAGCAUUUGCCUAAAUAUGUCAUGAUUGCUACGCCUAGUAGAACCAUCGUUUAC
ACAUUAGUUGGAAAACAACUAAAGGCAACUACUGCCACUGGAUGGGCUUACUAUGUAAAAUCUAAAGCUGGUG
AUUACUCAACAGAAGCACGUACUGAUAAUUUGAGUGAACAUGAAAAAUUAUUACAUAUGGUGGGCAGCGGCGC
GACCAACUUUAGCCUGCUGAAACAGGCGGGCGAUGUGGAAGAAAACCCGGGCCCGAUGGCCACACAGGGACAAC
GCGUCAACUGGGGAGAUGAACCUUCCAAAAGACGUGGUCGUUCUAACUCUCGUGGUCGGAAGAAUAACACUAU
ACCUCUUUCAUUCUUCAAUCCCAUCCAACUCGAACCAGGAUCAAAAUUUUGGAGCGUAUGUCCGAGAGAUUUU
GUUCCCAAGGGAAUAGGUAACAAGGAUCAACAAAUUGGUUAUUGGAAUAGACAAGAGCGUUACCGCAUUGUCA
AAGGUCAGCGUAAGGAACUUCCUGAGAGGUGGUUUUUCUACUUCUUAGGCACAGGACCUCAAGCUGAUGCUAA
AUUUAAAGACAAGAUUGAUGGAGUCUUCUGGGUUGCAAAGGAUGGUGCCAUGAAUAAACCAACAACACUUGGC
ACUCGUGGUACCAACAAUGAAUCCAAACCACUGAAAUUUGAUGGUAAGAUACCACCGCAAUUUCAGCUUGAAG
UGAACCGAUCUAGGAACAACUCAAGAAGUGGUUCUCAGUCUAGAUCUGUCUCUAGAAACAGGUCUCAAUCCAG
AGGAAGACAACAAUCCAAUAAUCAGAAUAAUGUUGAGGAUACAAUUGUAGCUGUGCUUCAGAAAUUAGGUGU
UACUGAAAAGCAAAGGUCACGUUCUAAAUCUAGAGAUCGUGGUGACUCUAAACCUAGAGACACAACACCUAAUA
AUGCCAACAAACACACCUGGAAGAAGACUGCAGGUAAAGGUGAUGUGACAAAUUUCUAUGGUGCUAGAAGUGC
UUCAGCUAACUUUGGUGAUAGUGAUCUCGUUGCCAACGGUAACGCUGCCAAAUCCUACCCUCAGAUAGCUGAA
UGCGUUCCAUCAGUGUCUAGCAUGCUCUUCGGUAGUCAAUGGUCUGCUGAAGAUGAUGGUGAUCAAGUGAAA
GUCACGCUCACUCAUACCUAUUACCUGCCAAAAGAUGAUGCCAAAACCAGCCAAUUCCUAGAACAGAUUGACGC
UUACAAGCGGCCAUCUCAAGUGGCUAAAGAUCAGAGGCAAAGAAAAUCUCGUUCUAAGUCUGCUGAGAAGAAG
CCUGAGGAAUUGUCUGUAACUCUUGUAGAGGCAUAUACGGAUGUGUUUGAUGACACACAGGUUGAGAUGAUU
GAUGAGGUUACGAACUAAGCUCCCUUUCUUGCUUUCCAGGGAAAGUCCCUUGGUCCCCAGACCCUAAUACCUG
GACAUGUCUAAAUCAUGAAAUUCUUUGAACAUCUGGCUCCUGCCUAAUAAAGAAUAUUUAUUUUCAUUGCAAA
AAAAAAAAAAAAAAAAAAAAAAAAAAAAAAAAAAAAAAAAAAAAAAAAAAAAAAAAAAAAAAAAAAAAAAAAAAAA
AAAAAAAAAAAAAAAAAAAAAAA
   5'-UTR+Kozak+N+M+3'-UTR+100 poly(A)(P2A) sequence (SEQ ID NO:49) 
GGAUAGCUACCAGCUUACACUUGCUUCUGACACAACUGUGUUCGCUAUCAACCACACAAACAGACACCGCCACCA
UGGCCACACAGGGACAACGCGUCAACUGGGGAGAUGAACCUUCCAAAAGACGUGGUCGUUCUAACUCUCGUGG
UCGGAAGAAUAACACUAUACCUCUUUCAUUCUUCAAUCCCAUCCAACUCGAACCAGGAUCAAAAUUUUGGAGCG
UAUGUCCGAGAGAUUUUGUUCCCAAGGGAAUAGGUAACAAGGAUCAACAAAUUGGUUAUUGGAAUAGACAAG
AGCGUUACCGCAUUGUCAAAGGUCAGCGUAAGGAACUUCCUGAGAGGUGGUUUUUCUACUUCUUAGGCACAG
GACCUCAAGCUGAUGCUAAAUUUAAAGACAAGAUUGAUGGAGUCUUCUGGGUUGCAAAGGAUGGUGCCAUGA
AUAAACCAACAACACUUGGCACUCGUGGUACCAACAAUGAAUCCAAACCACUGAAAUUUGAUGGUAAGAUACCA
CCGCAAUUUCAGCUUGAAGUGAACCGAUCUAGGAACAACUCAAGAAGUGGUUCUCAGUCUAGAUCUGUCUCUA
GAAACAGGUCUCAAUCCAGAGGAAGACAACAAUCCAAUAAUCAGAAUAAUGUUGAGGAUACAAUUGUAGCUGU
GCUUCAGAAAUUAGGUGUUACUGAAAAGCAAAGGUCACGUUCUAAAUCUAGAGAUCGUGGUGACUCUAAACCU
AGAGACACAACACCUAAUAAUGCCAACAAACACACCUGGAAGAAGACUGCAGGUAAAGGUGAUGUGACAAAUUU
CUAUGGUGCUAGAAGUGCUUCAGCUAACUUUGGUGAUAGUGAUCUCGUUGCCAACGGUAACGCUGCCAAAUCC
UACCCUCAGAUAGCUGAAUGCGUUCCAUCAGUGUCUAGCAUGCUCUUCGGUAGUCAAUGGUCUGCUGAAGAUG
AUGGUGAUCAAGUGAAAGUCACGCUCACUCAUACCUAUUACCUGCCAAAAGAUGAUGCCAAAACCAGCCAAUUC
CUAGAACAGAUUGACGCUUACAAGCGGCCAUCUCAAGUGGCUAAAGAUCAGAGGCAAAGAAAAUCUCGUUCUA
AGUCUGCUGAGAAGAAGCCUGAGGAAUUGUCUGUAACUCUUGUAGAGGCAUAUACGGAUGUGUUUGAUGACA
CACAGGUUGAGAUGAUUGAUGAGGUUACGAACGGCAGCGGCGCGACCAACUUUAGCCUGCUGAAACAGGCGGG
CGAUGUGGAAGAAAACCCGGGCCCGAUGAAGUACAUUUUGUUAAUACUCGCGUGCAUUAUUGCAUGCGUUUA
UGGAGAACGCUACUGUGCCAUGCAAAAUACAGGCUCGCAGUGCAUUAAUGGCACAGAUUCAUCAUGUAGCACC
UGUUUUGAACGUGGUGGUCUUAUUUGGCAUCUGGCUAACUGGAACUUCAGCUGGUCUGUAAUAUUGAUUGU
 
UUUUAUAACAGUGUUAAAAUAUGGAAGACCGCAAUUCAGCUGGCUCGUUUAUGGCAUUAAAAUGCUGAUCAU
GUGGCUAUUAUGGCCUAUUGUUCUAGCGCUUACGAUUUUUAAUGCAUACUCUGAGUACCAAGUUUCCAGAUA
UGUAAUGUUCGGCUUUAGUGUUGCAGGUGCAGUUGUAACGUUUGCACUAUGGAUGAUGUAUUUUGUGAGAU
CUAUUCAGCUGUAUAGACGGACCAAAUCAUGGUGGUCUUUUAAUCCUGAAACCAAUGCGAUUCUUUGUGUCAA
UGCAUUGGGUAGAAGCUAUGUACUCCCUCUUGAUGGCACUCCUACAGGUGUUACUCUUACCCUACUUUCAGGA
AAUCUAUACGCUGAAGGUUUUAAAAUGGCUGGUGGUCUUACCAUCGAGCAUUUGCCUAAAUAUGUCAUGAUU
GCUACGCCUAGUAGAACCAUCGUUUACACAUUAGUUGGAAAACAACUAAAGGCAACUACUGCCACUGGAUGGG
CUUACUAUGUAAAAUCUAAAGCUGGUGAUUACUCAACAGAAGCACGUACUGAUAAUUUGAGUGAACAUGAAAA
AUUAUUACAUAUGGUGUAAGCUCCCUUUCUUGCUUUCCAGGGAAAGUCCCUUGGUCCCCAGACCCUAAUACCU
GGACAUGUCUAAAUCAUGAAAUUCUUUGAACAUCUGGCUCCUGCCUAAUAAAGAAUAUUUAUUUUCAUUGCAA
AAAAAAAAAAAAAAAAAAAAAAAAAAAAAAAAAAAAAAAAAAAAAAAAAAAAAAAAAAAAAAAAAAAAAAAAAAA
AAAAAAAAAAAAAAAAAAAAAAAAA
   5'-UTR+Kozak+N+M+S+3'-UTR+100 poly(A)(T2A) sequence (SEQ ID NO:50) 
GGAUAGCUACCAGCUUACACUUGCUUCUGACACAACUGUGUUCGCUAUCAACCACACAAACAGACACCGCCACCA
UGGCCACACAGGGACAACGCGUCAACUGGGGAGAUGAACCUUCCAAAAGACGUGGUCGUUCUAACUCUCGUGG
UCGGAAGAAUAACACUAUACCUCUUUCAUUCUUCAAUCCCAUCCAACUCGAACCAGGAUCAAAAUUUUGGAGCG
UAUGUCCGAGAGAUUUUGUUCCCAAGGGAAUAGGUAACAAGGAUCAACAAAUUGGUUAUUGGAAUAGACAAG
AGCGUUACCGCAUUGUCAAAGGUCAGCGUAAGGAACUUCCUGAGAGGUGGUUUUUCUACUUCUUAGGCACAG
GACCUCAAGCUGAUGCUAAAUUUAAAGACAAGAUUGAUGGAGUCUUCUGGGUUGCAAAGGAUGGUGCCAUGA
AUAAACCAACAACACUUGGCACUCGUGGUACCAACAAUGAAUCCAAACCACUGAAAUUUGAUGGUAAGAUACCA
CCGCAAUUUCAGCUUGAAGUGAACCGAUCUAGGAACAACUCAAGAAGUGGUUCUCAGUCUAGAUCUGUCUCUA
GAAACAGGUCUCAAUCCAGAGGAAGACAACAAUCCAAUAAUCAGAAUAAUGUUGAGGAUACAAUUGUAGCUGU
GCUUCAGAAAUUAGGUGUUACUGAAAAGCAAAGGUCACGUUCUAAAUCUAGAGAUCGUGGUGACUCUAAACCU
AGAGACACAACACCUAAUAAUGCCAACAAACACACCUGGAAGAAGACUGCAGGUAAAGGUGAUGUGACAAAUUU
CUAUGGUGCUAGAAGUGCUUCAGCUAACUUUGGUGAUAGUGAUCUCGUUGCCAACGGUAACGCUGCCAAAUCC
UACCCUCAGAUAGCUGAAUGCGUUCCAUCAGUGUCUAGCAUGCUCUUCGGUAGUCAAUGGUCUGCUGAAGAUG
AUGGUGAUCAAGUGAAAGUCACGCUCACUCAUACCUAUUACCUGCCAAAAGAUGAUGCCAAAACCAGCCAAUUC
CUAGAACAGAUUGACGCUUACAAGCGGCCAUCUCAAGUGGCUAAAGAUCAGAGGCAAAGAAAAUCUCGUUCUA
AGUCUGCUGAGAAGAAGCCUGAGGAAUUGUCUGUAACUCUUGUAGAGGCAUAUACGGAUGUGUUUGAUGACA
CACAGGUUGAGAUGAUUGAUGAGGUUACGAACGGCAGCGGCGAAGGCCGCGGCAGCCUGCUGACCUGCGGCGA
UGUGGAAGAAAACCCGGGCCCGAUGAAGUACAUUUUGUUAAUACUCGCGUGCAUUAUUGCAUGCGUUUAUGG
AGAACGCUACUGUGCCAUGCAAAAUACAGGCUCGCAGUGCAUUAAUGGCACAGAUUCAUCAUGUAGCACCUGU
UUUGAACGUGGUGGUCUUAUUUGGCAUCUGGCUAACUGGAACUUCAGCUGGUCUGUAAUAUUGAUUGUUUU
UAUAACAGUGUUAAAAUAUGGAAGACCGCAAUUCAGCUGGCUCGUUUAUGGCAUUAAAAUGCUGAUCAUGUG
GCUAUUAUGGCCUAUUGUUCUAGCGCUUACGAUUUUUAAUGCAUACUCUGAGUACCAAGUUUCCAGAUAUGU
AAUGUUCGGCUUUAGUGUUGCAGGUGCAGUUGUAACGUUUGCACUAUGGAUGAUGUAUUUUGUGAGAUCUA
UUCAGCUGUAUAGACGGACCAAAUCAUGGUGGUCUUUUAAUCCUGAAACCAAUGCGAUUCUUUGUGUCAAUG
CAUUGGGUAGAAGCUAUGUACUCCCUCUUGAUGGCACUCCUACAGGUGUUACUCUUACCCUACUUUCAGGAAA
UCUAUACGCUGAAGGUUUUAAAAUGGCUGGUGGUCUUACCAUCGAGCAUUUGCCUAAAUAUGUCAUGAUUGC
UACGCCUAGUAGAACCAUCGUUUACACAUUAGUUGGAAAACAACUAAAGGCAACUACUGCCACUGGAUGGGCU
UACUAUGUAAAAUCUAAAGCUGGUGAUUACUCAACAGAAGCACGUACUGAUAAUUUGAGUGAACAUGAAAAAU
UAUUACAUAUGGUGGGCAGCGGCGAAGGCCGCGGCAGCCUGCUGACCUGCGGCGAUGUGGAAGAAAACCCGGG
CCCGAUGAUUGUGCUCGUAACUUGCCUCUUGUUGUUAUGUUCAUACCACACAGUUUUGAGUACAACAAAUAAU
GAAUGCAUACAAGUUAACGUAACACAAUUGGCUGGCAAUGAAAACCUUAUCAGAGAUUUUCUGUUUAGUAACU
UUAAAGAAGAAGGAAGUGUAGUUGUUGGUGGUUAUUACCCUACAGAGGUGUGGUACAACUGCUCUAGAACAG
CUCGAACUACUGCCUUUCAGUAUUUUAAUAAUAUACAUGCCUUUUAUUUUGUUAUGGAAGCCAUGGAAAAUA
GCACUGGUAAUGCACGUGGUAAACCAUUAUUAUUUCAUGUGCAUGGUGAGCCUGUUAGUGUUAUUAUAUCGG
CUUAUAGGGAUGAUGUGCAACAAAGGCCCCUUUUAAAACAUGGGUUAGUGUGCAUAACUAAAAAUCGCCAUAU
UAACUAUGAACAAUUCACCUCCAACCAGUGGAAUUCCACAUGUACGGGUGCUGACAGAAAAAUUCCUUUCUCU
GUCAUACCCACGGACAAUGGAACAAAAAUCUAUGGUCUUGAGUGGAAUGAUGACUUUGUUACAGCUUAUAUU
AGUGGUCGUUCUUAUCACUUGAACAUCAAUACUAAUUGGUUUAACAAUGUCACACUUUUGUAUUCACGCUCAA
GCACUGCUACCUGGGAAUACAGUGCUGCAUAUGCUUACCAAGGUGUUUCUAACUUCACUUAUUACAAGUUAAA
UAACACCAAUGGUCUAAAAACCUAUGAAUUAUGUGAAGAUUAUGAACAUUGCACUGGCUAUGCUACCAAUGUA
UUUGCUCCGACAUCAGGUGGUUACAUACCUGAUGGAUUUAGUUUUAACAAUUGGUUCUUGCUUACAAAUAGU
UCCACUUUUGUUAGUGGCAGGUUUGUAACAAAUCAACCAUUAUUGAUUAAUUGCUUGUGGCCAGUGCCCAGU
UUUGGUGUAGCAGCACAAGAAUUUUGUUUUGAAGGUGCACAGUUUAGCCAAUGUAAUGGUGUGUCUUUAAA
UAACACAGUGGAUGUUAUUAGAUUCAACCUUAAUUUCACUGCAGAUGUACAAUCUGGUAUGGGUGCUACAGU
AUUUUCACUGAAUACAACAGGUGGUGUCAUUCUUGAAAUUUCAUGUUAUAGUGACACAGUGAGUGAGUCUAG
UUCUUACAGUUAUGGUGAAAUCCCGUUCGGCAUAACUGACGGACCACGAUACUGUUAUGUACUUUACAAUGGC
ACAGCUCUUAAAUAUUUAGGAACAUUACCACCCAGUGUAAAGGAAAUUGCUAUUAGUAAGUGGGGCCAUUUU
UAUAUUAAUGGUUACAAUUUCUUUAGCACAUUUCCUAUUGGUUGUAUAUCUUUUAAUUUAACCACUGGUGU
UAGUGGAGCUUUUUGGACAAUUGCUUACACAUCGUAUACUGAAGCAUUAGUACAAGUUGAAAACACAGCUAU
UAAAAAUGUGACGUAUUGUAACAGUCACAUUAAUAACAUUAAAUGUUCUCAACUUACUGCUAAUUUGAAUAA
 
UGGAUUUUAUCCUGUUGCUUCAAGUGAAGUAGGUUUCGUUAAUAAGAGUGUUGUGUUAUUACCUAGCUUUU
UCACAUACACCGCUGUCAAUAUAACCAUUGAUCUUGGUAUGAAGCUUAGUGGUUAUGGUCAACCCAUAGCCUC
GACACUAAGUAACAUCACACUACCAAUGCAGGAUAACAAUACUGAUGUGUACUGUAUUCGUUCUAACCAAUUC
UCAGUUUAUGUUCAUUCCACUUGCAAAAGUUCUUUAUGGGACAAUAUUUUUAAUCAAGACUGCACGGAUGUU
UUAGAGGCUACAGCUGUUAUAAAAACUGGUACUUGUCCUUUCUCAUUUGAUAAAUUGAACAAUUACUUGACU
UUUAACAAGUUCUGUUUGUCGUUGAGUCCUGUUGGUGCUAAUUGCAAGUUUGAUGUUGCUGCACGUACAAG
AACCAAUGAGCAGGUUGUUAGAAGUCUAUAUGUAAUAUAUGAAGAAGGAGACAACAUAGUGGGUGUACCGUC
UGAUAAUAGCGGUCUGCACGAUUUGUCUGUGCUACACCUAGACUCCUGUACAGAUUACAAUAUAUAUGGUAG
AACUGGUGUUGGUAUUAUUAGACGAACUAACAGUACGCUACUUAGUGGCUUAUAUUACACAUCACUAUCAGG
UGAUUUGUUAGGCUUUAAAAAUGUUAGUGAUGGUGUCAUUUAUUCUGUGACGCCAUGUGAUGUAAGCGCAC
AAGCGGCUGUUAUUGAUGGUGCCAUAGUUGGAGCUAUGACUUCCAUUAACAGUGAACUGUUAGGUCUAACAC
AUUGGACAACGACACCUAAUUUUUAUUACUACUCUAUAUAUAAUUACACAAGUGAGAGGACUCGUGACACUGC
AAUUGACAGUAACGAUGUUGAUUGUGAACCUGUCAUAACCUAUUCUAAUAUAGGUGUUUGUAAAAAUGGUGC
UUUGGUUUUUAUUAACGUCACACAUUCUGACGGAGACGUGCAACCAAUUAGCACUGGUAAUGUCACGAUACCU
ACAAAUUUUACCAUAUCUGUGCAAGUUGAAUACAUGCAGGUUUACACUACACCAGUAUCAAUAGAUUGUGCAA
GAUACGUUUGUAAUGGUAACCCUAGAUGUAACAAAUUGUUAACACAAUAUGUGUCUGCAUGUCAAACUAUUG
AACAAGCACUUGCAAUGGGUGCCAGACUUGAAAACAUGGAGGUUGAUUCCAUGUUGUUUGUCUCGGAAAAUG
CCCUUAAAUUGGCAUCUGUUGAGGCGUUCAAUAGUACAGAAAAUUUAGAUUCUAUUUACAAAGAAUGGCCUA
GCAUAGGUGGUUCUUGGCUAGGAGGUCUAAAAGAUAUACUACCGUCCCAUAAUAGCAAACGUAAGUAUGGUU
CUGCUAUAGAAGAUUUGCUUUUUGAUAAAGUUGUAACAUCUGGUUUAGGUACAGUUGAUGAAGAUUAUAAA
CGUUGUACUGGUGGUUACGACAUAGCAGACUUGGUGUGUGCUCAAUAUUACAAUGGCAUCAUGGUUCUACCA
GGUGUAGCUAAUGCUGACAAGAUGACUAUGUACACAGCAUCACUUGCAGGUGGUAUAACAUUAGGUGCACUU
GGUGGUGGCGCCGUGGCUAUACCUUUUGCAGUAGCAGUACAGGCUAGACUUAAUUAUGUUGCUCUACAAACU
GAUGUAUUGAAUAAAAACCAACAGAUCCUGGCUAAUGCCUUCAAUCAAGCUAUUGGUAACAUUACACAGGCUU
UUGGUAAGGUUAAUGAUGCUAUACAUCAAACAUCACAAGGUCUUGCCACUGUUGCUAAAGCGUUGGCAAAAGU
GCAAGAUGUUGUCAACACACAAGGGCAAGCUUUAAGUCACCUUACAGUACAAUUGCAAAAUAAUUUUCAAGCC
AUUAGUAGUUCUAUUAGUGAUAUUUAUAACAGGCUUGACGAACUGAGUGCUGAUGCACAAGUUGAUAGGCUG
AUUACAGGUAGACUUACAGCACUUAAUGCAUUUGUGUCUCAGACUCUAACCAGACAAGCAGAGGUUAGGGCUA
GUAGACAACUUGCCAAAGACAAGGUUAAUGAAUGUGUUAGGUCUCAGUCUCAGAGAUUCGGAUUCUGUGGUA
AUGGUACACAUUUGUUUUCACUAGCAAAUGCAGCACCAAAUGGCAUGAUUUUCUUUCAUACAGUACUAUUACC
AACAGCUUAUGAAACUGUAACAGCUUGGUCAGGUAUUUGUGCUUCAGAUGGCGAUCGCACUUUCGGACUUGU
CGUUAAAGAUGUGCAGUUGACGUUGUUUCGUAAUCUAGAUGACAAGUUCUAUUUGACCCCCAGAACUAUGUA
UCAGCCUAGAGUUGCAACUAGUUCUGAUUUUGUUCAAAUUGAAGGGUGUGAUGUGUUGUUUGUCAACGCGAC
UGUAAUUGAUUUGCCUAGUAUUAUACCUGACUAUAUUGACAUUAAUCAAACUGUUCAAGACAUAUUAGAAAA
UUACAGACCAAACUGGACUGUACCUGAAUUUACACUUGAUAUUUUCAACGCAACCUAUUUAAAUCUGACUGGU
GAAAUUGAUGACUUAGAGUUUAGGUCAGAAAAGCUACAUAACACUACAGUAGAACUUGCCAUUCUCAUUGAUA
ACAUUAAUAAUACAUUAGUCAAUCUUGAAUGGCUCAAUAGAAUUGAAACUUAUGUAAAAUGGCCUUGGUAUG
UGUGGCUACUGAUAGGUUUAGUAGUAGUAUUUUGCAUACCAUUACUGCUAUUUUGCUGUUUUAGCACAGGU
UGUUGUGGAUGCAUAGGUUGUUUAGGAAGUUGUUGUCACUCUAUAUGUAGUAGAAGACAAUUUGAAAAUUA
UGAACCAAUUGAAAAAGUGCAUGUCCACUAAGCUCCCUUUCUUGCUUUCCAGGGAAAGUCCCUUGGUCCCCAG
ACCCUAAUACCUGGACAUGUCUAAAUCAUGAAAUUCUUUGAACAUCUGGCUCCUGCCUAAUAAAGAAUAUUUA
UUUUCAUUGCAAAAAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAA
   5'-UTR+Kozak+N+S+M+3'-UTR+100 poly(A)(T2A) sequence (SEQ ID NO:51) 
GGAUAGCUACCAGCUUACACUUGCUUCUGACACAACUGUGUUCGCUAUCAACCACACAAACAGACACCGCCACCA
UGGCCACACAGGGACAACGCGUCAACUGGGGAGAUGAACCUUCCAAAAGACGUGGUCGUUCUAACUCUCGUGG
UCGGAAGAAUAACACUAUACCUCUUUCAUUCUUCAAUCCCAUCCAACUCGAACCAGGAUCAAAAUUUUGGAGCG
UAUGUCCGAGAGAUUUUGUUCCCAAGGGAAUAGGUAACAAGGAUCAACAAAUUGGUUAUUGGAAUAGACAAG
AGCGUUACCGCAUUGUCAAAGGUCAGCGUAAGGAACUUCCUGAGAGGUGGUUUUUCUACUUCUUAGGCACAG
GACCUCAAGCUGAUGCUAAAUUUAAAGACAAGAUUGAUGGAGUCUUCUGGGUUGCAAAGGAUGGUGCCAUGA
AUAAACCAACAACACUUGGCACUCGUGGUACCAACAAUGAAUCCAAACCACUGAAAUUUGAUGGUAAGAUACCA
CCGCAAUUUCAGCUUGAAGUGAACCGAUCUAGGAACAACUCAAGAAGUGGUUCUCAGUCUAGAUCUGUCUCUA
GAAACAGGUCUCAAUCCAGAGGAAGACAACAAUCCAAUAAUCAGAAUAAUGUUGAGGAUACAAUUGUAGCUGU
GCUUCAGAAAUUAGGUGUUACUGAAAAGCAAAGGUCACGUUCUAAAUCUAGAGAUCGUGGUGACUCUAAACCU
AGAGACACAACACCUAAUAAUGCCAACAAACACACCUGGAAGAAGACUGCAGGUAAAGGUGAUGUGACAAAUUU
CUAUGGUGCUAGAAGUGCUUCAGCUAACUUUGGUGAUAGUGAUCUCGUUGCCAACGGUAACGCUGCCAAAUCC
UACCCUCAGAUAGCUGAAUGCGUUCCAUCAGUGUCUAGCAUGCUCUUCGGUAGUCAAUGGUCUGCUGAAGAUG
AUGGUGAUCAAGUGAAAGUCACGCUCACUCAUACCUAUUACCUGCCAAAAGAUGAUGCCAAAACCAGCCAAUUC
CUAGAACAGAUUGACGCUUACAAGCGGCCAUCUCAAGUGGCUAAAGAUCAGAGGCAAAGAAAAUCUCGUUCUA
AGUCUGCUGAGAAGAAGCCUGAGGAAUUGUCUGUAACUCUUGUAGAGGCAUAUACGGAUGUGUUUGAUGACA
CACAGGUUGAGAUGAUUGAUGAGGUUACGAACGGCAGCGGCGAAGGCCGCGGCAGCCUGCUGACCUGCGGCGA
UGUGGAAGAAAACCCGGGCCCGAUGAUUGUGCUCGUAACUUGCCUCUUGUUGUUAUGUUCAUACCACACAGUU
UUGAGUACAACAAAUAAUGAAUGCAUACAAGUUAACGUAACACAAUUGGCUGGCAAUGAAAACCUUAUCAGAG
 
AUUUUCUGUUUAGUAACUUUAAAGAAGGAAGUGUAGUUGUUGGUGGUUAUUACCCUACAGAGGUGUGG
UACAACUGCUCUAGAACAGCUCGAACUACUGCCUUUCAGUAUUUUAAUAAUAUACAUGCCUUUUAUUUUGUUA
UGGAAGCCAUGGAAAAUAGCACUGGUAAUGCACGUGGUAAACCAUUAUUAUUUCAUGUGCAUGGUGAGCCUG
UUAGUGUUAUUAUAUCGGCUUAUAGGGAUGAUGUGCAACAAAGGCCCCUUUUAAAACAUGGGUUAGUGUGCA
UAACUAAAAAUCGCCAUAUUAACUAUGAACAAUUCACCUCCAACCAGUGGAAUUCCACAUGUACGGGUGCUGAC
AGAAAAAUUCCUUUCUCUGUCAUACCCACGGACAAUGGAACAAAAAUCUAUGGUCUUGAGUGGAAUGAUGACU
UUGUUACAGCUUAUAUUAGUGGUCGUUCUUAUCACUUGAACAUCAAUACUAAUUGGUUUAACAAUGUCACAC
UUUUGUAUUCACGCUCAAGCACUGCUACCUGGGAAUACAGUGCUGCAUAUGCUUACCAAGGUGUUUCUAACUU
CACUUAUUACAAGUUAAAUAACACCAAUGGUCUAAAAACCUAUGAAUUAUGUGAAGAUUAUGAACAUUGCACU
GGCUAUGCUACCAAUGUAUUUGCUCCGACAUCAGGUGGUUACAUACCUGAUGGAUUUAGUUUUAACAAUUGG
UUCUUGCUUACAAAUAGUUCCACUUUUGUUAGUGGCAGGUUUGUAACAAAUCAACCAUUAUUGAUUAAUUGC
UUGUGGCCAGUGCCCAGUUUUGGUGUAGCAGCACAAGAAUUUUGUUUUGAAGGUGCACAGUUUAGCCAAUGU
AAUGGUGUGUCUUUAAAUAACACAGUGGAUGUUAUUAGAUUCAACCUUAAUUUCACUGCAGAUGUACAAUCU
GGUAUGGGUGCUACAGUAUUUUCACUGAAUACAACAGGUGGUGUCAUUCUUGAAAUUUCAUGUUAUAGUGAC
ACAGUGAGUGAGUCUAGUUCUUACAGUUAUGGUGAAAUCCCGUUCGGCAUAACUGACGGACCACGAUACUGU
UAUGUACUUUACAAUGGCACAGCUCUUAAAUAUUUAGGAACAUUACCACCCAGUGUAAAGGAAAUUGCUAUUA
GUAAGUGGGGCCAUUUUUAUAUUAAUGGUUACAAUUUCUUUAGCACAUUUCCUAUUGGUUGUAUAUCUUUU
AAUUUAACCACUGGUGUUAGUGGAGCUUUUUGGACAAUUGCUUACACAUCGUAUACUGAAGCAUUAGUACAA
GUUGAAAACACAGCUAUUAAAAAUGUGACGUAUUGUAACAGUCACAUUAAUAACAUUAAAUGUUCUCAACUUA
CUGCUAAUUUGAAUAAUGGAUUUUAUCCUGUUGCUUCAAGUGAAGUAGGUUUCGUUAAUAAGAGUGUUGUG
UUAUUACCUAGCUUUUUCACAUACACCGCUGUCAAUAUAACCAUUGAUCUUGGUAUGAAGCUUAGUGGUUAU
GGUCAACCCAUAGCCUCGACACUAAGUAACAUCACACUACCAAUGCAGGAUAACAAUACUGAUGUGUACUGUAU
UCGUUCUAACCAAUUCUCAGUUUAUGUUCAUUCCACUUGCAAAAGUUCUUUAUGGGACAAUAUUUUUAAUCA
AGACUGCACGGAUGUUUUAGAGGCUACAGCUGUUAUAAAAACUGGUACUUGUCCUUUCUCAUUUGAUAAAUU
GAACAAUUACUUGACUUUUAACAAGUUCUGUUUGUCGUUGAGUCCUGUUGGUGCUAAUUGCAAGUUUGAUG
UUGCUGCACGUACAAGAACCAAUGAGCAGGUUGUUAGAAGUCUAUAUGUAAUAUAUGAAGAAGGAGACAACAU
AGUGGGUGUACCGUCUGAUAAUAGCGGUCUGCACGAUUUGUCUGUGCUACACCUAGACUCCUGUACAGAUUAC
AAUAUAUAUGGUAGAACUGGUGUUGGUAUUAUUAGACGAACUAACAGUACGCUACUUAGUGGCUUAUAUUAC
ACAUCACUAUCAGGUGAUUUGUUAGGCUUUAAAAAUGUUAGUGAUGGUGUCAUUUAUUCUGUGACGCCAUGU
GAUGUAAGCGCACAAGCGGCUGUUAUUGAUGGUGCCAUAGUUGGAGCUAUGACUUCCAUUAACAGUGAACUG
UUAGGUCUAACACAUUGGACAACGACACCUAAUUUUUAUUACUACUCUAUAUAUAAUUACACAAGUGAGAGGA
CUCGUGACACUGCAAUUGACAGUAACGAUGUUGAUUGUGAACCUGUCAUAACCUAUUCUAAUAUAGGUGUUU
GUAAAAAUGGUGCUUUGGUUUUUAUUAACGUCACACAUUCUGACGGAGACGUGCAACCAAUUAGCACUGGUA
AUGUCACGAUACCUACAAAUUUUACCAUAUCUGUGCAAGUUGAAUACAUGCAGGUUUACACUACACCAGUAUC
AAUAGAUUGUGCAAGAUACGUUUGUAAUGGUAACCCUAGAUGUAACAAAUUGUUAACACAAUAUGUGUCUGC
AUGUCAAACUAUUGAACAAGCACUUGCAAUGGGUGCCAGACUUGAAAACAUGGAGGUUGAUUCCAUGUUGUU
UGUCUCGGAAAAUGCCCUUAAAUUGGCAUCUGUUGAGGCGUUCAAUAGUACAGAAAAUUUAGAUUCUAUUUA
CAAAGAAUGGCCUAGCAUAGGUGGUUCUUGGCUAGGAGGUCUAAAAGAUAUACUACCGUCCCAUAAUAGCAAA
CGUAAGUAUGGUUCUGCUAUAGAAGAUUUGCUUUUUGAUAAAGUUGUAACAUCUGGUUUAGGUACAGUUGA
UGAAGAUUAUAAACGUUGUACUGGUGGUUACGACAUAGCAGACUUGGUGUGUGCUCAAUAUUACAAUGGCAU
CAUGGUUCUACCAGGUGUAGCUAAUGCUGACAAGAUGACUAUGUACACAGCAUCACUUGCAGGUGGUAUAACA
UUAGGUGCACUUGGUGGUGGCGCCGUGGCUAUACCUUUUGCAGUAGCAGUACAGGCUAGACUUAAUUAUGUU
GCUCUACAAACUGAUGUAUUGAAUAAAAACCAACAGAUCCUGGCUAAUGCCUUCAAUCAAGCUAUUGGUAACA
UUACACAGGCUUUUGGUAAGGUUAAUGAUGCUAUACAUCAAACAUCACAAGGUCUUGCCACUGUUGCUAAAGC
GUUGGCAAAAGUGCAAGAUGUUGUCAACACACAAGGGCAAGCUUUAAGUCACCUUACAGUACAAUUGCAAAAU
AAUUUUCAAGCCAUUAGUAGUUCUAUUAGUGAUAUUUAUAACAGGCUUGACGAACUGAGUGCUGAUGCACAA
GUUGAUAGGCUGAUUACAGGUAGACUUACAGCACUUAAUGCAUUUGUGUCUCAGACUCUAACCAGACAAGCAG
AGGUUAGGGCUAGUAGACAACUUGCCAAAGACAAGGUUAAUGAAUGUGUUAGGUCUCAGUCUCAGAGAUUCG
GAUUCUGUGGUAAUGGUACACAUUUGUUUUCACUAGCAAAUGCAGCACCAAAUGGCAUGAUUUUCUUUCAUA
CAGUACUAUUACCAACAGCUUAUGAAACUGUAACAGCUUGGUCAGGUAUUUGUGCUUCAGAUGGCGAUCGCAC
UUUCGGACUUGUCGUUAAAGAUGUGCAGUUGACGUUGUUUCGUAAUCUAGAUGACAAGUUCUAUUUGACCCC
CAGAACUAUGUAUCAGCCUAGAGUUGCAACUAGUUCUGAUUUUGUUCAAAUUGAAGGGUGUGAUGUGUUGU
UUGUCAACGCGACUGUAAUUGAUUUGCCUAGUAUUAUACCUGACUAUAUUGACAUUAAUCAAACUGUUCAAG
ACAUAUUAGAAAAUUACAGACCAAACUGGACUGUACCUGAAUUUACACUUGAUAUUUUCAACGCAACCUAUUU
AAAUCUGACUGGUGAAAUUGAUGACUUAGAGUUUAGGUCAGAAAAGCUACAUAACACUACAGUAGAACUUGCC
AUUCUCAUUGAUAACAUUAAUAAUACAUUAGUCAAUCUUGAAUGGCUCAAUAGAAUUGAAACUUAUGUAAAA
UGGCCUUGGUAUGUGUGGCUACUGAUAGGUUUAGUAGUAGUAUUUUGCAUACCAUUACUGCUAUUUUGCUG
UUUUAGCACAGGUUGUUGUGGAUGCAUAGGUUGUUUAGGAAGUUGUUGUCACUCUAUAUGUAGUAGAAGAC
AAUUUGAAAAUUAUGAACCAAUUGAAAAAGUGCAUGUCCACUAAGGCAGCGGCGAAGGCCGCGGCAGCCUGCU
GACCUGCGGCGAUGUGGAAGAAAACCCGGGCCCGAUGAAGUACAUUUUGUUAAUACUCGCGUGCAUUAUUGCA
UGCGUUUAUGGAGAACGCUACUGUGCCAUGCAAAAUACAGGCUCGCAGUGCAUUAAUGGCACAGAUUCAUCAU
GUAGCACCUGUUUUGAACGUGGUGGUCUUAUUUGGCAUCUGGCUAACUGGAACUUCAGCUGGUCUGUAAUAU
UGAUUGUUUUUAUAACAGUGUUAAAAUAUGGAAGACCGCAAUUCAGCUGGCUCGUUUAUGGCAUUAAAAUGC
 
UGAUCAUGUGGCUAUUAUGGCCUAUUGUUCUAGCGCUUACGAUUUUUAAUGCAUACUCUGAGUACCAAGUUU
CCAGAUAUGUAAUGUUCGGCUUUAGUGUUGCAGGUGCAGUUGUAACGUUUGCACUAUGGAUGAUGUAUUUU
GUGAGAUCUAUUCAGCUGUAUAGACGGACCAAAUCAUGGUGGUCUUUUAAUCCUGAAACCAAUGCGAUUCUU
UGUGUCAAUGCAUUGGGUAGAAGCUAUGUACUCCCUCUUGAUGGCACUCCUACAGGUGUUACUCUUACCCUAC
UUUCAGGAAAUCUAUACGCUGAAGGUUUUAAAAUGGCUGGUGGUCUUACCAUCGAGCAUUUGCCUAAAUAUG
UCAUGAUUGCUACGCCUAGUAGAACCAUCGUUUACACAUUAGUUGGAAAACAACUAAAGGCAACUACUGCCAC
UGGAUGGGCUUACUAUGUAAAAUCUAAAGCUGGUGAUUACUCAACAGAAGCACGUACUGAUAAUUUGAGUGA
ACAUGAAAAAUUAUUACAUAUGGUGUAAGCUCCCUUUCUUGCUUUCCAGGGAAAGUCCCUUGGUCCCCAGACC
CUAAUACCUGGACAUGUCUAAAUCAUGAAAUUCUUUGAACAUCUGGCUCCUGCCUAAUAAAGAAUAUUUAUUU
UCAUUGCAAAAAAAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAA
   5'-UTR+Kozak+N+M+S2+3'-UTR+100 poly(A)(T2A) sequence (SEQ ID NO:52) 
GGAUAGCUACCAGCUUACACUUGCUUCUGACACAACUGUGUUCGCUAUCAACCACACAAACAGACACCGCCACCA
UGGCCACACAGGGACAACGCGUCAACUGGGGAGAUGAACCUUCCAAAAGACGUGGUCGUUCUAACUCUCGUGG
UCGGAAGAAUAACACUAUACCUCUUUCAUUCUUCAAUCCCAUCCAACUCGAACCAGGAUCAAAAUUUUGGAGCG
UAUGUCCGAGAGAUUUUGUUCCCAAGGGAAUAGGUAACAAGGAUCAACAAAUUGGUUAUUGGAAUAGACAAG
AGCGUUACCGCAUUGUCAAAGGUCAGCGUAAGGAACUUCCUGAGAGGUGGUUUUUCUACUUCUUAGGCACAG
GACCUCAAGCUGAUGCUAAAUUUAAAGACAAGAUUGAUGGAGUCUUCUGGGUUGCAAAGGAUGGUGCCAUGA
AUAAACCAACAACACUUGGCACUCGUGGUACCAACAAUGAAUCCAAACCACUGAAAUUUGAUGGUAAGAUACCA
CCGCAAUUUCAGCUUGAAGUGAACCGAUCUAGGAACAACUCAAGAAGUGGUUCUCAGUCUAGAUCUGUCUCUA
GAAACAGGUCUCAAUCCAGAGGAAGACAACAAUCCAAUAAUCAGAAUAAUGUUGAGGAUACAAUUGUAGCUGU
GCUUCAGAAAUUAGGUGUUACUGAAAAGCAAAGGUCACGUUCUAAAUCUAGAGAUCGUGGUGACUCUAAACCU
AGAGACACAACACCUAAUAAUGCCAACAAACACACCUGGAAGAAGACUGCAGGUAAAGGUGAUGUGACAAAUUU
CUAUGGUGCUAGAAGUGCUUCAGCUAACUUUGGUGAUAGUGAUCUCGUUGCCAACGGUAACGCUGCCAAAUCC
UACCCUCAGAUAGCUGAAUGCGUUCCAUCAGUGUCUAGCAUGCUCUUCGGUAGUCAAUGGUCUGCUGAAGAUG
AUGGUGAUCAAGUGAAAGUCACGCUCACUCAUACCUAUUACCUGCCAAAAGAUGAUGCCAAAACCAGCCAAUUC
CUAGAACAGAUUGACGCUUACAAGCGGCCAUCUCAAGUGGCUAAAGAUCAGAGGCAAAGAAAAUCUCGUUCUA
AGUCUGCUGAGAAGAAGCCUGAGGAAUUGUCUGUAACUCUUGUAGAGGCAUAUACGGAUGUGUUUGAUGACA
CACAGGUUGAGAUGAUUGAUGAGGUUACGAACGGCAGCGGCGAAGGCCGCGGCAGCCUGCUGACCUGCGGCGA
UGUGGAAGAAAACCCGGGCCCGAUGAAGUACAUUUUGUUAAUACUCGCGUGCAUUAUUGCAUGCGUUUAUGG
AGAACGCUACUGUGCCAUGCAAAAUACAGGCUCGCAGUGCAUUAAUGGCACAGAUUCAUCAUGUAGCACCUGU
UUUGAACGUGGUGGUCUUAUUUGGCAUCUGGCUAACUGGAACUUCAGCUGGUCUGUAAUAUUGAUUGUUUU
UAUAACAGUGUUAAAAUAUGGAAGACCGCAAUUCAGCUGGCUCGUUUAUGGCAUUAAAAUGCUGAUCAUGUG
GCUAUUAUGGCCUAUUGUUCUAGCGCUUACGAUUUUUAAUGCAUACUCUGAGUACCAAGUUUCCAGAUAUGU
AAUGUUCGGCUUUAGUGUUGCAGGUGCAGUUGUAACGUUUGCACUAUGGAUGAUGUAUUUUGUGAGAUCUA
UUCAGCUGUAUAGACGGACCAAAUCAUGGUGGUCUUUUAAUCCUGAAACCAAUGCGAUUCUUUGUGUCAAUG
CAUUGGGUAGAAGCUAUGUACUCCCUCUUGAUGGCACUCCUACAGGUGUUACUCUUACCCUACUUUCAGGAAA
UCUAUACGCUGAAGGUUUUAAAAUGGCUGGUGGUCUUACCAUCGAGCAUUUGCCUAAAUAUGUCAUGAUUGC
UACGCCUAGUAGAACCAUCGUUUACACAUUAGUUGGAAAACAACUAAAGGCAACUACUGCCACUGGAUGGGCU
UACUAUGUAAAAUCUAAAGCUGGUGAUUACUCAACAGAAGCACGUACUGAUAAUUUGAGUGAACAUGAAAAAU
UAUUACAUAUGGUGGGCAGCGGCGAAGGCCGCGGCAGCCUGCUGACCUGCGGCGAUGUGGAAGAAAACCCGGG
CCCGAUGUCUAUAUAUAAUUACACAAGUGAGAGGACUCGUGACACUGCAAUUGACAGUAACGAUGUUGAUUGU
GAACCUGUCAUAACCUAUUCUAAUAUAGGUGUUUGUAAAAAUGGUGCUUUGGUUUUUAUUAACGUCACACAU
UCUGACGGAGACGUGCAACCAAUUAGCACUGGUAAUGUCACGAUACCUACAAAUUUUACCAUAUCUGUGCAAG
UUGAAUACAUGCAGGUUUACACUACACCAGUAUCAAUAGAUUGUGCAAGAUACGUUUGUAAUGGUAACCCUAG
AUGUAACAAAUUGUUAACACAAUAUGUGUCUGCAUGUCAAACUAUUGAACAAGCACUUGCAAUGGGUGCCAGA
CUUGAAAACAUGGAGGUUGAUUCCAUGUUGUUUGUCUCGGAAAAUGCCCUUAAAUUGGCAUCUGUUGAGGCG
UUCAAUAGUACAGAAAAUUUAGAUUCUAUUUACAAAGAAUGGCCUAGCAUAGGUGGUUCUUGGCUAGGAGGU
CUAAAAGAUAUACUACCGUCCCAUAAUAGCAAACGUAAGUAUGGUUCUGCUAUAGAAGAUUUGCUUUUUGAU
AAAGUUGUAACAUCUGGUUUAGGUACAGUUGAUGAAGAUUAUAAACGUUGUACUGGUGGUUACGACAUAGCA
GACUUGGUGUGUGCUCAAUAUUACAAUGGCAUCAUGGUUCUACCAGGUGUAGCUAAUGCUGACAAGAUGACU
AUGUACACAGCAUCACUUGCAGGUGGUAUAACAUUAGGUGCACUUGGUGGUGGCGCCGUGGCUAUACCUUUU
GCAGUAGCAGUACAGGCUAGACUUAAUUAUGUUGCUCUACAAACUGAUGUAUUGAAUAAAAACCAACAGAUCC
UGGCUAAUGCCUUCAAUCAAGCUAUUGGUAACAUUACACAGGCUUUUGGUAAGGUUAAUGAUGCUAUACAUC
AAACAUCACAAGGUCUUGCCACUGUUGCUAAAGCGUUGGCAAAAGUGCAAGAUGUUGUCAACACACAAGGGCA
AGCUUUAAGUCACCUUACAGUACAAUUGCAAAAUAAUUUUCAAGCCAUUAGUAGUUCUAUUAGUGAUAUUUA
UAACAGGCUUGACGAACUGAGUGCUGAUGCACAAGUUGAUAGGCUGAUUACAGGUAGACUUACAGCACUUAAU
GCAUUUGUGUCUCAGACUCUAACCAGACAAGCAGAGGUUAGGGCUAGUAGACAACUUGCCAAAGACAAGGUUA
AUGAAUGUGUUAGGUCUCAGUCUCAGAGAUUCGGAUUCUGUGGUAAUGGUACACAUUUGUUUUCACUAGCAA
AUGCAGCACCAAAUGGCAUGAUUUUCUUUCAUACAGUACUAUUACCAACAGCUUAUGAAACUGUAACAGCUUG
GUCAGGUAUUUGUGCUUCAGAUGGCGAUCGCACUUUCGGACUUGUCGUUAAAGAUGUGCAGUUGACGUUGU
UUCGUAAUCUAGAUGACAAGUUCUAUUUGACCCCCAGAACUAUGUAUCAGCCUAGAGUUGCAACUAGUUCUGA
UUUUGUUCAAAUUGAAGGGUGUGAUGUGUUGUUUGUCAACGCGACUGUAAUUGAUUUGCCUAGUAUUAUAC
 
CUGACUAUAUUGACAUUAAUCAAACUGUUCAAGACAUAUUAGAAAAUUACAGACCAAACUGGACUGUACCUGA
AUUUACACUUGAUAUUUUCAACGCAACCUAUUUAAAUCUGACUGGUGAAAUUGAUGACUUAGAGUUUAGGUC
AGAAAAGCUACAUAACACUACAGUAGAACUUGCCAUUCUCAUUGAUAACAUUAAUAAUACAUUAGUCAAUCUU
GAAUGGCUCAAUAGAAUUGAAACUUAUGUAAAAUGGCCUUGGUAUGUGUGGCUACUGAUAGGUUUAGUAGU
AGUAUUUUGCAUACCAUUACUGCUAUUUUGCUGUUUUAGCACAGGUUGUUGUGGAUGCAUAGGUUGUUUAG
GAAGUUGUUGUCACUCUAUAUGUAGUAGAAGACAAUUUGAAAAUUAUGAACCAAUUGAAAAAGUGCAUGUCC
ACUAAGCUCCCUUUCUUGCUUUCCAGGGAAAGUCCCUUGGUCCCCAGACCCUAAUACCUGGACAUGUCUAAAUC
AUGAAAUUCUUUGAACAUCUGGCUCCUGCCUAAUAAAGAAUAUUUAUUUUCAUUGCAAAAAAAAAAAAAAAAA
AAAAAAAAAAAAAAAAAAAAAAAAAAAAAAAAAAAAAAAAAAAAAAAAAAAAAAAAAAAAAAAAAAAAAAAAA
AAAAAAAAA
   5'-UTR+Kozak+N+S2+M+3'-UTR+100 poly(A)(T2A) sequence (SEQ ID NO:53) 
GGAUAGCUACCAGCUUACACUUGCUUCUGACACAACUGUGUUCGCUAUCAACCACACAAACAGACACCGCCACCA
UGGCCACACAGGGACAACGCGUCAACUGGGGAGAUGAACCUUCCAAAAGACGUGGUCGUUCUAACUCUCGUGG
UCGGAAGAAUAACACUAUACCUCUUUCAUUCUUCAAUCCCAUCCAACUCGAACCAGGAUCAAAAUUUUGGAGCG
UAUGUCCGAGAGAUUUUGUUCCCAAGGGAAUAGGUAACAAGGAUCAACAAAUUGGUUAUUGGAAUAGACAAG
AGCGUUACCGCAUUGUCAAAGGUCAGCGUAAGGAACUUCCUGAGAGGUGGUUUUUCUACUUCUUAGGCACAG
GACCUCAAGCUGAUGCUAAAUUUAAAGACAAGAUUGAUGGAGUCUUCUGGGUUGCAAAGGAUGGUGCCAUGA
AUAAACCAACAACACUUGGCACUCGUGGUACCAACAAUGAAUCCAAACCACUGAAAUUUGAUGGUAAGAUACCA
CCGCAAUUUCAGCUUGAAGUGAACCGAUCUAGGAACAACUCAAGAAGUGGUUCUCAGUCUAGAUCUGUCUCUA
GAAACAGGUCUCAAUCCAGAGGAAGACAACAAUCCAAUAAUCAGAAUAAUGUUGAGGAUACAAUUGUAGCUGU
GCUUCAGAAAUUAGGUGUUACUGAAAAGCAAAGGUCACGUUCUAAAUCUAGAGAUCGUGGUGACUCUAAACCU
AGAGACACAACACCUAAUAAUGCCAACAAACACACCUGGAAGAAGACUGCAGGUAAAGGUGAUGUGACAAAUUU
CUAUGGUGCUAGAAGUGCUUCAGCUAACUUUGGUGAUAGUGAUCUCGUUGCCAACGGUAACGCUGCCAAAUCC
UACCCUCAGAUAGCUGAAUGCGUUCCAUCAGUGUCUAGCAUGCUCUUCGGUAGUCAAUGGUCUGCUGAAGAUG
AUGGUGAUCAAGUGAAAGUCACGCUCACUCAUACCUAUUACCUGCCAAAAGAUGAUGCCAAAACCAGCCAAUUC
CUAGAACAGAUUGACGCUUACAAGCGGCCAUCUCAAGUGGCUAAAGAUCAGAGGCAAAGAAAAUCUCGUUCUA
AGUCUGCUGAGAAGAAGCCUGAGGAAUUGUCUGUAACUCUUGUAGAGGCAUAUACGGAUGUGUUUGAUGACA
CACAGGUUGAGAUGAUUGAUGAGGUUACGAACGGCAGCGGCGAAGGCCGCGGCAGCCUGCUGACCUGCGGCGA
UGUGGAAGAAAACCCGGGCCCGAUGUCUAUAUAUAAUUACACAAGUGAGAGGACUCGUGACACUGCAAUUGAC
AGUAACGAUGUUGAUUGUGAACCUGUCAUAACCUAUUCUAAUAUAGGUGUUUGUAAAAAUGGUGCUUUGGU
UUUUAUUAACGUCACACAUUCUGACGGAGACGUGCAACCAAUUAGCACUGGUAAUGUCACGAUACCUACAAAU
UUUACCAUAUCUGUGCAAGUUGAAUACAUGCAGGUUUACACUACACCAGUAUCAAUAGAUUGUGCAAGAUACG
UUUGUAAUGGUAACCCUAGAUGUAACAAAUUGUUAACACAAUAUGUGUCUGCAUGUCAAACUAUUGAACAAGC
ACUUGCAAUGGGUGCCAGACUUGAAAACAUGGAGGUUGAUUCCAUGUUGUUUGUCUCGGAAAAUGCCCUUAA
AUUGGCAUCUGUUGAGGCGUUCAAUAGUACAGAAAAUUUAGAUUCUAUUUACAAAGAAUGGCCUAGCAUAGG
UGGUUCUUGGCUAGGAGGUCUAAAAGAUAUACUACCGUCCCAUAAUAGCAAACGUAAGUAUGGUUCUGCUAU
AGAAGAUUUGCUUUUUGAUAAAGUUGUAACAUCUGGUUUAGGUACAGUUGAUGAAGAUUAUAAACGUUGUA
CUGGUGGUUACGACAUAGCAGACUUGGUGUGUGCUCAAUAUUACAAUGGCAUCAUGGUUCUACCAGGUGUAG
CUAAUGCUGACAAGAUGACUAUGUACACAGCAUCACUUGCAGGUGGUAUAACAUUAGGUGCACUUGGUGGUG
GCGCCGUGGCUAUACCUUUUGCAGUAGCAGUACAGGCUAGACUUAAUUAUGUUGCUCUACAAACUGAUGUAU
UGAAUAAAAACCAACAGAUCCUGGCUAAUGCCUUCAAUCAAGCUAUUGGUAACAUUACACAGGCUUUUGGUAA
GGUUAAUGAUGCUAUACAUCAAACAUCACAAGGUCUUGCCACUGUUGCUAAAGCGUUGGCAAAAGUGCAAGAU
GUUGUCAACACACAAGGGCAAGCUUUAAGUCACCUUACAGUACAAUUGCAAAAUAAUUUUCAAGCCAUUAGUA
GUUCUAUUAGUGAUAUUUAUAACAGGCUUGACGAACUGAGUGCUGAUGCACAAGUUGAUAGGCUGAUUACAG
GUAGACUUACAGCACUUAAUGCAUUUGUGUCUCAGACUCUAACCAGACAAGCAGAGGUUAGGGCUAGUAGACA
ACUUGCCAAAGACAAGGUUAAUGAAUGUGUUAGGUCUCAGUCUCAGAGAUUCGGAUUCUGUGGUAAUGGUAC
ACAUUUGUUUUCACUAGCAAAUGCAGCACCAAAUGGCAUGAUUUUCUUUCAUACAGUACUAUUACCAACAGCU
UAUGAAACUGUAACAGCUUGGUCAGGUAUUUGUGCUUCAGAUGGCGAUCGCACUUUCGGACUUGUCGUUAAA
GAUGUGCAGUUGACGUUGUUUCGUAAUCUAGAUGACAAGUUCUAUUUGACCCCCAGAACUAUGUAUCAGCCU
AGAGUUGCAACUAGUUCUGAUUUUGUUCAAAUUGAAGGGUGUGAUGUGUUGUUUGUCAACGCGACUGUAAU
UGAUUUGCCUAGUAUUAUACCUGACUAUAUUGACAUUAAUCAAACUGUUCAAGACAUAUUAGAAAAUUACAGA
CCAAACUGGACUGUACCUGAAUUUACACUUGAUAUUUUCAACGCAACCUAUUUAAAUCUGACUGGUGAAAUUG
AUGACUUAGAGUUUAGGUCAGAAAAGCUACAUAACACUACAGUAGAACUUGCCAUUCUCAUUGAUAACAUUAA
UAAUACAUUAGUCAAUCUUGAAUGGCUCAAUAGAAUUGAAACUUAUGUAAAAUGGCCUUGGUAUGUGUGGCU
ACUGAUAGGUUUAGUAGUAGUAUUUUGCAUACCAUUACUGCUAUUUUGCUGUUUUAGCACAGGUUGUUGUG
GAUGCAUAGGUUGUUUAGGAAGUUGUUGUCACUCUAUAUGUAGUAGAAGACAAUUUGAAAAUUAUGAACCAA
UUGAAAAAGUGCAUGUCCACGGCAGCGGCGAAGGCCGCGGCAGCCUGCUGACCUGCGGCGAUGUGGAAGAAAA
CCCGGGCCCGAUGAAGUACAUUUUGUUAAUACUCGCGUGCAUUAUUGCAUGCGUUUAUGGAGAACGCUACUG
UGCCAUGCAAAAUACAGGCUCGCAGUGCAUUAAUGGCACAGAUUCAUCAUGUAGCACCUGUUUUGAACGUGGU
GGUCUUAUUUGGCAUCUGGCUAACUGGAACUUCAGCUGGUCUGUAAUAUUGAUUGUUUUUAUAACAGUGUU
AAAAUAUGGAAGACCGCAAUUCAGCUGGCUCGUUUAUGGCAUUAAAAUGCUGAUCAUGUGGCUAUUAUGGCC
UAUUGUUCUAGCGCUUACGAUUUUUAAUGCAUACUCUGAGUACCAAGUUUCCAGAUAUGUAAUGUUCGGCUU
UAGUGUUGCAGGUGCAGUUGUAACGUUUGCACUAUGGAUGAUGUAUUUUGUGAGAUCUAUUCAGCUGUAUA
 
GACGGACCAAAUCAUGGUGGUCUUUUAAUCCUGAAACCAAUGCGAUUCUUUGUGUCAAUGCAUUGGGUAGAA
GCUAUGUACUCCCUCUUGAUGGCACUCCUACAGGUGUUACUCUUACCCUACUUUCAGGAAAUCUAUACGCUGA
AGGUUUUAAAAUGGCUGGUGGUCUUACCAUCGAGCAUUUGCCUAAAUAUGUCAUGAUUGCUACGCCUAGUAG
AACCAUCGUUUACACAUUAGUUGGAAAACAACUAAAGGCAACUACUGCCACUGGAUGGGCUUACUAUGUAAAA
UCUAAAGCUGGUGAUUACUCAACAGAAGCACGUACUGAUAAUUUGAGUGAACAUGAAAAAUUAUUACAUAUG
GUGUAAGCUCCCUUUCUUGCUUUCCAGGGAAAGUCCCUUGGUCCCCAGACCCUAAUACCUGGACAUGUCUAAA
UCAUGAAAUUCUUUGAACAUCUGGCUCCUGCCUAAUAAAGAAUAUUUAUUUUCAUUGCAAAAAAAAAAAAAAA
AAAAAAAAAAAAAAAAAAAAAAAAAAAAAAAAAAAAAAAAAAAAAAAAAAAAAAAAAAAAAAAAAAAAAAAAAA
AAAAAAAAAAA
   5'-UTR+Kozak+N+M+S+3'-UTR+100 poly(A)(P2A) sequence (SEQ ID NO:54) 
GGAUAGCUACCAGCUUACACUUGCUUCUGACACAACUGUGUUCGCUAUCAACCACACAAACAGACACCGCCACCA
UGGCCACACAGGGACAACGCGUCAACUGGGGAGAUGAACCUUCCAAAAGACGUGGUCGUUCUAACUCUCGUGG
UCGGAAGAAUAACACUAUACCUCUUUCAUUCUUCAAUCCCAUCCAACUCGAACCAGGAUCAAAAUUUUGGAGCG
UAUGUCCGAGAGAUUUUGUUCCCAAGGGAAUAGGUAACAAGGAUCAACAAAUUGGUUAUUGGAAUAGACAAG
AGCGUUACCGCAUUGUCAAAGGUCAGCGUAAGGAACUUCCUGAGAGGUGGUUUUUCUACUUCUUAGGCACAG
GACCUCAAGCUGAUGCUAAAUUUAAAGACAAGAUUGAUGGAGUCUUCUGGGUUGCAAAGGAUGGUGCCAUGA
AUAAACCAACAACACUUGGCACUCGUGGUACCAACAAUGAAUCCAAACCACUGAAAUUUGAUGGUAAGAUACCA
CCGCAAUUUCAGCUUGAAGUGAACCGAUCUAGGAACAACUCAAGAAGUGGUUCUCAGUCUAGAUCUGUCUCUA
GAAACAGGUCUCAAUCCAGAGGAAGACAACAAUCCAAUAAUCAGAAUAAUGUUGAGGAUACAAUUGUAGCUGU
GCUUCAGAAAUUAGGUGUUACUGAAAAGCAAAGGUCACGUUCUAAAUCUAGAGAUCGUGGUGACUCUAAACCU
AGAGACACAACACCUAAUAAUGCCAACAAACACACCUGGAAGAAGACUGCAGGUAAAGGUGAUGUGACAAAUUU
CUAUGGUGCUAGAAGUGCUUCAGCUAACUUUGGUGAUAGUGAUCUCGUUGCCAACGGUAACGCUGCCAAAUCC
UACCCUCAGAUAGCUGAAUGCGUUCCAUCAGUGUCUAGCAUGCUCUUCGGUAGUCAAUGGUCUGCUGAAGAUG
AUGGUGAUCAAGUGAAAGUCACGCUCACUCAUACCUAUUACCUGCCAAAAGAUGAUGCCAAAACCAGCCAAUUC
CUAGAACAGAUUGACGCUUACAAGCGGCCAUCUCAAGUGGCUAAAGAUCAGAGGCAAAGAAAAUCUCGUUCUA
AGUCUGCUGAGAAGAAGCCUGAGGAAUUGUCUGUAACUCUUGUAGAGGCAUAUACGGAUGUGUUUGAUGACA
CACAGGUUGAGAUGAUUGAUGAGGUUACGAACGGCAGCGGCGCGACCAACUUUAGCCUGCUGAAACAGGCGGG
CGAUGUGGAAGAAAACCCGGGCCCGAUGAAGUACAUUUUGUUAAUACUCGCGUGCAUUAUUGCAUGCGUUUA
UGGAGAACGCUACUGUGCCAUGCAAAAUACAGGCUCGCAGUGCAUUAAUGGCACAGAUUCAUCAUGUAGCACC
UGUUUUGAACGUGGUGGUCUUAUUUGGCAUCUGGCUAACUGGAACUUCAGCUGGUCUGUAAUAUUGAUUGU
UUUUAUAACAGUGUUAAAAUAUGGAAGACCGCAAUUCAGCUGGCUCGUUUAUGGCAUUAAAAUGCUGAUCAU
GUGGCUAUUAUGGCCUAUUGUUCUAGCGCUUACGAUUUUUAAUGCAUACUCUGAGUACCAAGUUUCCAGAUA
UGUAAUGUUCGGCUUUAGUGUUGCAGGUGCAGUUGUAACGUUUGCACUAUGGAUGAUGUAUUUUGUGAGAU
CUAUUCAGCUGUAUAGACGGACCAAAUCAUGGUGGUCUUUUAAUCCUGAAACCAAUGCGAUUCUUUGUGUCAA
UGCAUUGGGUAGAAGCUAUGUACUCCCUCUUGAUGGCACUCCUACAGGUGUUACUCUUACCCUACUUUCAGGA
AAUCUAUACGCUGAAGGUUUUAAAAUGGCUGGUGGUCUUACCAUCGAGCAUUUGCCUAAAUAUGUCAUGAUU
GCUACGCCUAGUAGAACCAUCGUUUACACAUUAGUUGGAAAACAACUAAAGGCAACUACUGCCACUGGAUGGG
CUUACUAUGUAAAAUCUAAAGCUGGUGAUUACUCAACAGAAGCACGUACUGAUAAUUUGAGUGAACAUGAAAA
AUUAUUACAUAUGGUGGGCAGCGGCGCGACCAACUUUAGCCUGCUGAAACAGGCGGGCGAUGUGGAAGAAAAC
CCGGGCCCGAUGAUUGUGCUCGUAACUUGCCUCUUGUUGUUAUGUUCAUACCACACAGUUUUGAGUACAACAA
AUAAUGAAUGCAUACAAGUUAACGUAACACAAUUGGCUGGCAAUGAAAACCUUAUCAGAGAUUUUCUGUUUAG
UAACUUUAAAGAAGAAGGAAGUGUAGUUGUUGGUGGUUAUUACCCUACAGAGGUGUGGUACAACUGCUCUAG
AACAGCUCGAACUACUGCCUUUCAGUAUUUUAAUAAUAUACAUGCCUUUUAUUUUGUUAUGGAAGCCAUGGA
AAAUAGCACUGGUAAUGCACGUGGUAAACCAUUAUUAUUUCAUGUGCAUGGUGAGCCUGUUAGUGUUAUUAU
AUCGGCUUAUAGGGAUGAUGUGCAACAAAGGCCCCUUUUAAAACAUGGGUUAGUGUGCAUAACUAAAAAUCGC
CAUAUUAACUAUGAACAAUUCACCUCCAACCAGUGGAAUUCCACAUGUACGGGUGCUGACAGAAAAAUUCCUU
UCUCUGUCAUACCCACGGACAAUGGAACAAAAAUCUAUGGUCUUGAGUGGAAUGAUGACUUUGUUACAGCUUA
UAUUAGUGGUCGUUCUUAUCACUUGAACAUCAAUACUAAUUGGUUUAACAAUGUCACACUUUUGUAUUCACG
CUCAAGCACUGCUACCUGGGAAUACAGUGCUGCAUAUGCUUACCAAGGUGUUUCUAACUUCACUUAUUACAAG
UUAAAUAACACCAAUGGUCUAAAAACCUAUGAAUUAUGUGAAGAUUAUGAACAUUGCACUGGCUAUGCUACCA
AUGUAUUUGCUCCGACAUCAGGUGGUUACAUACCUGAUGGAUUUAGUUUUAACAAUUGGUUCUUGCUUACAA
AUAGUUCCACUUUUGUUAGUGGCAGGUUUGUAACAAAUCAACCAUUAUUGAUUAAUUGCUUGUGGCCAGUGC
CCAGUUUUGGUGUAGCAGCACAAGAAUUUUGUUUUGAAGGUGCACAGUUUAGCCAAUGUAAUGGUGUGUCU
UUAAAUAACACAGUGGAUGUUAUUAGAUUCAACCUUAAUUUCACUGCAGAUGUACAAUCUGGUAUGGGUGCU
ACAGUAUUUUCACUGAAUACAACAGGUGGUGUCAUUCUUGAAAUUUCAUGUUAUAGUGACACAGUGAGUGAG
UCUAGUUCUUACAGUUAUGGUGAAAUCCCGUUCGGCAUAACUGACGGACCACGAUACUGUUAUGUACUUUACA
AUGGCACAGCUCUUAAAUAUUUAGGAACAUUACCACCCAGUGUAAAGGAAAUUGCUAUUAGUAAGUGGGGCCA
UUUUUAUAUUAAUGGUUACAAUUUCUUUAGCACAUUUCCUAUUGGUUGUAUAUCUUUUAAUUUAACCACUG
GUGUUAGUGGAGCUUUUUGGACAAUUGCUUACACAUCGUAUACUGAAGCAUUAGUACAAGUUGAAAACACAG
CUAUUAAAAAUGUGACGUAUUGUAACAGUCACAUUAAUAACAUUAAAUGUUCUCAACUUACUGCUAAUUUGAA
UAAUGGAUUUUAUCCUGUUGCUUCAAGUGAAGUAGGUUUCGUUAAUAAGAGUGUUGUGUUAUUACCUAGCU
UUUUCACAUACACCGCUGUCAAUAUAACCAUUGAUCUUGGUAUGAAGCUUAGUGGUUAUGGUCAACCCAUAGC
CUCGACACUAAGUAACAUCACACUACCAAUGCAGGAUAACAAUACUGAUGUGUACUGUAUUCGUUCUAACCAAU
 
UCUCAGUUUAUGUUCAUUCCACUUGCAAAAGUUCUUUAUGGGACAAUAUUUUUAAUCAAGACUGCACGGAUG
UUUUAGAGGCUACAGCUGUUAUAAAAACUGGUACUUGUCCUUUCUCAUUUGAUAAAUUGAACAAUUACUUGA
CUUUUAACAAGUUCUGUUUGUCGUUGAGUCCUGUUGGUGCUAAUUGCAAGUUUGAUGUUGCUGCACGUACA
AGAACCAAUGAGCAGGUUGUUAGAAGUCUAUAUGUAAUAUAUGAAGAAGGAGACAACAUAGUGGGUGUACCG
UCUGAUAAUAGCGGUCUGCACGAUUUGUCUGUGCUACACCUAGACUCCUGUACAGAUUACAAUAUAUAUGGUA
GAACUGGUGUUGGUAUUAUUAGACGAACUAACAGUACGCUACUUAGUGGCUUAUAUUACACAUCACUAUCAG
GUGAUUUGUUAGGCUUUAAAAAUGUUAGUGAUGGUGUCAUUUAUUCUGUGACGCCAUGUGAUGUAAGCGCA
CAAGCGGCUGUUAUUGAUGGUGCCAUAGUUGGAGCUAUGACUUCCAUUAACAGUGAACUGUUAGGUCUAACA
CAUUGGACAACGACACCUAAUUUUUAUUACUACUCUAUAUAUAAUUACACAAGUGAGAGGACUCGUGACACUG
CAAUUGACAGUAACGAUGUUGAUUGUGAACCUGUCAUAACCUAUUCUAAUAUAGGUGUUUGUAAAAAUGGUG
CUUUGGUUUUUAUUAACGUCACACAUUCUGACGGAGACGUGCAACCAAUUAGCACUGGUAAUGUCACGAUACC
UACAAAUUUUACCAUAUCUGUGCAAGUUGAAUACAUGCAGGUUUACACUACACCAGUAUCAAUAGAUUGUGCA
AGAUACGUUUGUAAUGGUAACCCUAGAUGUAACAAAUUGUUAACACAAUAUGUGUCUGCAUGUCAAACUAUU
GAACAAGCACUUGCAAUGGGUGCCAGACUUGAAAACAUGGAGGUUGAUUCCAUGUUGUUUGUCUCGGAAAAU
GCCCUUAAAUUGGCAUCUGUUGAGGCGUUCAAUAGUACAGAAAAUUUAGAUUCUAUUUACAAAGAAUGGCCU
AGCAUAGGUGGUUCUUGGCUAGGAGGUCUAAAAGAUAUACUACCGUCCCAUAAUAGCAAACGUAAGUAUGGU
UCUGCUAUAGAAGAUUUGCUUUUUGAUAAAGUUGUAACAUCUGGUUUAGGUACAGUUGAUGAAGAUUAUAA
ACGUUGUACUGGUGGUUACGACAUAGCAGACUUGGUGUGUGCUCAAUAUUACAAUGGCAUCAUGGUUCUACC
AGGUGUAGCUAAUGCUGACAAGAUGACUAUGUACACAGCAUCACUUGCAGGUGGUAUAACAUUAGGUGCACU
UGGUGGUGGCGCCGUGGCUAUACCUUUUGCAGUAGCAGUACAGGCUAGACUUAAUUAUGUUGCUCUACAAAC
UGAUGUAUUGAAUAAAAACCAACAGAUCCUGGCUAAUGCCUUCAAUCAAGCUAUUGGUAACAUUACACAGGCU
UUUGGUAAGGUUAAUGAUGCUAUACAUCAAACAUCACAAGGUCUUGCCACUGUUGCUAAAGCGUUGGCAAAAG
UGCAAGAUGUUGUCAACACACAAGGGCAAGCUUUAAGUCACCUUACAGUACAAUUGCAAAAUAAUUUUCAAGC
CAUUAGUAGUUCUAUUAGUGAUAUUUAUAACAGGCUUGACGAACUGAGUGCUGAUGCACAAGUUGAUAGGCU
GAUUACAGGUAGACUUACAGCACUUAAUGCAUUUGUGUCUCAGACUCUAACCAGACAAGCAGAGGUUAGGGCU
AGUAGACAACUUGCCAAAGACAAGGUUAAUGAAUGUGUUAGGUCUCAGUCUCAGAGAUUCGGAUUCUGUGGU
AAUGGUACACAUUUGUUUUCACUAGCAAAUGCAGCACCAAAUGGCAUGAUUUUCUUUCAUACAGUACUAUUAC
CAACAGCUUAUGAAACUGUAACAGCUUGGUCAGGUAUUUGUGCUUCAGAUGGCGAUCGCACUUUCGGACUUG
UCGUUAAAGAUGUGCAGUUGACGUUGUUUCGUAAUCUAGAUGACAAGUUCUAUUUGACCCCCAGAACUAUGU
AUCAGCCUAGAGUUGCAACUAGUUCUGAUUUUGUUCAAAUUGAAGGGUGUGAUGUGUUGUUUGUCAACGCG
ACUGUAAUUGAUUUGCCUAGUAUUAUACCUGACUAUAUUGACAUUAAUCAAACUGUUCAAGACAUAUUAGAA
AAUUACAGACCAAACUGGACUGUACCUGAAUUUACACUUGAUAUUUUCAACGCAACCUAUUUAAAUCUGACUG
GUGAAAUUGAUGACUUAGAGUUUAGGUCAGAAAAGCUACAUAACACUACAGUAGAACUUGCCAUUCUCAUUGA
UAACAUUAAUAAUACAUUAGUCAAUCUUGAAUGGCUCAAUAGAAUUGAAACUUAUGUAAAAUGGCCUUGGUA
UGUGUGGCUACUGAUAGGUUUAGUAGUAGUAUUUUGCAUACCAUUACUGCUAUUUUGCUGUUUUAGCACAG
GUUGUUGUGGAUGCAUAGGUUGUUUAGGAAGUUGUUGUCACUCUAUAUGUAGUAGAAGACAAUUUGAAAAU
UAUGAACCAAUUGAAAAAGUGCAUGUCCACUAAGCUCCCUUUCUUGCUUUCCAGGGAAAGUCCCUUGGUCCCC
AGACCCUAAUACCUGGACAUGUCUAAAUCAUGAAAUUCUUUGAACAUCUGGCUCCUGCCUAAUAAAGAAUAUU
UAUUUUCAUUGCAAAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAA
   5'-UTR+Kozak+N+S+M+3'-UTR+100 poly(A)(P2A) sequence (SEQ ID NO:55) 
GGAUAGCUACCAGCUUACACUUGCUUCUGACACAACUGUGUUCGCUAUCAACCACACAAACAGACACCGCCACCA
UGGCCACACAGGGACAACGCGUCAACUGGGGAGAUGAACCUUCCAAAAGACGUGGUCGUUCUAACUCUCGUGG
UCGGAAGAAUAACACUAUACCUCUUUCAUUCUUCAAUCCCAUCCAACUCGAACCAGGAUCAAAAUUUUGGAGCG
UAUGUCCGAGAGAUUUUGUUCCCAAGGGAAUAGGUAACAAGGAUCAACAAAUUGGUUAUUGGAAUAGACAAG
AGCGUUACCGCAUUGUCAAAGGUCAGCGUAAGGAACUUCCUGAGAGGUGGUUUUUCUACUUCUUAGGCACAG
GACCUCAAGCUGAUGCUAAAUUUAAAGACAAGAUUGAUGGAGUCUUCUGGGUUGCAAAGGAUGGUGCCAUGA
AUAAACCAACAACACUUGGCACUCGUGGUACCAACAAUGAAUCCAAACCACUGAAAUUUGAUGGUAAGAUACCA
CCGCAAUUUCAGCUUGAAGUGAACCGAUCUAGGAACAACUCAAGAAGUGGUUCUCAGUCUAGAUCUGUCUCUA
GAAACAGGUCUCAAUCCAGAGGAAGACAACAAUCCAAUAAUCAGAAUAAUGUUGAGGAUACAAUUGUAGCUGU
GCUUCAGAAAUUAGGUGUUACUGAAAAGCAAAGGUCACGUUCUAAAUCUAGAGAUCGUGGUGACUCUAAACCU
AGAGACACAACACCUAAUAAUGCCAACAAACACACCUGGAAGAAGACUGCAGGUAAAGGUGAUGUGACAAAUUU
CUAUGGUGCUAGAAGUGCUUCAGCUAACUUUGGUGAUAGUGAUCUCGUUGCCAACGGUAACGCUGCCAAAUCC
UACCCUCAGAUAGCUGAAUGCGUUCCAUCAGUGUCUAGCAUGCUCUUCGGUAGUCAAUGGUCUGCUGAAGAUG
AUGGUGAUCAAGUGAAAGUCACGCUCACUCAUACCUAUUACCUGCCAAAAGAUGAUGCCAAAACCAGCCAAUUC
CUAGAACAGAUUGACGCUUACAAGCGGCCAUCUCAAGUGGCUAAAGAUCAGAGGCAAAGAAAAUCUCGUUCUA
AGUCUGCUGAGAAGAAGCCUGAGGAAUUGUCUGUAACUCUUGUAGAGGCAUAUACGGAUGUGUUUGAUGACA
CACAGGUUGAGAUGAUUGAUGAGGUUACGAACGGCAGCGGCGCGACCAACUUUAGCCUGCUGAAACAGGCGGG
CGAUGUGGAAGAAAACCCGGGCCCGAUGAUUGUGCUCGUAACUUGCCUCUUGUUGUUAUGUUCAUACCACACA
GUUUUGAGUACAACAAAUAAUGAAUGCAUACAAGUUAACGUAACACAAUUGGCUGGCAAUGAAAACCUUAUCA
GAGAUUUUCUGUUUAGUAACUUUAAAGAAGAAGGAAGUGUAGUUGUUGGUGGUUAUUACCCUACAGAGGUG
UGGUACAACUGCUCUAGAACAGCUCGAACUACUGCCUUUCAGUAUUUUAAUAAUAUACAUGCCUUUUAUUUU
GUUAUGGAAGCCAUGGAAAAUAGCACUGGUAAUGCACGUGGUAAACCAUUAUUAUUUCAUGUGCAUGGUGAG
 
CCUGUUAGUGUUAUUAUAUCGGCUUAUAGGGAUGAUGUGCAACAAAGGCCCCUUUUAAAACAUGGGUUAGUG
UGCAUAACUAAAAAUCGCCAUAUUAACUAUGAACAAUUCACCUCCAACCAGUGGAAUUCCACAUGUACGGGUGC
UGACAGAAAAAUUCCUUUCUCUGUCAUACCCACGGACAAUGGAACAAAAAUCUAUGGUCUUGAGUGGAAUGAU
GACUUUGUUACAGCUUAUAUUAGUGGUCGUUCUUAUCACUUGAACAUCAAUACUAAUUGGUUUAACAAUGUC
ACACUUUUGUAUUCACGCUCAAGCACUGCUACCUGGGAAUACAGUGCUGCAUAUGCUUACCAAGGUGUUUCUA
ACUUCACUUAUUACAAGUUAAAUAACACCAAUGGUCUAAAAACCUAUGAAUUAUGUGAAGAUUAUGAACAUUG
CACUGGCUAUGCUACCAAUGUAUUUGCUCCGACAUCAGGUGGUUACAUACCUGAUGGAUUUAGUUUUAACAA
UUGGUUCUUGCUUACAAAUAGUUCCACUUUUGUUAGUGGCAGGUUUGUAACAAAUCAACCAUUAUUGAUUAA
UUGCUUGUGGCCAGUGCCCAGUUUUGGUGUAGCAGCACAAGAAUUUUGUUUUGAAGGUGCACAGUUUAGCCA
AUGUAAUGGUGUGUCUUUAAAUAACACAGUGGAUGUUAUUAGAUUCAACCUUAAUUUCACUGCAGAUGUACA
AUCUGGUAUGGGUGCUACAGUAUUUUCACUGAAUACAACAGGUGGUGUCAUUCUUGAAAUUUCAUGUUAUAG
UGACACAGUGAGUGAGUCUAGUUCUUACAGUUAUGGUGAAAUCCCGUUCGGCAUAACUGACGGACCACGAUAC
UGUUAUGUACUUUACAAUGGCACAGCUCUUAAAUAUUUAGGAACAUUACCACCCAGUGUAAAGGAAAUUGCUA
UUAGUAAGUGGGGCCAUUUUUAUAUUAAUGGUUACAAUUUCUUUAGCACAUUUCCUAUUGGUUGUAUAUCU
UUUAAUUUAACCACUGGUGUUAGUGGAGCUUUUUGGACAAUUGCUUACACAUCGUAUACUGAAGCAUUAGUA
CAAGUUGAAAACACAGCUAUUAAAAAUGUGACGUAUUGUAACAGUCACAUUAAUAACAUUAAAUGUUCUCAAC
UUACUGCUAAUUUGAAUAAUGGAUUUUAUCCUGUUGCUUCAAGUGAAGUAGGUUUCGUUAAUAAGAGUGUU
GUGUUAUUACCUAGCUUUUUCACAUACACCGCUGUCAAUAUAACCAUUGAUCUUGGUAUGAAGCUUAGUGGU
UAUGGUCAACCCAUAGCCUCGACACUAAGUAACAUCACACUACCAAUGCAGGAUAACAAUACUGAUGUGUACUG
UAUUCGUUCUAACCAAUUCUCAGUUUAUGUUCAUUCCACUUGCAAAAGUUCUUUAUGGGACAAUAUUUUUAA
UCAAGACUGCACGGAUGUUUUAGAGGCUACAGCUGUUAUAAAAACUGGUACUUGUCCUUUCUCAUUUGAUAA
AUUGAACAAUUACUUGACUUUUAACAAGUUCUGUUUGUCGUUGAGUCCUGUUGGUGCUAAUUGCAAGUUUG
AUGUUGCUGCACGUACAAGAACCAAUGAGCAGGUUGUUAGAAGUCUAUAUGUAAUAUAUGAAGAAGGAGACA
ACAUAGUGGGUGUACCGUCUGAUAAUAGCGGUCUGCACGAUUUGUCUGUGCUACACCUAGACUCCUGUACAGA
UUACAAUAUAUAUGGUAGAACUGGUGUUGGUAUUAUUAGACGAACUAACAGUACGCUACUUAGUGGCUUAUA
UUACACAUCACUAUCAGGUGAUUUGUUAGGCUUUAAAAAUGUUAGUGAUGGUGUCAUUUAUUCUGUGACGCC
AUGUGAUGUAAGCGCACAAGCGGCUGUUAUUGAUGGUGCCAUAGUUGGAGCUAUGACUUCCAUUAACAGUGA
ACUGUUAGGUCUAACACAUUGGACAACGACACCUAAUUUUUAUUACUACUCUAUAUAUAAUUACACAAGUGAG
AGGACUCGUGACACUGCAAUUGACAGUAACGAUGUUGAUUGUGAACCUGUCAUAACCUAUUCUAAUAUAGGU
GUUUGUAAAAAUGGUGCUUUGGUUUUUAUUAACGUCACACAUUCUGACGGAGACGUGCAACCAAUUAGCACU
GGUAAUGUCACGAUACCUACAAAUUUUACCAUAUCUGUGCAAGUUGAAUACAUGCAGGUUUACACUACACCAG
UAUCAAUAGAUUGUGCAAGAUACGUUUGUAAUGGUAACCCUAGAUGUAACAAAUUGUUAACACAAUAUGUGU
CUGCAUGUCAAACUAUUGAACAAGCACUUGCAAUGGGUGCCAGACUUGAAAACAUGGAGGUUGAUUCCAUGUU
GUUUGUCUCGGAAAAUGCCCUUAAAUUGGCAUCUGUUGAGGCGUUCAAUAGUACAGAAAAUUUAGAUUCUAU
UUACAAAGAAUGGCCUAGCAUAGGUGGUUCUUGGCUAGGAGGUCUAAAAGAUAUACUACCGUCCCAUAAUAGC
AAACGUAAGUAUGGUUCUGCUAUAGAAGAUUUGCUUUUUGAUAAAGUUGUAACAUCUGGUUUAGGUACAGU
UGAUGAAGAUUAUAAACGUUGUACUGGUGGUUACGACAUAGCAGACUUGGUGUGUGCUCAAUAUUACAAUGG
CAUCAUGGUUCUACCAGGUGUAGCUAAUGCUGACAAGAUGACUAUGUACACAGCAUCACUUGCAGGUGGUAUA
ACAUUAGGUGCACUUGGUGGUGGCGCCGUGGCUAUACCUUUUGCAGUAGCAGUACAGGCUAGACUUAAUUAU
GUUGCUCUACAAACUGAUGUAUUGAAUAAAAACCAACAGAUCCUGGCUAAUGCCUUCAAUCAAGCUAUUGGUA
ACAUUACACAGGCUUUUGGUAAGGUUAAUGAUGCUAUACAUCAAACAUCACAAGGUCUUGCCACUGUUGCUAA
AGCGUUGGCAAAAGUGCAAGAUGUUGUCAACACACAAGGGCAAGCUUUAAGUCACCUUACAGUACAAUUGCAA
AAUAAUUUUCAAGCCAUUAGUAGUUCUAUUAGUGAUAUUUAUAACAGGCUUGACGAACUGAGUGCUGAUGCA
CAAGUUGAUAGGCUGAUUACAGGUAGACUUACAGCACUUAAUGCAUUUGUGUCUCAGACUCUAACCAGACAAG
CAGAGGUUAGGGCUAGUAGACAACUUGCCAAAGACAAGGUUAAUGAAUGUGUUAGGUCUCAGUCUCAGAGAU
UCGGAUUCUGUGGUAAUGGUACACAUUUGUUUUCACUAGCAAAUGCAGCACCAAAUGGCAUGAUUUUCUUUC
AUACAGUACUAUUACCAACAGCUUAUGAAACUGUAACAGCUUGGUCAGGUAUUUGUGCUUCAGAUGGCGAUC
GCACUUUCGGACUUGUCGUUAAAGAUGUGCAGUUGACGUUGUUUCGUAAUCUAGAUGACAAGUUCUAUUUGA
CCCCCAGAACUAUGUAUCAGCCUAGAGUUGCAACUAGUUCUGAUUUUGUUCAAAUUGAAGGGUGUGAUGUGU
UGUUUGUCAACGCGACUGUAAUUGAUUUGCCUAGUAUUAUACCUGACUAUAUUGACAUUAAUCAAACUGUUC
AAGACAUAUUAGAAAAUUACAGACCAAACUGGACUGUACCUGAAUUUACACUUGAUAUUUUCAACGCAACCUA
UUUAAAUCUGACUGGUGAAAUUGAUGACUUAGAGUUUAGGUCAGAAAAGCUACAUAACACUACAGUAGAACU
UGCCAUUCUCAUUGAUAACAUUAAUAAUACAUUAGUCAAUCUUGAAUGGCUCAAUAGAAUUGAAACUUAUGU
AAAAUGGCCUUGGUAUGUGUGGCUACUGAUAGGUUUAGUAGUAGUAUUUUGCAUACCAUUACUGCUAUUUU
GCUGUUUUAGCACAGGUUGUUGUGGAUGCAUAGGUUGUUUAGGAAGUUGUUGUCACUCUAUAUGUAGUAGA
AGACAAUUUGAAAAUUAUGAACCAAUUGAAAAAGUGCAUGUCCACUAAGGCAGCGGCGCGACCAACUUUAGCC
UGCUGAAACAGGCGGGCGAUGUGGAAGAAAACCCGGGCCCGAUGAAGUACAUUUUGUUAAUACUCGCGUGCAU
UAUUGCAUGCGUUUAUGGAGAACGCUACUGUGCCAUGCAAAAUACAGGCUCGCAGUGCAUUAAUGGCACAGAU
UCAUCAUGUAGCACCUGUUUUGAACGUGGUGGUCUUAUUUGGCAUCUGGCUAACUGGAACUUCAGCUGGUCU
GUAAUAUUGAUUGUUUUUAUAACAGUGUUAAAAUAUGGAAGACCGCAAUUCAGCUGGCUCGUUUAUGGCAUU
AAAAUGCUGAUCAUGUGGCUAUUAUGGCCUAUUGUUCUAGCGCUUACGAUUUUUAAUGCAUACUCUGAGUAC
CAAGUUUCCAGAUAUGUAAUGUUCGGCUUUAGUGUUGCAGGUGCAGUUGUAACGUUUGCACUAUGGAUGAU
GUAUUUUGUGAGAUCUAUUCAGCUGUAUAGACGGACCAAAUCAUGGUGGUCUUUUAAUCCUGAAACCAAUGC
 
GAUUCUUUGUGUCAAUGCAUUGGGUAGAAGCUAUGUACUCCCUCUUGAUGGCACUCCUACAGGUGUUACUCU
UACCCUACUUUCAGGAAAUCUAUACGCUGAAGGUUUUAAAAUGGCUGGUGGUCUUACCAUCGAGCAUUUGCCU
AAAUAUGUCAUGAUUGCUACGCCUAGUAGAACCAUCGUUUACACAUUAGUUGGAAAACAACUAAAGGCAACUA
CUGCCACUGGAUGGGCUUACUAUGUAAAAUCUAAAGCUGGUGAUUACUCAACAGAAGCACGUACUGAUAAUUU
GAGUGAACAUGAAAAAUUAUUACAUAUGGUGUAAGCUCCCUUUCUUGCUUUCCAGGGAAAGUCCCUUGGUCCC
CAGACCCUAAUACCUGGACAUGUCUAAAUCAUGAAAUUCUUUGAACAUCUGGCUCCUGCCUAAUAAAGAAUAU
UUAUUUUCAUUGCAAAAAAAAAAAAAAAAAAAAAAAAAAAAAAAAAAAAAAAAAAAAAAAAAAAAAAAAAAAA
AAAAAAAAAAAAAAAAAAAAAAAAAAAAAAAAAAAAAAAA
   5'-UTR+Kozak+N+M+S2+3'-UTR+100 poly(A)(P2A) sequence (SEQ ID NO:56) 
GGAUAGCUACCAGCUUACACUUGCUUCUGACACAACUGUGUUCGCUAUCAACCACACAAACAGACACCGCCACCA
UGGCCACACAGGGACAACGCGUCAACUGGGGAGAUGAACCUUCCAAAAGACGUGGUCGUUCUAACUCUCGUGG
UCGGAAGAAUAACACUAUACCUCUUUCAUUCUUCAAUCCCAUCCAACUCGAACCAGGAUCAAAAUUUUGGAGCG
UAUGUCCGAGAGAUUUUGUUCCCAAGGGAAUAGGUAACAAGGAUCAACAAAUUGGUUAUUGGAAUAGACAAG
AGCGUUACCGCAUUGUCAAAGGUCAGCGUAAGGAACUUCCUGAGAGGUGGUUUUUCUACUUCUUAGGCACAG
GACCUCAAGCUGAUGCUAAAUUUAAAGACAAGAUUGAUGGAGUCUUCUGGGUUGCAAAGGAUGGUGCCAUGA
AUAAACCAACAACACUUGGCACUCGUGGUACCAACAAUGAAUCCAAACCACUGAAAUUUGAUGGUAAGAUACCA
CCGCAAUUUCAGCUUGAAGUGAACCGAUCUAGGAACAACUCAAGAAGUGGUUCUCAGUCUAGAUCUGUCUCUA
GAAACAGGUCUCAAUCCAGAGGAAGACAACAAUCCAAUAAUCAGAAUAAUGUUGAGGAUACAAUUGUAGCUGU
GCUUCAGAAAUUAGGUGUUACUGAAAAGCAAAGGUCACGUUCUAAAUCUAGAGAUCGGUGACUCUAAACCU
AGAGACACAACACCUAAUAAUGCCAACAAACACACCUGGAAGAAGACUGCAGGUAAAGGUGAUGUGACAAAUUU
CUAUGGUGCUAGAAGUGCUUCAGCUAACUUUGGUGAUAGUGAUCUCGUUGCCAACGGUAACGCUGCCAAAUCC
UACCCUCAGAUAGCUGAAUGCGUUCCAUCAGUGUCUAGCAUGCUCUUCGGUAGUCAAUGGUCUGCUGAAGAUG
AUGGUGAUCAAGUGAAAGUCACGCUCACUCAUACCUAUUACCUGCCAAAAGAUGAUGCCAAAACCAGCCAAUUC
CUAGAACAGAUUGACGCUUACAAGCGGCCAUCUCAAGUGGCUAAAGAUCAGAGGCAAAGAAAAUCUCGUUCUA
AGUCUGCUGAGAAGAAGCCUGAGGAAUUGUCUGUAACUCUUGUAGAGGCAUAUACGGAUGUGUUUGAUGACA
CACAGGUUGAGAUGAUUGAUGAGGUUACGAACGGCAGCGGCGCGACCAACUUUAGCCUGCUGAAACAGGCGGG
CGAUGUGGAAGAAAACCCGGGCCCGAUGAAGUACAUUUUGUUAAUACUCGCGUGCAUUAUUGCAUGCGUUUA
UGGAGAACGCUACUGUGCCAUGCAAAAUACAGGCUCGCAGUGCAUUAAUGGCACAGAUUCAUCAUGUAGCACC
UGUUUUGAACGUGGUGGUCUUAUUUGGCAUCUGGCUAACUGGAACUUCAGCUGGUCUGUAAUAUUGAUUGU
UUUUAUAACAGUGUUAAAAUAUGGAAGACCGCAAUUCAGCUGGCUCGUUUAUGGCAUUAAAAUGCUGAUCAU
GUGGCUAUUAUGGCCUAUUGUUCUAGCGCUUACGAUUUUUAAUGCAUACUCUGAGUACCAAGUUUCCAGAUA
UGUAAUGUUCGGCUUUAGUGUUGCAGGUGCAGUUGUAACGUUUGCACUAUGGAUGAUGUAUUUUGUGAGAU
CUAUUCAGCUGUAUAGACGGACCAAAUCAUGGUGGUCUUUUAAUCCUGAAACCAAUGCGAUUCUUUGUGUCAA
UGCAUUGGGUAGAAGCUAUGUACUCCCUCUUGAUGGCACUCCUACAGGUGUUACUCUUACCCUACUUUCAGGA
AAUCUAUACGCUGAAGGUUUUAAAAUGGCUGGUGGUCUUACCAUCGAGCAUUUGCCUAAAUAUGUCAUGAUU
GCUACGCCUAGUAGAACCAUCGUUUACACAUUAGUUGGAAAACAACUAAAGGCAACUACUGCCACUGGAUGGG
CUUACUAUGUAAAAUCUAAAGCUGGUGAUUACUCAACAGAAGCACGUACUGAUAAUUUGAGUGAACAUGAAAA
AUUAUUACAUAUGGUGGGCAGCGGCGCGACCAACUUUAGCCUGCUGAAACAGGCGGGCGAUGUGGAAGAAAAC
CCGGGCCCGAUGUCUAUAUAUAAUUACACAAGUGAGAGGACUCGUGACACUGCAAUUGACAGUAACGAUGUUG
AUUGUGAACCUGUCAUAACCUAUUCUAAUAUAGGUGUUUGUAAAAAUGGUGCUUUGGUUUUUAUUAACGUC
ACACAUUCUGACGGAGACGUGCAACCAAUUAGCACUGGUAAUGUCACGAUACCUACAAAUUUUACCAUAUCUG
UGCAAGUUGAAUACAUGCAGGUUUACACUACACCAGUAUCAAUAGAUUGUGCAAGAUACGUUUGUAAUGGUA
ACCCUAGAUGUAACAAAUUGUUAACACAAUAUGUGUCUGCAUGUCAAACUAUUGAACAAGCACUUGCAAUGGG
UGCCAGACUUGAAAACAUGGAGGUUGAUUCCAUGUUGUUUGUCUCGGAAAAUGCCCUUAAAUUGGCAUCUGU
UGAGGCGUUCAAUAGUACAGAAAAUUUAGAUUCUAUUUACAAAGAAUGGCCUAGCAUAGGUGGUUCUUGGCU
AGGAGGUCUAAAAGAUAUACUACCGUCCCAUAAUAGCAAACGUAAGUAUGGUUCUGCUAUAGAAGAUUUGCUU
UUUGAUAAAGUUGUAACAUCUGGUUUAGGUACAGUUGAUGAAGAUUAUAAACGUUGUACUGGUGGUUACGA
CAUAGCAGACUUGGUGUGUGCUCAAUAUUACAAUGGCAUCAUGGUUCUACCAGGUGUAGCUAAUGCUGACAA
GAUGACUAUGUACACAGCAUCACUUGCAGGUGGUAUAACAUUAGGUGCACUUGGUGGUGGCGCCGUGGCUAU
ACCUUUUGCAGUAGCAGUACAGGCUAGACUUAAUUAUGUUGCUCUACAAACUGAUGUAUUGAAUAAAAACCAA
CAGAUCCUGGCUAAUGCCUUCAAUCAAGCUAUUGGUAACAUUACACAGGCUUUUGGUAAGGUUAAUGAUGCU
AUACAUCAAACAUCACAAGGUCUUGCCACUGUUGCUAAAGCGUUGGCAAAAGUGCAAGAUGUUGUCAACACAC
AAGGGCAAGCUUUAAGUCACCUUACAGUACAAUUGCAAAAUAAUUUUCAAGCCAUUAGUAGUUCUAUUAGUGA
UAUUUAUAACAGGCUUGACGAACUGAGUGCUGAUGCACAAGUUGAUAGGCUGAUUACAGGUAGACUUACAGC
ACUUAAUGCAUUUGUGUCUCAGACUCUAACCAGACAAGCAGAGGUUAGGGCUAGUAGACAACUUGCCAAAGAC
AAGGUUAAUGAAUGUGUUAGGUCUCAGUCUCAGAGAUUCGGAUUCUGUGGUAAUGGUACACAUUUGUUUUC
ACUAGCAAAUGCAGCACCAAAUGGCAUGAUUUUCUUUCAUACAGUACUAUUACCAACAGCUUAUGAAACUGUA
ACAGCUUGGUCAGGUAUUUGUGCUUCAGAUGGCGAUCGCACUUUCGGACUUGUCGUUAAAGAUGUGCAGUUG
ACGUUGUUUCGUAAUCUAGAUGACAAGUUCUAUUUGACCCCCAGAACUAUGUAUCAGCCUAGAGUUGCAACUA
GUUCUGAUUUUGUUCAAAUUGAAGGGUGUGAUGUGUUGUUUGUCAACGCGACUGUAAUUGAUUUGCCUAGU
AUUAUACCUGACUAUAUUGACAUUAAUCAAACUGUUCAAGACAUAUUAGAAAAUUACAGACCAAACUGGACUG
UACCUGAAUUUACACUUGAUAUUUUCAACGCAACCUAUUUAAAUCUGACUGGUGAAAUUGAUGACUUAGAGU
UUAGGUCAGAAAAGCUACAUAACACUACAGUAGAACUUGCCAUUCUCAUUGAUAACAUUAAUAAUACAUUAGU
 
CAAUCUUGAAUGGCUCAAUAGAAUUGAAACUUAUGUAAAAUGGCCUUGGUAUGUGUGGCUACUGAUAGGUUU
AGUAGUAGUAUUUUGCAUACCAUUACUGCUAUUUUGCUGUUUUAGCACAGGUUGUUGUGGAUGCAUAGGUU
GUUUAGGAAGUUGUUGUCACUCUAUAUGUAGUAGAAGACAAUUUGAAAAUUAUGAACCAAUUGAAAAAGUGC
AUGUCCACUAAGCUCCCUUUCUUGCUUUCCAGGGAAAGUCCCUUGGUCCCCAGACCCUAAUACCUGGACAUGUC
UAAAUCAUGAAAUUCUUUGAACAUCUGGCUCCUGCCUAAUAAAGAAUAUUUAUUUUCAUUGCAAAAAAAAAAA
AAAAAAAAAAAAAAAAAAAAAAAAAAAAAAAAAAAAAAAAAAAAAAAAAAAAAAAAAAAAAAAAAAAAAAAAAA
AAAAAAAAAAAAAAA
   5'-UTR+Kozak+N+S2+M+3'-UTR+100 poly(A)(P2A) sequence (SEQ ID NO:57)

GGAUAGCUACCAGCUUACACUUGCUUCUGACACAACUGUGUUCGCUAUCAACCACACAAACAGACACCGCCACCA
UGGCCACACAGGGACAACGCGUCAACUGGGGAGAUGAACCUUCCAAAAGACGUGGUCGUUCUAACUCUCGUGG
UCGGAAGAAUAACACUAUACCUCUUUCAUUCUUCAAUCCCAUCCAACUCGAACCAGGAUCAAAAUUUUGGAGCG
UAUGUCCGAGAGAUUUUGUUCCCAAGGGAAUAGGUAACAAGGAUCAACAAAUUGGUUAUUGGAAUAGACAAG
AGCGUUACCGCAUUGUCAAAGGUCAGCGUAAGGAACUUCCUGAGAGGUGGUUUUUCUACUUCUUAGGCACAG
GACCUCAAGCUGAUGCUAAAUUUAAAGACAAGAUUGAUGGAGUCUUCUGGGUUGCAAAGGAUGGUGCCAUGA
AUAAACCAACAACACUUGGCACUCGUGGUACCAACAAUGAAUCCAAACCACUGAAAUUUGAUGGUAAGAUACCA
CCGCAAUUUCAGCUUGAAGUGAACCGAUCUAGGAACAACUCAAGAAGUGGUUCUCAGUCUAGAUCUGUCUCUA
GAAACAGGUCUCAAUCCAGAGGAAGACAACAAUCCAAUAAUCAGAAUAAUGUUGAGGAUACAAUUGUAGCUGU
GCUUCAGAAAUUAGGUGUUACUGAAAAGCAAAGGUCACGUUCUAAAUCUAGAGAUCGUGGUGACUCUAAACCU
AGAGACACAACACCUAAUAAUGCCAACAAACACACCUGGAAGAAGACUGCAGGUAAAGGUGAUGUGACAAAUUU
CUAUGGUGCUAGAAGUGCUUCAGCUAACUUUGGUGAUAGUGAUCUCGUUGCCAACGGUAACGCUGCCAAAUCC
UACCCUCAGAUAGCUGAAUGCGUUCCAUCAGUGUCUAGCAUGCUCUUCGGUAGUCAAUGGUCUGCUGAAGAUG
AUGGUGAUCAAGUGAAAGUCACGCUCACUCAUACCUAUUACCUGCCAAAAGAUGAUGCCAAAACCAGCCAAUUC
CUAGAACAGAUUGACGCUUACAAGCGGCCAUCUCAAGUGGCUAAAGAUCAGAGGCAAAGAAAAUCUCGUUCUA
AGUCUGCUGAGAAGAAGCCUGAGGAAUUGUCUGUAACUCUUGUAGAGGCAUAUACGGAUGUGUUUGAUGACA
CACAGGUUGAGAUGAUUGAUGAGGUUACGAACGGCAGCGGCGCGACCAACUUUAGCCUGCUGAAACAGGCGG
CGAUGUGGAAGAAAACCCGGGCCCGAUGUCUAUAUAUAAUUACACAAGUGAGAGGACUCGUGACACUGCAAUU
GACAGUAACGAUGUUGAUUGUGAACCUGUCAUAACCUAUUCUAAUAUAGGUGUUUGUAAAAAUGGUGCUUUG
GUUUUUAUUAACGUCACACAUUCUGACGGAGACGUGCAACCAAUUAGCACUGGUAAUGUCACGAUACCUACAA
AUUUUACCAUAUCUGUGCAAGUUGAAUACAUGCAGGUUUACACUACACCAGUAUCAAUAGAUUGUGCAAGAUA
CGUUUGUAAUGGUAACCCUAGAUGUAACAAAUUGUUAACACAAUAUGUGUCUGCAUGUCAAACUAUUGAACAA
GCACUUGCAAUGGGUGCCAGACUUGAAAACAUGGAGGUUGAUUCCAUGUUGUUUGUCUCGGAAAAUGCCCUU
AAAUUGGCAUCUGUUGAGGCGUUCAAUAGUACAGAAAAUUUAGAUUCUAUUUACAAAGAAUGGCCUAGCAUA
GGUGGUUCUUGGCUAGGAGGUCUAAAAGAUAUACUACCGUCCCAUAAUAGCAAACGUAAGUAUGGUUCUGCU
AUAGAAGAUUUGCUUUUUGAUAAAGUUGUAACAUCUGGUUUAGGUACAGUUGAUGAAGAUUAUAAACGUUG
UACUGGUGGUUACGACAUAGCAGACUUGGUGUGUGCUCAAUAUUACAAUGGCAUCAUGGUUCUACCAGGUGU
AGCUAAUGCUGACAAGAUGACUAUGUACACAGCAUCACUUGCAGGUGGUAUAACAUUAGGUGCACUUGGUGG
UGGCGCCGUGGCUAUACCUUUUGCAGUAGCAGUACAGGCUAGACUUAAUUAUGUUGCUCUACAAACUGAUGU
AUUGAAUAAAAACCAACAGAUCCUGGCUAAUGCCUUCAAUCAAGCUAUUGGUAACAUUACACAGGCUUUUGGU
AAGGUUAAUGAUGCUAUACAUCAAACAUCACAAGGUCUUGCCACUGUUGCUAAAGCGUUGGCAAAAGUGCAAG
AUGUUGUCAACACACAAGGGCAAGCUUUAAGUCACCUUACAGUACAAUUGCAAAAUAAUUUUCAAGCCAUUAG
UAGUUCUAUUAGUGAUAUUUAUAACAGGCUUGACGAACUGAGUGCUGAUGCACAAGUUGAUAGGCUGAUUAC
AGGUAGACUUACAGCACUUAAUGCAUUUGUGUCUCAGACUCUAACCAGACAAGCAGAGGUUAGGGCUAGUAGA
CAACUUGCCAAAGACAAGGUUAAUGAAUGUGUUAGGUCUCAGUCUCAGAGAUUCGGAUUCUGUGGUAAUGGU
ACACAUUUGUUUUCACUAGCAAAUGCAGCACCAAAUGGCAUGAUUUUCUUUCAUACAGUACUAUUACCAACAG
CUUAUGAAACUGUAACAGCUUGGUCAGGUAUUUGUGCUUCAGAUGGCGAUCGCACUUUCGGACUUGUCGUUA
AAGAUGUGCAGUUGACGUUGUUUCGUAAUCUAGAUGACAAGUUCUAUUUGACCCCCAGAACUAUGUAUCAGCC
UAGAGUUGCAACUAGUUCUGAUUUUGUUCAAAUUGAAGGGUGUGAUGUGUUGUUUGUCAACGCGACUGUAA
UUGAUUUGCCUAGUAUUAUACCUGACUAUAUUGACAUUAAUCAAACUGUUCAAGACAUAUUAGAAAAUUACA
GACCAAACUGGACUGUACCUGAAUUUACACUUGAUAUUUUCAACGCAACCUAUUUAAAUCUGACUGGUGAAAU
UGAUGACUUAGAGUUUAGGUCAGAAAAGCUACAUAACACUACAGUAGAACUUGCCAUUCUCAUUGAUAACAUU
AAUAAUACAUUAGUCAAUCUUGAAUGGCUCAAUAGAAUUGAAACUUAUGUAAAAUGGCCUUGGUAUGUGUGG
CUACUGAUAGGUUUAGUAGUAGUAUUUUGCAUACCAUUACUGCUAUUUUGCUGUUUUAGCACAGGUUGUUG
UGGAUGCAUAGGUUGUUUAGGAAGUUGUUGUCACUCUAUAUGUAGUAGAAGACAAUUUGAAAAUUAUGAACC
AAUUGAAAAAGUGCAUGUCCACGGCAGCGGCGCGACCAACUUUAGCCUGCUGAAACAGGCGGCGAUGUGGAA
GAAAACCCGGGCCCGAUGAAGUACAUUUUGUUAAUACUCGCGUGCAUUAUUGCAUGCGUUUAUGGAGAACGC
UACUGUGCCAUGCAAAAUACAGGCUCGCAGUGCAUUAAUGGCACAGAUUCAUCAUGUAGCACCUGUUUUGAAC
GUGGUGGUCUUAUUUGGCAUCUGGCUAACUGGAACUUCAGCUGGUCUGUAAUAUUGAUUGUUUUUAUAACA
GUGUUAAAAUAUGGAAGACCGCAAUUCAGCUGGCUCGUUUAUGGCAUUAAAAUGCUGAUCAUGUGGCUAUUA
UGGCCUAUUGUUCUAGCGCUUACGAUUUUUAAUGCAUACUCUGAGUACCAAGUUUCCAGAUAUGUAAUGUUC
GGCUUUAGUGUUGCAGGUGCAGUUGUAACGUUUGCACUAUGGAUGAUGUAUUUUGUGAGAUCUAUUCAGCU
GUAUAGACGGACCAAAUCAUGGUGGUCUUUUAAUCCUGAAACCAAUGCGAUUCUUUGUGUCAAUGCAUUGGG
UAGAAGCUAUGUACUCCCUCUUGAUGGCACUCCUACAGGUGUUACUCUUACCCUACUUUCAGGAAAUCUAUAC
GCUGAAGGUUUUAAAAUGGCUGGUGGUCUUACCAUCGAGCAUUUGCCUAAAUAUGUCAUGAUUGCUACGCCU

AGUAGAACCAUCGUUUACACAUUAGUUGGAAAACAACUAAAGGCAACUACUGCCACUGGAUGGGCUUACUAUG
UAAAAUCUAAAGCUGGUGAUUACUCAACAGAAGCACGUACUGAUAAUUUGAGUGAACAUGAAAAAUUAUUACA
UAUGGUGUAAGCUCCCUUUCUUGCUUUCCAGGGAAAGUCCCUUGGUCCCCAGACCCUAAUACCUGGACAUGUC
UAAAUCAUGAAAUUCUUUGAACAUCUGGCUCCUGCCUAAUAAAGAAUAUUUAUUUUCAUUGCAAAAAAAAAA
AAAAAAAAAAAAAAAAAAAAAAAAAAAAAAAAAAAAAAAAAAAAAAAAAAAAAAAAAAAAAAAAAAAAAAAAAA
AAAAAAAAAAAAAAA

## Claims

1. A DNA element which is derived from linking nucleotides comprising at least one element selected from the three elements for M protein, N protein, and S protein of an FCoV virus strain.

2. The DNA element according to claim 1, wherein the DNA element can be linked by linker peptides; preferably, when the number of linker peptides is greater than one, a plurality of linker peptides can be same or different, preferably the same; preferably, the linker peptides can be 2A linkers or IRES sequences, preferably 2A linkers (P2A/ T2A/E2A/F2A).

3. The DNA element according to claim 1, wherein the element number of the DNA element is preferably 2-3.

4. The DNA element according to claim 1, wherein the structure of the recombinant DNA molecule can be any one of combinations of nucleotide sequences of M protein, N protein, S protein, M+N, N+M, M+S, S+M, N+S, S+N, N+M+S, N+S+M, S+N+M, M+N+S, M+S+N, and S+M+N; preferably M+N, N+M, N+M+S, N+S+M, S+N+M, M+N+S, M+S+N, and S+M+N; more preferably N+M, N+M+S, and N+S+M.

5. The DNA element according to any one of claims 1-4, wherein the S protein is any one of full-length S, S1 protein, and S2 protein.

6. The DNA element according to claim 4, wherein the DNA sequences of the element are set forth in SEQ ID NOs: 7-23, and the amino acid sequences are set forth in SEQ ID NOs: 60-76; the DNA sequences of the linker peptides are set forth in SEQ ID NOs: 4-5, and the amino acid sequences are set forth in SEQ ID NOs: 58-59; and sequences having at least 66%, 75%, 85%, or 95% identity and capable of achieving the same function.

7. A recombinant nucleic acid molecule, of which the structure thereof is formed by linking a plurality of elements comprising a 5' untranslated region, a Kozak sequence region, at least one antigen-coding region, a 3' untranslated region, and polyA, wherein the antigen-coding region is the DNA element according to any one of claims 1-6, and the nucleic acid molecule is a DNA molecule or an RNA molecule.

8. The recombinant nucleic acid molecule according to claim 7, wherein the sequence of the 5' untranslated region is derived from DNAH2, feline hemoglobin, or feline albumin gene; the sequence of the 3' untranslated region is derived from feline hemoglobin or feline albumin gene; and the polyA element is a sequence formed by linking 1 to 200 A nucleotides.

9. The recombinant nucleic acid molecule according to any one of claims 7-8, wherein a promoter can be further included at 5' terminal of the 5' untranslated region, and a signal peptide coding region can be further included at 3' terminal of the 5' untranslated region.

10. The recombinant nucleic acid molecule according to claim 9, wherein the 5' untranslated region sequence is set forth in SEQ ID NO:1; the Kozak sequence is set forth in SEQ ID NO:2; the 3' untranslated region is set forth in SEQ ID NO:3; the polyA sequence is set forth in SEQ ID NO:6; the DNA sequence of the recombinant nucleic acid molecule is set forth in SEQ ID NOs: 24-40; and sequences having at least 66%, 75%, 85%, or 95% identity thereto and capable of achieving the same function.

11. A recombinant vector, comprising the DNA element according to any one of claims 1-6 or the recombinant nucleic acid molecule according to any one of claims 7-10.

12. An mRNA obtained by transcription of the recombinant nucleic acid molecule according to any one of claims 7-10, which preferably can be set forth in SEQ ID NOs: 41-57.

13. A protein obtained by translation of the DNA element according to any one of claims 1-6 or the recombinant nucleic acid molecule according to any one of claims 7-10.

14. A nucleotide sequence expressing the protein according to claim 13.

15. A recombinant cell obtained by introducing the DNA element according to any one of claims 1-6, the recombinant nucleic acid molecule according to any one of claims 7-10, the recombinant vector according to claim 11, or the mRNA according to claim 12 into cells.

16. A pharmaceutical composition comprising the DNA element according to any one of claims 1-6, the recombinant nucleic acid molecule according to any one of claims 7-10, the recombinant vector according to claim 11, or the mRNA according to claim 12; wherein the pharmaceutical composition is a therapeutic composition or a vaccine composition.

17. The pharmaceutical composition according to claim 16, which can be in the form of single component or a mixture of multiple components, wherein the multiple components can be mixed in different ratios, and the components used in the mixture can besame or different.

18. The pharmaceutical composition according to claim 16, further comprising a pharmaceutically acceptable excipient, adjuvant, vehicle, and lipid.

19. The pharmaceutical composition according to claim 18, wherein the lipid comprises a cationic lipid, a PEGylated lipid, cholesterol, and/or a phospholipid.

20. A method for preparing an mRNA vaccine, comprising steps of: (1) DNA template synthesis; (2) mRNA transcription; (3) mRNA capping; (4) mRNA purification; wherein the DNA template is the DNA element according to any one of claims 1-6 or the recombinant DNA molecule according to any one of claims 7-10.

21. Use of the DNA element according to any one of claims 1-6, the recombinant DNA molecule according to any one of claims 7-10, the recombinant vector according to claim 11, the mRNA according to claim 12, the protein according to claim 13-14, the recombinant cell according to claim 15, or the pharmaceutical composition according to any one of claims 16-19, for treating and/or preventing an animal coronavirus infection disease, or for preparation of a medication for treating and/or preventing an animal coronavirus infection disease.

22. The use according to claim 21, wherein the animal coronavirus infection disease is a disease caused by an enteric coronavirus infection or an infectious peritonitis virus infection.

23. A method for treating and/or preventing an animal coronavirus infection disease, the method comprising administering to a subject the DNA element according to any one of claims 1-6, the recombinant DNA molecule according to any one of claims 7-10, the recombinant vector according to claim 11, the mRNA according to claim 12, the protein according to claim 13-14, the recombinant cell according to claim 15, or the pharmaceutical composition according to any one of claims 16-19.

24. The use and method according to claims 21-23, wherein the animal is a mammal, including but not limited to humans, cats, dogs, mice, horses, cattle, sheep, pigs, and monkeys.

25. The use according to claim 24, wherein the use is further for treating and/or preventing Feline Infectious Peritonitis (FIP) caused by Feline Coronavirus (FCoV).

26. The method according to claim 25, wherein the administration method is single or multiple doses, and an interval between administrations is 18-26 days, wherein when the administration method involves multiple doses, the medications administered in each dose can be the same or different.
